# EUROPEAN PATENT APPLICATION

(11) **EP 4 850 700 A1**
(43) Date of publication of application: **09.12.2026**
(21) Application number: 25747873.5
(22) Date of filing: 24.01.2025
(51) Int. Cl.: A61K 47/68, A61K 47/54, A61P 35/00, A61P 31/00, A61P 7/00, A61P 3/00, A61P 29/00, A61P 37/00, A61P 9/00

(54) **ANTIBODY-DRUG CONJUGATE CONTAINING QUATERNARY AMMONIUM SALT AND MEDICAL USE THEREOF**

(30) Priority: 01.02.2024 CN 202410145541
(71) Applicant: ZHEJIANG YANGSHENGTANG INSTITUTE OF NATURAL MEDICATION CO., LTD., HANGZHOU, Zhejiang 310024 (CN)
(72) Inventor: XIAO, Dian, Beijing 100850 (CN); ZHOU, Xinbo, Beijing 100850 (CN); LI, Song, Beijing 100850 (CN); ZHONG, Wu, Beijing 100850 (CN); LIU, Lianqi, Beijing 100850 (CN); XIE, Fei, Beijing 100850 (CN); XIAO, Junhai, Beijing 100850 (CN); ZHENG, Zhibing, Beijing 100850 (CN); LI, Xingzhou, Beijing 100850 (CN); FAN, Shiyong, Beijing 100850 (CN); CAO, Ruiyuan, Beijing 100850 (CN); LI, Wei, Beijing 100850 (CN); LI, Pengyun, Beijing 100850 (CN); WANG, Zihao, Beijing 100850 (CN); YAN, Linjie, Beijing 100850 (CN)
(74) Representative: J A Kemp LLP
(86) International application number: PCT/CN2025/074881
(87) International publication number: WO 2025/162305

(57) **Abstract**

The present application relates to an antibody-drug conjugate containing a quaternary ammonium salt, further relates to a pharmaceutical composition comprising the antibody-drug conjugate, further relates to an intermediate for preparing the antibody-drug conjugate, and further relates to a use of the antibody-drug conjugate for treatment and/or prevention of a disease or condition.

## Description

### Cross-Reference

The present application is based on and claims the benefit of priority from Chinese Application No. 202410145541.7, filed on February 01, 2024, the disclosures of which are incorporated herein by reference in its entirety.

### Technical Field

The present application belongs to the field of medicinal chemistry, specifically relates to an antibody-drug conjugate containing a quaternary ammonium salt, also to a pharmaceutical composition comprising the antibody-drug conjugate, further to an intermediate for preparing the antibody-drug conjugate, and also further to the use of the antibody-drug conjugate for treatment and/or prevention of a disease or condition.

### Background Art

Immunotherapy has revolutionized the field of cancer treatment. Immunotherapeutic agents have been incorporated into clinical guidelines for various cancer types, and investigations are ongoing in both adjuvant therapy and neoadjuvant therapy. Current studies are focused on identifying targets capable of triggering or enhancing anti-tumor immune responses. The stimulator of interferon genes (STING) receptor is an adaptor proteins located in the endoplasmic reticulum and exist as a dimer, the C-terminus of which is located in the cytoplasm, and participates in immune responses through a cyclic GMP-AMP synthetase (cGAS)-stimulator of interferon genes (STING) signaling pathway, the activation of which can efficiently induce intracellular release of type I interferon, further to exert immune effect to efficiently inhibit tumor cells (Nature, 2008, 455, 674-678). STING agonists are one of the hot spots in current anti-tumor studies, and multiple drug candidates have advanced into clinical studies. In 2018, the research team led by Professor Ramanjulu at GlaxoSmithKline (GSK), a world-famous pharmaceutical enterprise, discovered a novel immune agonist diaBZI. Compared with traditional cyclic dinucleotide agonists, diaBZI exhibits improved stability, tissue penetrance and drug efficacy (Nature, 2018 Dec; 564 (7736): 439-443). Although the STING agonists have been proved to have good anti-tumor and anti-viral effects, due to the immunotoxicity possibly caused by systemic administrations (especially at a high dose), their administration is currently limited to intratumoral injection in human clinical trials.

Conventional antibody-drug conjugates (ADCs) organically combine monoclonal antibodies with cytotoxins, integrating the advantages of both the antibodies and the cytotoxins, and have characteristics such as strong targeting ability, high cytotoxicity, low toxic and side effects and long degradation half-life. An ADC structurally comprise three components, an antibody, a small molecule cytotoxin and a linker, wherein the antibody serves to achieve targeted delivery, the cytotoxin functions to kill target cells, and the linker covalently conjugates the antibody and cytotoxin into an integrated entity. Studies have demonstrated that compared with small-molecule drugs, ADCs exhibit over 100-fold improvement in targeting efficiency (Clinical Cancer Research, 2015, 21(22): 5131-5138), and thus the ADCs, as a platform technology, possess prominent advantages in targeted therapy. The FDA has approved 15 ADC drugs such as Mylotarg, Adcetris, Kadcyla, Besponsa, Lumoxiti, Polivy, Padcev and Enhertu, and more than 100 ADC candidates are under clinical studies showing tremendous application potentials in various fields such oncology, infectious diseases and immunology.

With the rapid advancement of ADC research, a variety of novel conjugated drug forms have emerged continuously. Among them, immune-stimulating antibody conjugates (ISACs) is a novel conjugated drug form (Nat cancer. 2021 Jan; 2(1): 18-33). ISACs structurally consist of an antibody, a linker, and a small-molecule immune agonists (an innate immune agent or immunomodulator). ISACs deliver the small-molecule immune agonist to tumor tissues relying on the targeting capability of the antibody and enter cells via antigen-mediated endocytosis, and then they are cleaved under the action of cathepsin to release small-molecule immune agonists, exerting drug efficacy. Compared with small-molecule immune agonists, ISACs can selectively act on tumor cells or immune cells surrounding tumor cells, triggering localized anti-tumor immune responses to skill tumor cells, and avoiding systemic immune responses induced by small-molecule immune agonist and reducing the risk of cytokine storm.

In 2022, Wu et al. reported a novel ISAC, αEGFR-172 (Proc Natl Acad Sci U S A. 2022 Dec 6;119(49): e2214278119), its antibody is an epidermal growth factor receptor (EGFR)-targeted monoclonal antibody, and its payload is a CDN-based STING agonist IMSA172, which are conjugated via a cleavable linker. In vivo studies have demonstrated that αEGFR-172 can efficiently activate the cGAS-STING pathway in EGFR-overexpressed cells and efficiently inhibit tumor growth in the mouse B16F10 tumor model. Although the ISACs perfectly integrate the precise tumor-targeting capability of antibodies with the tumoricidal potency of small-molecule immune agonists, and have important clinical values and application prospects, their clinical application still faces safety challenges. In March 2023, Mersana Therapeutics' ISAC candidate XMT-2056 had been placed on clinical hold after a Grade 5 (fatal) treatment-related severe adverse event emerged during its phase I clinical trial. The failure of XMT-2056 stems from insufficient stability of the ester-based linker it employs in the circulatory system. Accordingly, novel linker technologies are urgently required to advance the development of ISACs.

### Summary of the Disclosure

The inventors found that conjugating an antibody, a linker and a small-molecule immune agonist through a quaternary ammonium salt can efficiently enhance the stability of ISACs in the circulatory system, improve the solubility of effector molecules and enable targeted delivery of immunostimulants.

Accordingly, a first aspect of the present application relates to a compound represented by Formula II, a tautomer, a geometric isomer, an optical isomer, a polymorph or a pharmaceutically acceptable hydrate or solvate thereof, 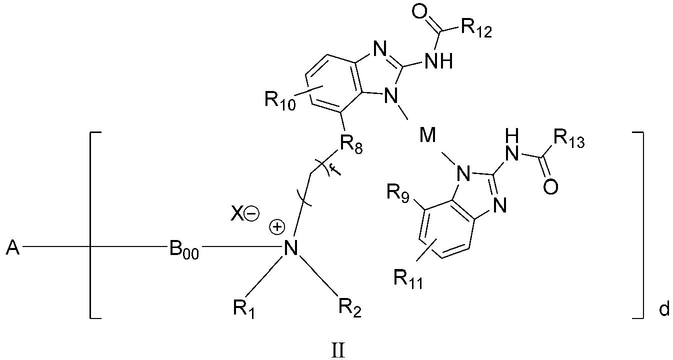 wherein:
A represents a biological macromolecule or a fragment thereof,
B₀₀ represents a linker, for conjugating A to the -NR₁R₂- group,
X represents a negatively charged anion,
R₁ and R₂ each independently are C₁₋₈ alkyl, C₁₋₈ alkyl cycloalkyl, C₄₋₈ cycloalkyl, 4- to 8-membered heterocycloalkyl, C₁₋₈ alkyl glycosyl, C₅₋₁₄ aryl, or 5- to 14-membered heteroaryl, or
R₁ and R₂, together with the nitrogen atom to which they are linked, form 5- to 7-membered heterocycloalkyl or heteroaryl, wherein the heterocycloalkyl contains one N atom and one, two or three other heteroatoms selected from the group consisting of N, O or S atom, the heterocyclyl or heteroaryl is optionally substituted with one, two, three or four R', and each R' independently is H, C₁₋₈ alkyl, C₁₋₈ alkoxy, fluoro, chloro, bromo, iodo, hydroxy, cyano, amino or nitro,
M is -(CH₂)ₒ-, -(CH₂)ₒ-CH=CH-(CH₂)ₚ-, -O-(CH₂)ₒ-, -NH-(CH₂)ₒ-, -O-(CH₂)ₒ-CH=CH-(CH₂)ₚ-O-, -NH-(CH₂)ₒ-CH=CH-(CH₂)ₚ-NH- or -CH₂-CHOH-CHOH-CH₂-, wherein o and p each independently are an integer from 0 to 9,
f is 0 or 1,
R₈ is -(CH₂)ₐ-, -(CH₂)ₐ-O-, -(CH₂)ₐ-S- or -(CH₂)ₐ-NH-, wherein a is an integer from 1 to 8,
R₉ is C₁₋₈ alkyl, C₁₋₈ alkoxy, C₁₋₈ alkylthio or C₁₋₈ alkylamino,
R₁₀ and R₁₁ each independently are C₁₋₄ alkyl, -CONR^{a}R^{b}, -CH₂NR^{a}R^{b}, -NR^{a}R^{b}, -NR^{a}C(=O)-R^{b}, - CH₂NR^{a}C(=O)-R^{b}, -C(=O)OCH₃ or -O-(CH₂)ₑ-OH, wherein R^{a} 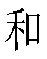 R^{b} each independently are H, hydroxy or C₁₋₄ alkyl, and e is an integer from 1 to 9,
R₁₂ and R₁₃ each independently are -(CH₂)_{g}-CH₃, -O-(CH₂)_{g}-OH, -O-(CH₂)_{g}-CH₃, -CONH₂, 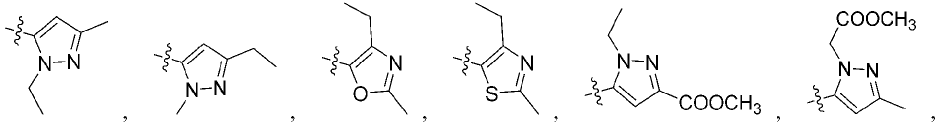 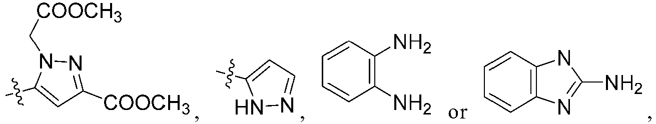 wherein g is a number from 1 to 9,
d is a number from 1 to 20.

A second aspect of the present application relates to an intermediate compound for the preparation of the above compound represented by Formula II, and specifically, the intermediate compound is a compound represented by Formula I, a tautomer, a geometric isomer, an optical isomer, a polymorph or a pharmaceutically acceptable hydrate or solvate thereof, 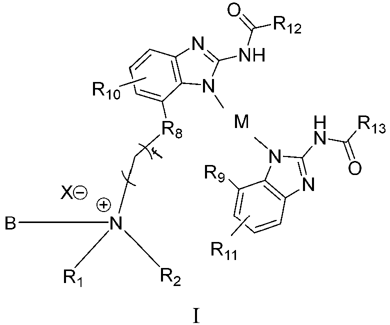 wherein:
B represents a linker, for conjugating the -NR₁R₂- group to a biological macromolecule or a fragment thereof,
X represents a negatively charged anion,
R₁ and R₂ each independently are C₁₋₈ alkyl, C₁₋₈ alkyl cycloalkyl, C₄₋₈ cycloalkyl, 4- to 8-membered heterocycloalkyl, C₁₋₈ alkyl glycosyl, C₅₋₁₄ aryl, 5- to 14-membered heteroaryl, or
R₁ and R₂, together with the nitrogen atom to which they are linked, form 5- to 7-membered heterocycloalkyl or heteroaryl, wherein the heterocycloalkyl contains one N atom and one, two or three other heteroatoms selected from the group consisting of N, O or S atom, the heterocyclyl or heteroaryl is optionally substituted with one, two, three or four R', and each R' independently is H, C₁₋₈ alkyl, C₁₋₈ alkoxy, fluoro, chloro, bromo, iodo, hydroxy, cyano, amino or nitro,
M is -(CH₂)ₒ-, -(CH₂)ₒ-CH=CH-(CH₂)ₚ-, -O-(CH₂)ₒ-, -NH-(CH₂)ₒ-, -O-(CH₂)ₒ-CH=CH-(CH₂)ₚ-O-, -NH-(CH₂)ₒ-CH=CH-(CH₂)ₚ-NH- or -CH₂-CHOH-CHOH-CH₂-, wherein o and p each independently are an integer from 0 to 9,
f is 0 or 1,
R₈ is -(CH₂)ₐ-, -(CH₂)ₐ-O-, -(CH₂)ₐ-S- or -(CH₂)ₐ-NH-, wherein a is an integer from 1 to 8,
R₉ is C₁₋₈ alkyl, C₁₋₈ alkoxy, C₁₋₈ alkylthio or C₁₋₈ alkylamino,
R₁₀ and R₁₁ each independently are C₁₋₄ alkyl, -CONR^{a}R^{b}, -CH₂NR^{a}R^{b}, -NR^{a}R^{b}, -NR^{a}C(=O)-R^{b}, - CH₂NR^{a}C(=O)-R^{b}, -C(=O)OCH₃ or -O-(CH₂)ₑ-OH, wherein R^{a} and R^{b} each independently are H, hydroxy or C₁₋₄ alkyl, and e is an integer from 1 to 9,
R₁₂ and R₁₃ each independently are -(CH₂)_{g}-CH₃, -O-(CH₂)_{g}-OH, -O-(CH₂)_{g}-CH₃, -CONH₂, 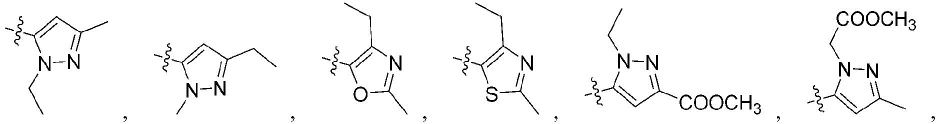 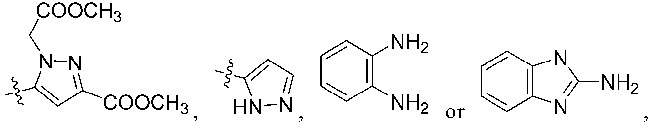 wherein g is a number from 1 to 9.

A third aspect of the present application relates to the use of the above compound represented by Formula I, a tautomer, a geometric isomer, an optical isomer, a polymorph or a pharmaceutically acceptable hydrate or solvate thereof in the preparation of an antibody-drug conjugate (e.g., an immune-stimulating antibody conjugate).

A fourth aspect of the present application relates to a pharmaceutical composition or vaccine, comprising the above compound represented by Formula II, a tautomer, a geometric isomer, an optical isomer, a polymorph or a pharmaceutically acceptable hydrate or solvate thereof, and optionally one or more pharmaceutically acceptable carriers or excipients.

A fifth aspect of the present application relates to the use of the above compound represented by Formula II, a tautomer, a geometric isomer, an optical isomer, a polymorph or a pharmaceutically acceptable hydrate or solvate thereof in the manufacture of a medicament for treating and/or preventing a disease or condition or alleviating the severity of the disease or condition, wherein the disease or condition is selected from the group consisting of a tumor, a pathogen infection, a disease caused by a pathogen infection, a hematological disease, a metabolic disease, an inflammation, an allergic disease, an autoimmune disease, a precancerous syndrome, tumor metastasis, and a cardiovascular disease.

A six aspect of the present application relates to a method for treating and/or preventing a disease or condition or alleviating the severity of the disease or condition, comprising administering a therapeutically effective amount of the compound represented by Formula II, a tautomer, a geometric isomer, an optical isomer, a polymorph or a pharmaceutically acceptable hydrate or solvate thereof to a subject in need thereof, wherein the disease or condition is selected from the group consisting of a tumor, a pathogen infection, a disease caused by a pathogen infection, a hematological disease, a metabolic disease, an inflammation, an allergic disease, an autoimmune disease, a precancerous syndrome, tumor metastasis, and a cardiovascular disease.

A seventh aspect of the present application relates to the compound represented by Formula II, a tautomer, a geometric isomer, an optical isomer, a polymorph or a pharmaceutically acceptable hydrate or solvate thereof, for use in treating and/or preventing a disease or condition or alleviating the severity of the disease or condition, wherein the disease or condition is selected from the group consisting of a tumor, a pathogen infection, a disease caused by a pathogen infection, a hematological disease, a metabolic disease, an inflammation, an allergic disease, an autoimmune disease, a precancerous syndrome, tumor metastasis, and a cardiovascular disease.

### Detailed Descriptions of the Disclosure

### Immune-stimulating Antibody Conjugates

The first aspect of the present application relates to an immune-stimulating antibody conjugate, which is a compound represented by Formula II, a tautomer, a geometric isomer, an optical isomer, a polymorph or a pharmaceutically acceptable hydrate or solvate thereof, 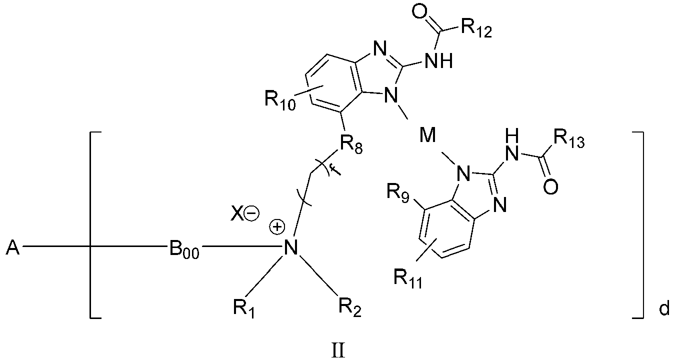 wherein:
A represents a biological macromolecule or a fragment thereof,
B₀₀ represents a linker, for conjugating A to the -NR₁R₂- group,
X represents a negatively charged anion,
R₁ and R₂ each independently are C₁₋₈ alkyl, C₁₋₈ alkyl cycloalkyl, C₄₋₈ cycloalkyl, 4- to 8-membered heterocycloalkyl, C₁₋₈ alkyl glycosyl, C₅₋₁₄ aryl, or 5- to 14-membered heteroaryl, or
R₁ and R₂, together with the nitrogen atom to which they are linked, form 5- to 7-membered heterocycloalkyl or heteroaryl, wherein the heterocycloalkyl contains one N atom and one, two or three other heteroatoms selected from the group consisting of N, O or S atom, the heterocyclyl or heteroaryl is optionally substituted with one, two, three or four R', and each R' independently is H, C₁₋₈ alkyl, C₁₋₈ alkoxy, fluoro, chloro, bromo, iodo, hydroxy, cyano, amino or nitro,
M is -(CH₂)ₒ-, -(CH₂)ₒ-CH=CH-(CH₂)ₚ-, -O-(CH₂)ₒ-, -NH-(CH₂)ₒ-, -O-(CH₂)ₒ-CH=CH-(CH₂)ₚ-O-, -NH-(CH₂)ₒ-CH=CH-(CH₂)ₚ-NH- or -CH₂-CHOH-CHOH-CH₂-, wherein o and p each independently are an integer from 0 to 9,
f is 0 or 1,
R₈ is -(CH₂)ₐ-, -(CH₂)ₐ-O-, -(CH₂)ₐ-S- or -(CH₂)ₐ-NH-, wherein a is an integer from 1 to 8,
R₉ is C₁₋₈ alkyl, C₁₋₈ alkoxy, C₁₋₈ alkylthio or C₁₋₈ alkylamino,
R₁₀ and R₁₁ each independently are C₁₋₄ alkyl, -CONR^{a}R^{b}, -CH₂NR^{a}R^{b}, -NR^{a}R^{b}, -NR^{a}C(=O)-R^{b}, - CH₂NR^{a}C(=O)-R^{b}, -C(=O)OCH₃ or -O-(CH₂)ₑ-OH, wherein R^{a} and R^{b} each independently are H, hydroxy or C₁₋₄ alkyl, and e is an integer from 1 to 9,
R₁₂ and R₁₃ each independently are -(CH₂)_{g}-CH₃, -O-(CH₂)_{g}-OH, -O-(CH₂)_{g}-CH₃, -CONH₂, 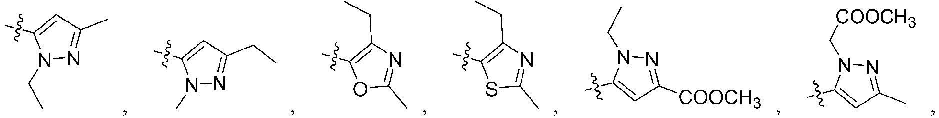 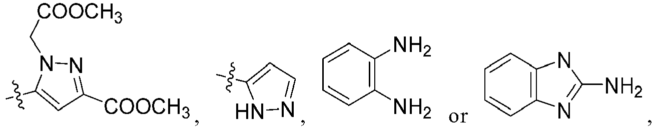 wherein g is a number from 1 to 9,
d is a number from 1 to 20.

In some embodiments, in the compound represented by Formula II, R₁₀ is ortho, meta, or para of R₈; and/or R₁₁ is ortho, meta, or para of R₉.

In some embodiments, in the compound represented by Formula II, R₁₀ is ortho of R₈, and R₁₁ is ortho of R₉.

In some embodiments, the compound represented by Formula II has a structure represented by Formula II-1, 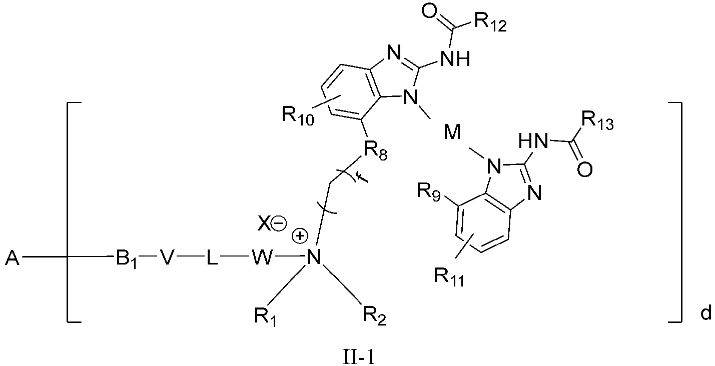
wherein A, X, R₁ and R₂, f, R₈, R₉, R₁₀, R₁₁, R₁₂, R₁₃, and d are as defined in any embodiment of the present application,
-B₁-V-L-W- represents a linker, for conjugating A to the -NR₁R₂- group, wherein
B₁ is selected from the group consisting of 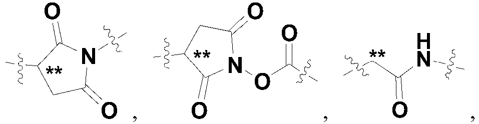 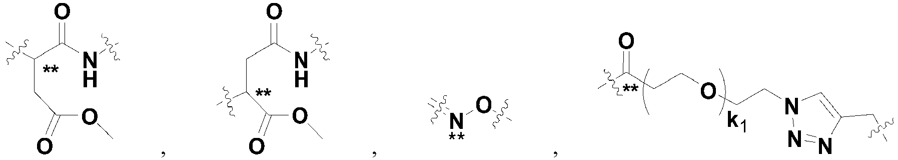 and 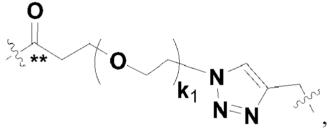 wherein k₁ is 1, 2, 3, 4, 5, 6, 7, 8, 9, 10, 11 or 12;
V is 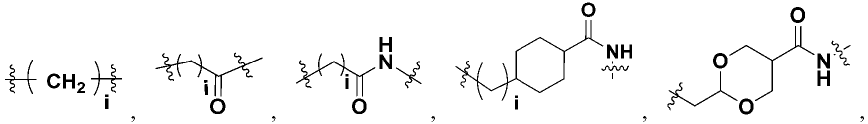 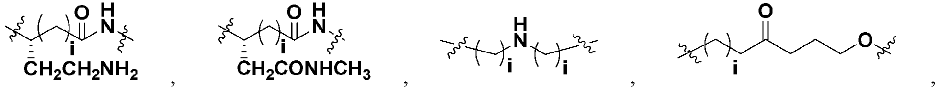 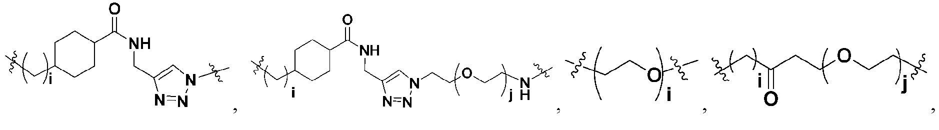 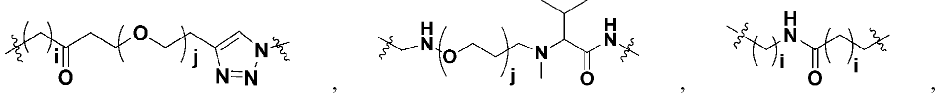 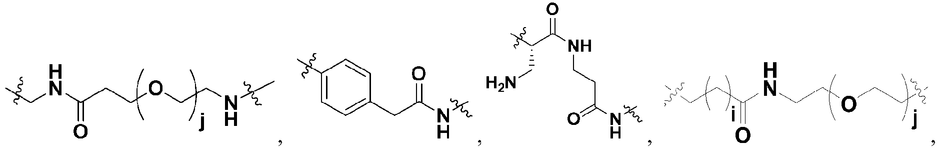 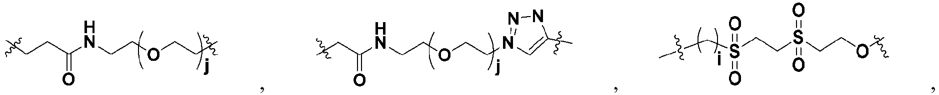 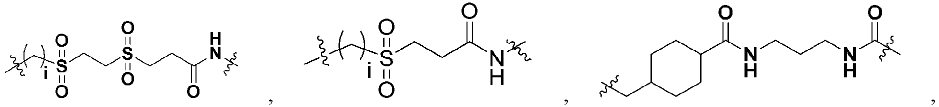 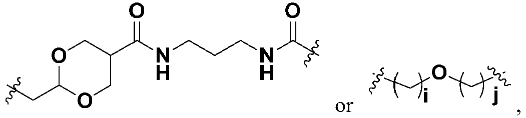 wherein each i independently is an integer from 0 to 12, each j independently is an integer from 0 to 12, or V is absent;
L is -(CH₂CH₂O)ₖ-(CH₂)₁-, -CHZ-, -(CH₂)ₘ -, or 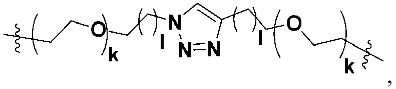 wherein Z is -(CH₂)ₙ-NHC(O)-(CH₂CH₂O)ₖ-CH₃, each k independently is an integer from 0 to 12, each 1 independently is an integer from 0 to 12, n is an integer from 0 to 12, m is an integer from 0 to 30, or L is absent;
W is 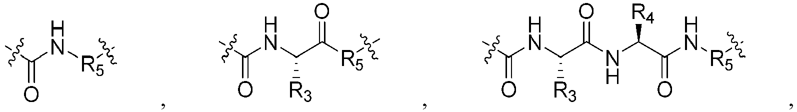 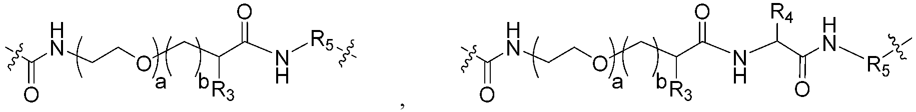 or 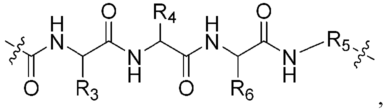 wherein R₃, R₄ and R₆ each independently are H, methyl, ethyl, n-propyl, iso-propyl, n-butyl, sec-butyl, iso-butyl, tert-butyl, phenyl, cyclobutyl, cyclopentyl, cyclohexyl, -CH₂(C₆H₅), -(CH2)_{c}-NH₂, -(CH₂)_{c}-NHC(NH)NH₂, -CHCH(CH₃)CH₃ or -(CH₂)_{c}-NHC(=O)NH₂, or 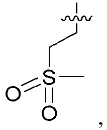 wherein each c independently is a number from 0 to 8; R₅ is 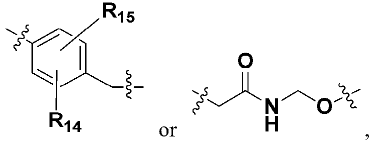 and R₁₅ each independently are H, methyl, ethyl, n-propyl, iso-propyl, n-butyl, sec-butyl, iso-butyl, tert-butyl, phenyl, cyclobutyl, cyclopentyl, cyclohexyl, -CH₂(C₆H₅), -(CH₂)ₕ-NH₂ or -(CH₂)ₕ, and each h independently is an number from 0 to 8,
A is conjugated to the position ** of the group B₁ via the S atom or the N atom in the biological macromolecule or a fragment thereof.

In some embodiments, in the compound represented by Formula II, X is Cl⁻, Br⁻, I⁻, NO₃⁻, HCOO⁻, CH₃COO⁻, CF₃COO⁻, SO₄²⁻, PO₄,³⁻, HPO₄²⁻ or H₂PO₄⁻.

In some embodiments, in the compound represented by Formula II, X is Cl⁻, Br⁻, I⁻, NO₃⁻, HCOO⁻, CH₃COO⁻, or CF₃COO⁻.

In some embodiments, in the compound represented by Formula II, X is Cl⁻, HCOO⁻, CH₃COO⁻, or CF₃COO⁻.

In some embodiments, in the compound represented by Formula II, X is Cl⁻, Br⁻, or I⁻.

In some embodiments, in the compound represented by Formula II, X is Cl⁻.

In some embodiments, in the compound represented by Formula II, R₁ and R₂ each independently are C₁₋₈ alkyl. In some embodiments, in the compound represented by Formula II, R₁ and R₂ each independently are C₁₋₆ alkyl. In some embodiments, in the compound represented by Formula II, R₁ and R₂ each independently are C₁₋₄ alkyl. In some embodiments, in the compound represented by Formula II, R₁ and R₂ each independently are methyl, ethyl, n-propyl, iso-propyl, n-butyl, iso-butyl, tert-butyl, n-pentyl, tert-pentyl, neopentyl, or n-hexyl.

In some embodiments, in the compound represented by Formula II, R₁ and R₂, together with the nitrogen atom to which they are linked, form 5- to 7-membered heterocycloalkyl, wherein the heterocycloalkyl contains one N atom and one, two or three other heteroatoms selected from the group consisting of N, O or S atom, the heterocycloalkyl is optionally substituted with one, two, three or four R', and each R' independently is H, C₁₋₈ alkyl, C₁₋₈ alkoxy, fluoro, chloro, bromo, iodo, hydroxy, cyano, amino or nitro. In some embodiments, in the compound represented by Formula II, R₁ and R₂, together with the nitrogen atom to which they are linked, form pyrrolidinyl, piperazinyl, piperidinyl, morpholinyl, or thiomorpholinyl. In some embodiments, in the compound represented by Formula II, R₁ and R₂, together with the nitrogen atom to which they are linked, form

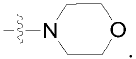

In some embodiments, in the compound represented by Formula II, R₁ and R₂, together with the nitrogen atom to which they are linked, form 5- to 7-membered heteroaryl, wherein the heteroaryl contains one N atom and one, two or three other heteroatoms selected from the group consisting of N, O or S atom, the heteroaryl is optionally substituted with one, two, three or four R', and each R' independently is H, C₁₋₈ alkyl, C₁₋₈ alkoxy, fluoro, chloro, bromo, iodo, hydroxy, cyano, amino or nitro. In some embodiments, in the compound represented by Formula II, R₁ and R₂, together with the nitrogen atom to which they are linked, form imidazolyl, triazolyl, indolyl, tetrazolyl, pyridyl, pteridinyl, pyrimidinyl, quinolinyl, isoquinolinyl, quinazolinyl, quinoxalinyl, or thiazinyl.

In some embodiments, in the compound represented by Formula II, R' is H, C₁₋₆ alkyl, C₁₋₆ alkoxy, fluoro, chloro, bromo, iodo, hydroxy, cyano, amino or nitro. In some embodiments, in the compound represented by Formula II, R' is H, C₁₋₄ alkyl, C₁₋₄ alkoxy, fluoro, chloro, bromo, iodo, hydroxy, cyano, amino or nitro. In some embodiments, in the compound represented by Formula II, R' is H. In some embodiments, in the compound represented by Formula II, R' is methyl, ethyl, n-propyl, iso-propyl, n-butyl, iso-butyl, tert-butyl, n-pentyl, tert-pentyl, neopentyl, or n-hexyl. In some embodiments, in the compound represented by Formula II, R' is methyl, ethyl or n-propyl. In some embodiments, in the compound represented by Formula II, R' is methoxy, ethoxy, propoxy or butoxy. In some embodiments, in the compound represented by Formula II, R' is fluoro, chloro, bromo, or iodo. In some embodiments, in the compound represented by Formula II, R' is fluoro, chloro, or brom. In some embodiments, in the compound represented by Formula II, R' is cyano, amino, or nitro.

In some embodiments, in the compound represented by Formula II, M is -(CH₂)ₒ-CH=CH-(CH₂)ₚ-, wherein o and p each independently are an integer from 0 to 9. In some embodiments, in the compound represented by Formula II, o and p each independently are an integer from 1 to 6, such as 1, 2, 3, 4, 5 or 6.

In some embodiments, in the compound represented by Formula II, f is 0. In some embodiments, in the compound represented by Formula II, f is 1.

In some embodiments, in the compound represented by Formula II, R₈ is -(CH₂)ₐ-O-, -(CH₂)ₐ-S- or -(CH₂)ₛ-NH-. In some embodiments, in the compound represented by Formula II, R₈ is -(CH₂)ₐ-O-.

In some embodiments, in the compound represented by Formula II, a is an integer from 1 to 6, such as 1, 2, 3, 4, 5 or 6.

In some embodiments, in the compound represented by Formula II, R₉ is C₁₋₈ alkoxy, C₁₋₈ alkylthio or C₁₋₈ alkylamino. In some embodiments, in the compound represented by Formula II, R₉ is C₁₋₈ alkoxy. In some embodiments, in the compound represented by Formula II, R₉ is C₁₋₆ alkoxy. In some embodiments, in the compound represented by Formula II, R₉ is C₁₋₄ alkoxy. In some embodiments, in the compound represented by Formula II, R₉ is methoxy, ethoxy, n-propoxy, iso-propoxy, n-butoxy, tert-butoxy, sec-butoxy, n-pentyloxy, n-hexyloxy or 1,2-dimethylbutoxy. In some embodiments, in the compound represented by Formula II, R₉ is methoxy, ethoxy, n-propoxy or n-butoxy. In some embodiments, in the compound represented by Formula II, R₉ is C₁₋₆ alkylthio. In some embodiments, in the compound represented by Formula II, R₉ is C₁₋₄ alkylthio. In some embodiments, in the compound represented by Formula II, R₉ is methylthio, ethylthio, propylthio, or butylthio. In some embodiments, in the compound represented by Formula II, R₉ is C₁₋₆ alkylamino. In some embodiments, in the compound represented by Formula II, R₉ is C₁₋₄ alkylamino. In some embodiments, in the compound represented by Formula II, R₉ is methylamino, ethylamino, propylamino, or butylamino.

In some embodiments, in the compound represented by Formula II, R₁₀ and R₁₁ each independently are -CONR^{a}R^{b}, -CH₂NR^{a}R^{b} or -CH₂NR^{a}C(=O)-R^{b}. In some embodiments, in the compound represented by Formula II, R₁₀ and R₁₁ each independently are -CONR^{a}R^{b}.

In some embodiments, in the compound represented by Formula II, R^{a} and R^{b} each independently are H or C₁₋₄ alkyl. In some embodiments, in the compound represented by Formula II, R^{a} and R^{b} each independently are H. In some embodiments, in the compound represented by Formula II, R^{a} and R^{b} each independently are methyl, ethyl, n-propyl or n-butyl.

In some embodiments, in the compound represented by Formula II, R₁₂ and R₁₃ each independently is 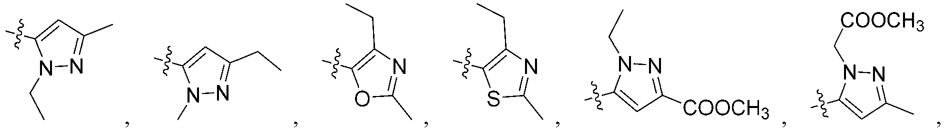 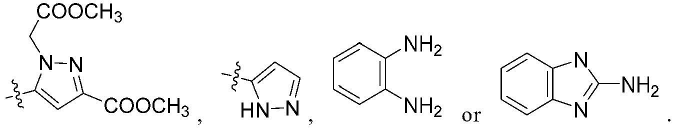 In some embodiments, in the compound represented by Formula II, R₁₂ and R₁₃ each independently are 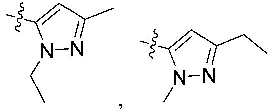 or 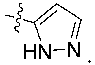 In some embodiments, in the compound represented by Formula II, R₁₂ and R₁₃ each independently are

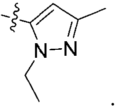

In some embodiments, in the compound represented by Formula II, d is a number from 1 to 8. In some embodiments, in the compound represented by Formula II, d is a number from 1 to 6, such as about 1, about 2, about 3, about 4, about 5 or about 6.

In some embodiments, in the compound represented by Formula II, A is an antibody or an antigen-binding fragment thereof, a polypeptide, a protein or an antigen.

In some embodiments, in the compound represented by Formula II, A is an antibody or an antigen-binding fragment thereof, a protein or an antigen.

In some embodiments, in the compound represented by Formula II, A is an antigen.

In some embodiments, in the compound represented by Formula II, A is an antibody or an antigen-binding fragment thereof, a polypeptide or a protein capable of specifically binding to a target.

In some embodiments, in the compound represented by Formula II, A is an antibody or an antigen-binding fragment thereof or a protein capable of specifically binding to a target.

In some embodiments, in the compound represented by Formula II, A is an antibody or an antigen-binding fragment thereof capable of specifically binding to a target.

In some embodiments, in the compound represented by Formula II, A is a protein capable of specifically binding to a target.

In some embodiments, the antibody is selected from the group consisting of a monoclonal antibody (MAB), a polyclonal antibody, an IgG antibody, a murine antibody, a rabbit antibody, a humanized antibody, a fully human antibody, a chimeric antibody (e.g., a human-mouse chimeric antibody), a bispecific antibody, a multispecific antibody, and a probody.

In some embodiments, the antibody-binding fragment is selected from the group consisting of a single chain antibody, dAb, a complementarity determining region fragment, Fv, singlechain Fv (scfv), Fd, Fab, Fab', F(ab')₂ and VHH (a nanometer antibody, Nanobody or Nb).

In some embodiments, the protein is albumin, preferably is human serum albumin.

In some embodiments, in the compound represented by Formula II, A is a monoclonal antibody or an antigen-binding fragment thereof.

In some embodiments, in the compound represented by Formula II, A is a monoclonal antibody or an antigen-binding fragment thereof with thiol or amino group as conjugating sites, or a site-directed mutated or modified monoclonal antibody or an antigen-binding fragment thereof with thiol or amino group as conjugating sites.

In some embodiments, in the compound represented by Formula II, A is selected from the group consisting of: humanized anti-HER2 monoclonal antibody mil40, trastuzumab (HERCEPTIN), pertuzumab (PERJETA), cetuximab (ERBITUX), panitumumab (VECTIBIX), rituximab (RITUXAN), alemtuzumab (CAMPATH), ibritumomab tiuxetan (ZEVALIN), tositumomab (BEXXAR), ofatumumab (ARZERRA), bevacizumab (AVASTIN), ipilimumab (YERVOY), denosumab (XGEVA), pembrolizumab (KEYTRUDA), nivolumab (Opdivo), avelumab (Bavencio), atezolizumab (Tecentriq), durvalumab (Imfinzi), sacituzumab, rovalpituzumab, an antibacterial antibody, a biological analog thereof or an antigen-binding fragment thereof, and a site-directed mutated or modified version of any of the above monoclonal antibodies or antigen-binding fragments thereof.

In some embodiments, in the compound represented by Formula II, Ais trastuzumab or an antigen-binding fragment thereof.

In some embodiments, in the compound represented by Formula II, B₁ is 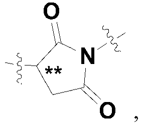 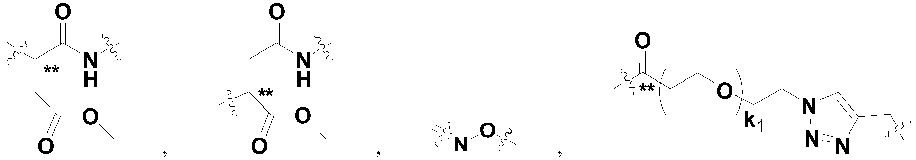 or 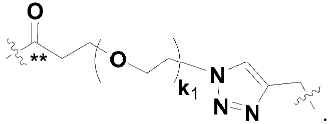 In some embodiments, in the compound represented by Formula II, B₁ is 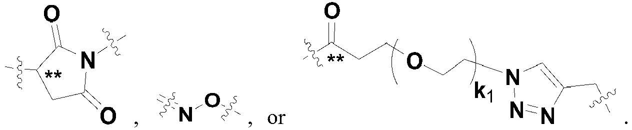 In some embodiments, in the compound represented by Formula II, B₁ is

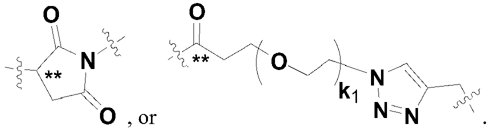

In some embodiments, in the compound represented by Formula II, k₁ is 1, 2, 3, 4, 5, 6, 7, 8, 9 or 10. In some embodiments, in the compound represented by Formula II, k₁ is 1, 2, 3, 4, 5, 6, 7 or 8. In some embodiments, in the compound represented by Formula II, k₁ is 2, 3, 4, 5 or 6.

In some embodiments, in the compound represented by Formula II, V is 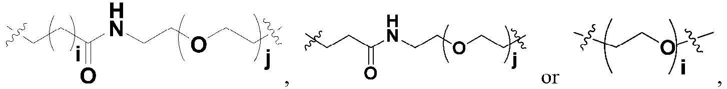 wherein i and j are as defined in any embodiment of the present application.

In some embodiments, in the compound represented by Formula II, i and j each independently are 1, 2, 3, 4, 5, 6, 7, 8, 9 or 10. In some embodiments, in the compound represented by Formula II, i and j each independently are 1, 2, 3, 4, 5, 6, 7 or 8. In some embodiments, in the compound represented by Formula II, i and j each independently are 1, 2, 3, 4, 5 or 6.

In some embodiments, in the compound represented by Formula II, L is -(CH₂CH₂O)ₖ-(CH₂)ₗ-, - CHZ-, or -(CH₂)ₘ -, wherein Z is -(CH₂)ₙ-NHC(O)-(CH₂CH₂O)ₖ-CH₃, and k, l, m, and n are as defined in any embodiment of the present application. In some embodiments, in the compound represented by Formula II, L is absent.

In some embodiments, in the compound represented by Formula II, each k independently is an integer from 0 to 10, such as 0, 1, 2, 3, 4, 5, 6, 7, 8, 9 or 10.

In some embodiments, in the compound represented by Formula II, each l independently is an integer from 0 to 10, such as 0, 1, 2, 3, 4, 5, 6, 7, 8, 9 or 10.

In some embodiments, in the compound represented by Formula II, n is an integer from 0 to 10, such as 0, 1, 2, 3, 4, 5, 6, 7, 8, 9 or 10.

In some embodiments, in the compound represented by Formula II, m is an integer from 0 to 20. In some embodiments, in the compound represented by Formula II, m is an integer from 0 to 16, such as 0, 1, 2, 3, 4, 5, 6, 7, 8, 9, 10, 11, 12, 13, 14, 15, or 16.

In some embodiments, in the compound represented by Formula II, L is absent.

In some embodiments, in the compound represented by Formula II, W is 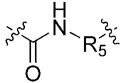 or 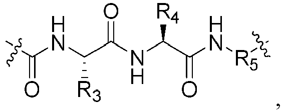 wherein R₃, R₄ and R₅ are as defined in any embodiment of the present application.

In some embodiments, in the compound represented by Formula II, R₃ and R₄ each independently are methyl, ethyl, n-propyl, iso-propyl or -(CH₂)_{c}- NHC(=O)NH₂, wherein c is as defined in any embodiment of the present application.

In some embodiments, in the compound represented by Formula II, R₅ is 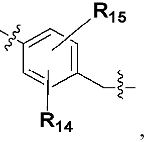 wherein R₁₄ and R₁₅ are as defined in any embodiment of the present application.

In some embodiments, in the compound represented by Formula II, R₃ and R₄ each independently are methyl, iso-propyl or -(CH₂)_{c}- NHC(=O)NH₂.

In some embodiments, in the compound represented by Formula II, c is an integer from 0 to 6, such as 0, 1, 2, 3, 4, 5, or 6.

In some embodiments, in the compound represented by Formula II, R₁₄ and R₁₅ each independently are H, methyl, ethyl, n-propyl, iso-propyl, n-butyl, sec-butyl, iso-butyl or tert-butyl. In some embodiments, in the compound represented by Formula II, R₁₄ and R₁₅ each independently are H, methyl, ethyl or n-propyl. In some embodiments, in the compound represented by Formula II, R₁₄ and R₁₅ each independently are H or methyl. In some embodiments, in the compound represented by Formula II, R₁₄ and R₁₅ each independently are H.

In some embodiments, in the compound represented by Formula II, -V-L-W- is 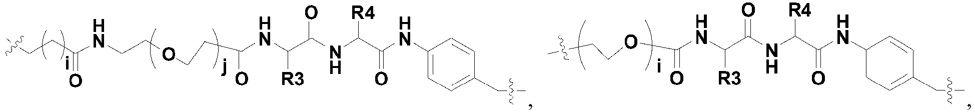 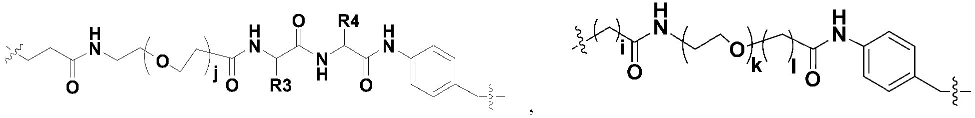 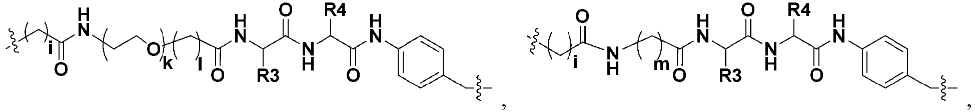 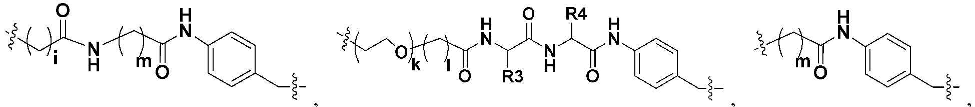 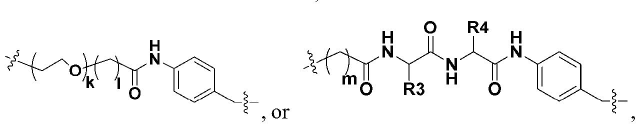 wherein i, j, k, l, m, R₃, and R₄ are as defined in any embodiment of the present application.

In some embodiments, in the compound represented by Formula II, -V-L-W- is 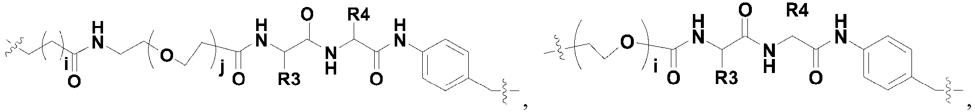 or 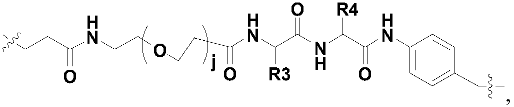 wherein i, j, k, l, m, R₃, and R₄ are as defined in any embodiment of the present application.

In some embodiments, the compound represented by Formula II has a structure represented by Formula II-2 or II-3, 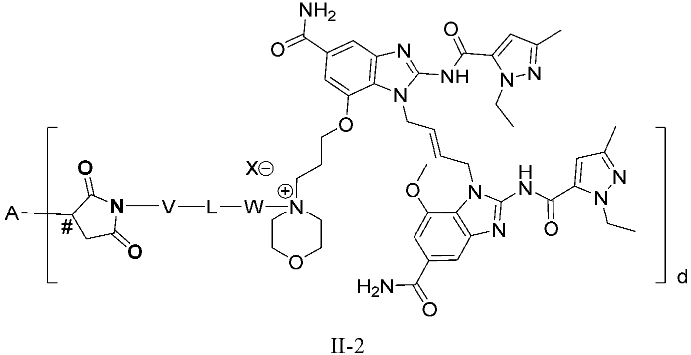 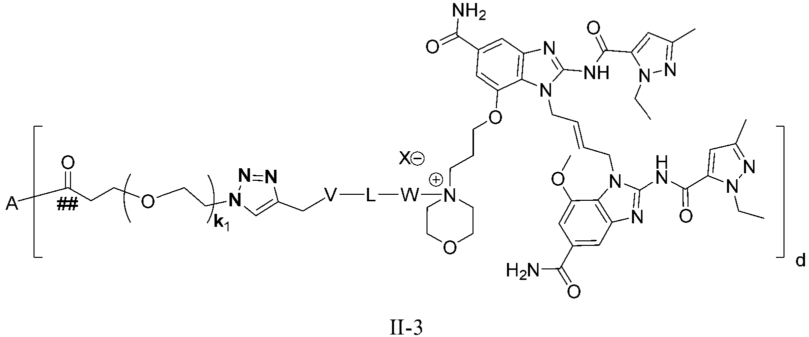 wherein A, X, V, L, W, and k₁ are as defined in any embodiment of the present application, A is conjugated to the position # via the S atom in the biological macromolecule or a fragment thereof, or A is conjugated to the position ## via the N atom in the biological macromolecule or a fragment thereof.

In some embodiments, the compound represented by Formula II is selected from the group consisting of: 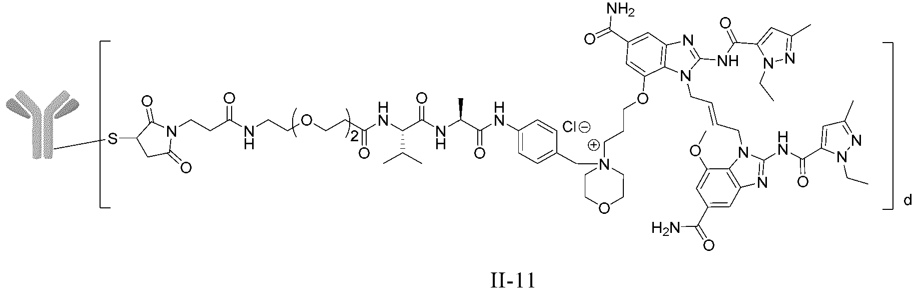 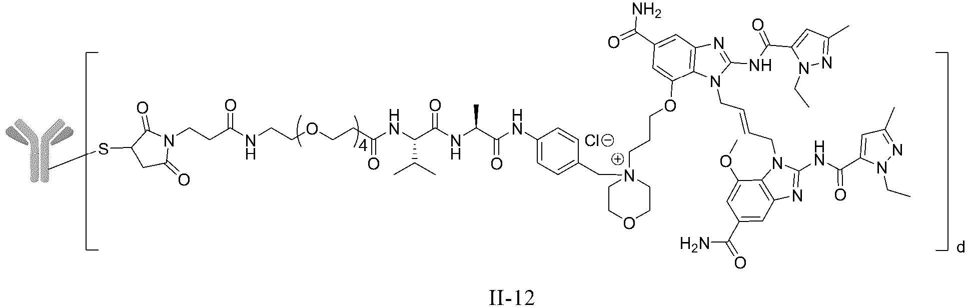 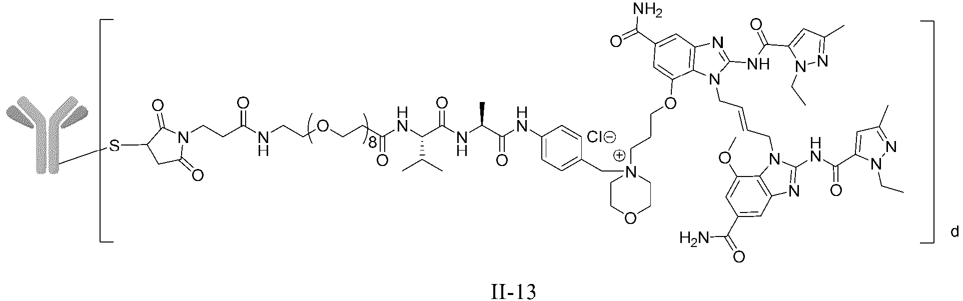 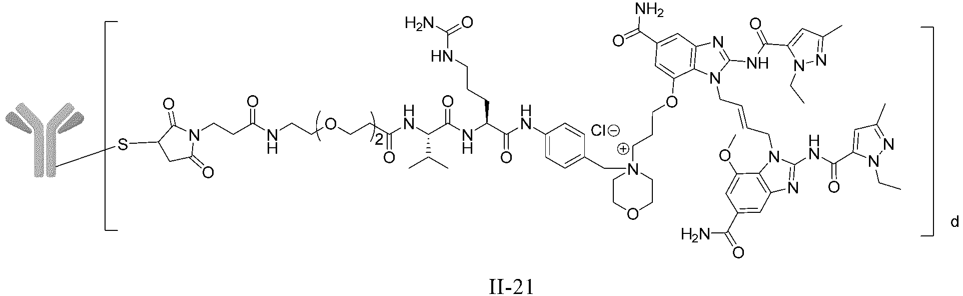 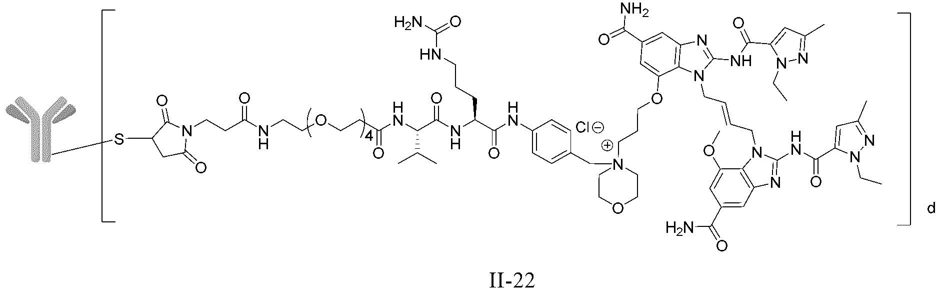 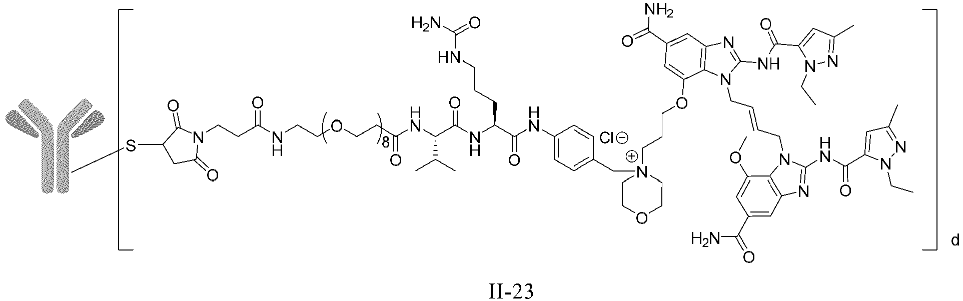 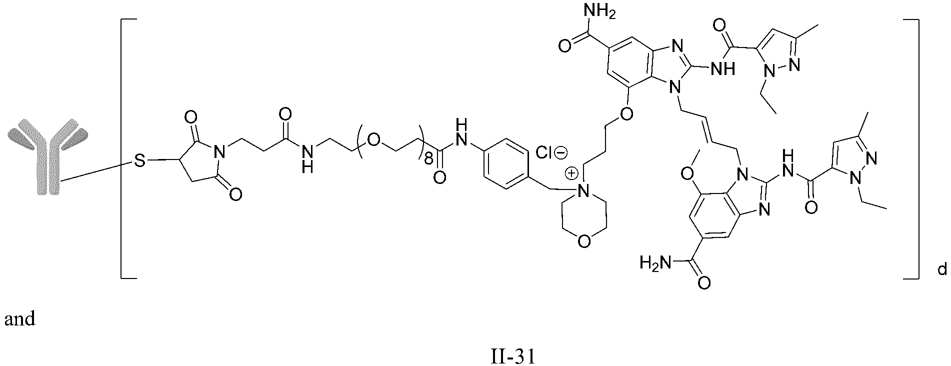 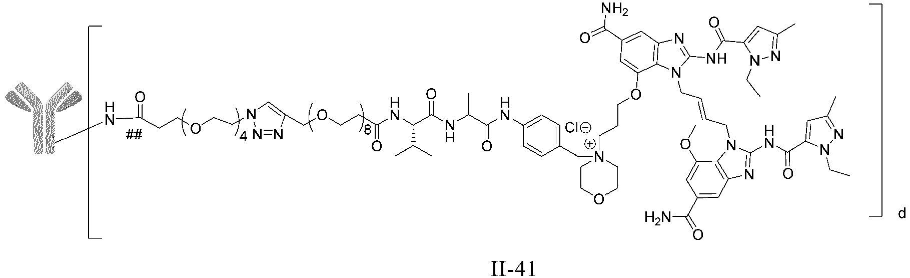 wherein, 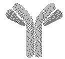 represents A, and A and d are as defined in any embodiment of the present application.

In some embodiments, in the compound represented by Formula II, the S atom linked to A is derived from a sulfur atom in A (e.g., a sulfur atom in a cysteine residue), and the -NH- group linked to A is derived from an amino group in A (e.g., a side-chain amino group of lysine, arginine, asparagine, or glutamine residue).

### Intermediate Compounds

The second aspect of the present application relates to an intermediate compound for the preparation of an immune-stimulating antibody conjugate, which is a compound represented by Formula I, a tautomer, a geometric isomer, an optical isomer, a polymorph or a pharmaceutically acceptable hydrate or solvate thereof, 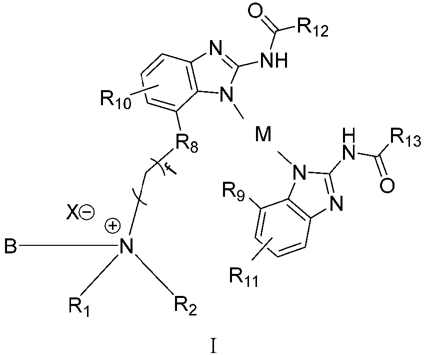 wherein:
B represents a linker, for conjugating the -NR₁R₂- group to a biological macromolecule or a fragment thereof,
X represents a negatively charged anion,
R₁ and R₂ each independently are C₁₋₈ alkyl, C₁₋₈ alkyl cycloalkyl, C₄₋₈ cycloalkyl, 4- to 8-membered heterocycloalkyl, C₁₋₈ alkyl glycosyl, C₅₋₁₄ aryl, 5- to 14-membered heteroaryl, or
R₁ and R₂, together with the nitrogen atom to which they are linked, form 5- to 7-membered heterocycloalkyl or heteroaryl, wherein the heterocycloalkyl contains one N atom and one, two or three other heteroatoms selected from the group consisting of N, O or S atom, the heterocyclyl or heteroaryl is optionally substituted with one, two, three or four R', and each R' independently is H, C₁₋₈ alkyl, C₁₋₈ alkoxy, fluoro, chloro, bromo, iodo, hydroxy, cyano, amino or nitro,
M is -(CH₂)ₒ-, -(CH₂)ₒ-CH=CH-(CH₂)ₚ-, -O-(CH₂)ₒ-, -NH-(CH₂)ₒ-, -O-(CH₂)ₒ-CH=CH-(CH₂)ₚ-O-, -NH-(CH₂)ₒ-CH=CH-(CH₂)ₚ-NH- or -CH₂-CHOH-CHOH-CH₂-, wherein o and p each independently are an integer from 0 to 9,
f is 0 or 1,
R₈ is -(CH₂)ₐ-, -(CH₂)ₐ-O-, -(CH₂)ₐ-S- or -(CH₂)ₐ-NH-, wherein a is an integer from 1 to 8,
R₉ is C₁₋₈ alkyl, C₁₋₈ alkoxy, C₁₋₈ alkylthio or C₁₋₈ alkylamino,
R₁₀ and R₁₁ each independently are C₁₋₄ alkyl, -CONR^{a}R^{b}, -CH₂NR^{a}R^{b}, -NR^{a}R^{b}, -NR^{a}C(=O)-R^{b}, - CH₂NR^{a}C(=O)-R^{b}, -C(=O)OCH₃ or -O-(CH₂)ₑ-OH, wherein R^{a} and R^{b} each independently are H, hydroxyl or C₁₋₄ alkyl, and e is an integer from 1 to 9,
R₁₂ and R₁₃ each independently are -(CH₂)_{g}-CH₃, -O-(CH₂)_{g}-OH, -O-(CH₂)_{g}-CH₃, -CONH₂, 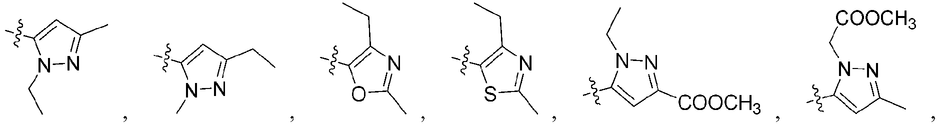 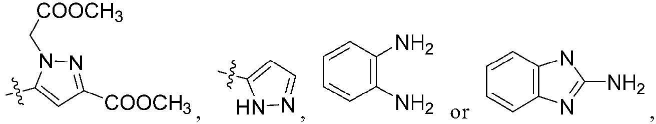 wherein g is a number from 1 to 9.

In some embodiments, in the compound represented by Formula I, R₁₀ is ortho, meta, or para of R₈; and/or R₁₁ is ortho, meta, or para of R₉.

In some embodiments, in the compound represented by Formula I, R₁₀ is ortho of R₈, and R₁₁ is ortho of R₉.

In some embodiments, the compound represented by Formula I has a structure represented by Formula I-1, 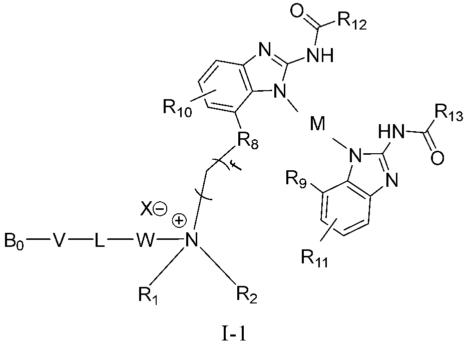
wherein X, R₁, R₂, f, R₈, R₉, R₁₀, R₁₁, R₁₂, R₁₃, and d are as defined in any embodiment of the present application,
B₀-V-L-W- represents a linker, for conjugating the -NR₁R₂- group to a biological macromolecule or a fragment thereof, wherein
B₀ is selected from the group consisting of 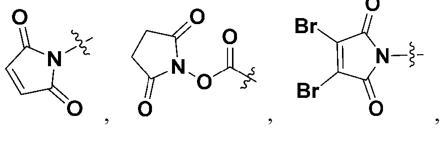 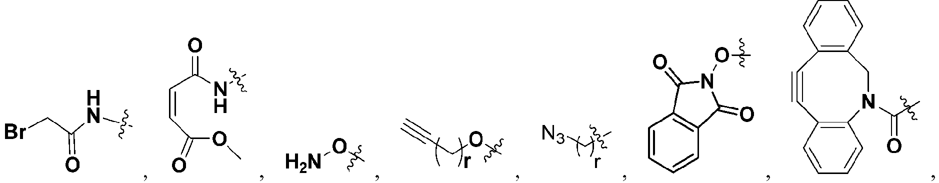 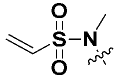 and H, wherein r is an integer from 1 to 4;
V is 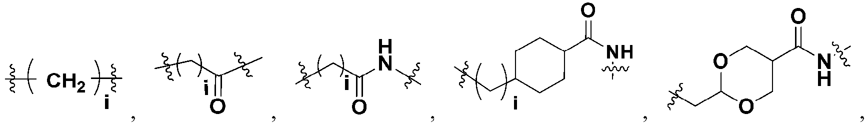 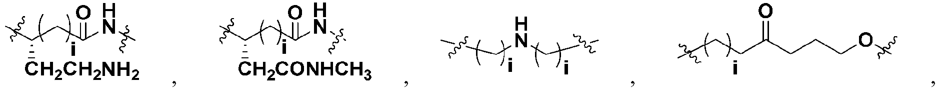 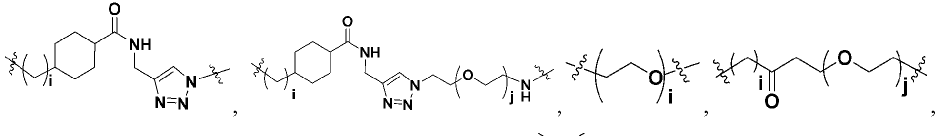 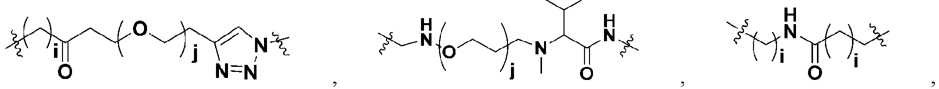 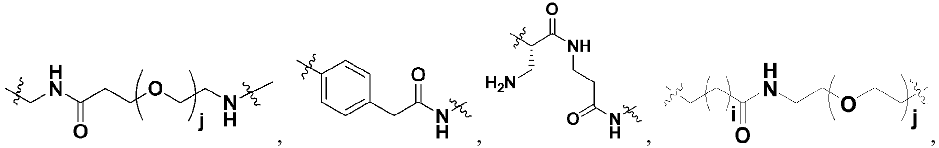 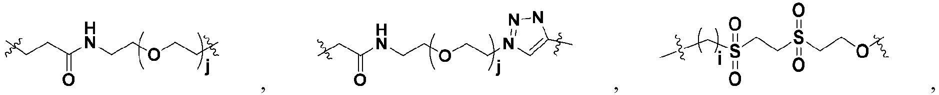 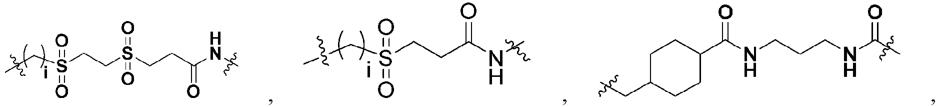 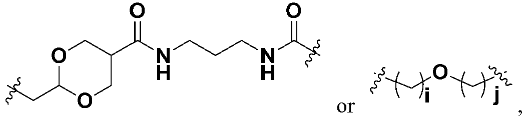 wherein each i independently is an integer from 0 to 12, each j independently is an integer from 0 to 12, or V is absent;
L is -(CH₂CH₂O)ₖ-(CH₂)ₗ-, -CHZ-, -(CH₂)ₘ -, or 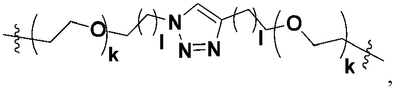 wherein Z is -(CH₂)ₙ-NHC(O)-(CH₂CH₂O)ₖ-CH₃, each k independently is an integer from 0 to 12, each l independently is an integer from 0 to 12, n is an integer from 0 to 12, m is an integer from 0 to 30, or L is absent;
W is 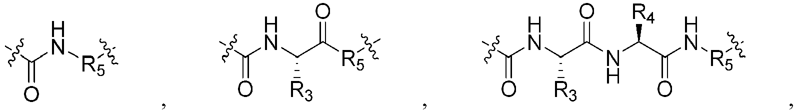 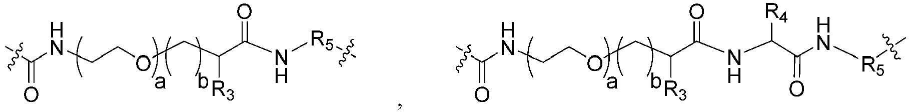 or 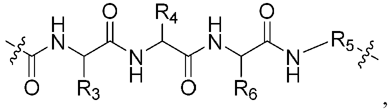 wherein R₃, R₄ and R₆ each independently are H, methyl, ethyl, n-propyl, iso-propyl, n-butyl, sec-butyl, iso-butyl, tert-butyl, phenyl, cyclobutyl, cyclopentyl, cyclohexyl, -CH₂(C₆H₅), -(CH2)_{c}-NH₂, -(CH₂)_{c}-NHC(NH)NH₂, -CHCH(CH₃)CH₃ or -(CH₂)_{c}-NHC(=O)NH₂, or 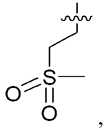 wherein c is a number from 0 to 8; R₅ is 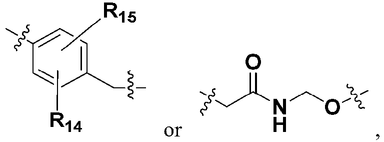 R₁₄ and R₁₅ each independently are H, methyl, ethyl, n-propyl, iso-propyl, n-butyl, sec-butyl, iso-butyl, tert-butyl, phenyl, cyclobutyl, cyclopentyl, cyclohexyl, -CH₂(C₆H₅), -(CH₂)ₕ-NH₂ or -(CH₂)ₕ, and each h independently is a number from 0 to 8.

In some embodiments, in the compound represented by Formula I, X is Cl⁻, Br⁻, I⁻, NO₃⁻, HCOO⁻, CH₃COO⁻, CF₃COO⁻, SO₄²⁻, PO₄,³⁻, HPO₄²⁻ or H₂PO₄⁻. In some embodiments, in the compound represented by Formula I, X is Cl⁻, Br⁻, I⁻, NO₃⁻, HCOO⁻, CH₃COO⁻, or CF₃COO⁻. In some embodiments, in the compound represented by Formula I, X is Cl⁻, HCOO⁻, CH₃COO⁻, or CF₃COO⁻. In some embodiments, in the compound represented by Formula I, X is Cl⁻, Br⁻, or I⁻. In some embodiments, in the compound represented by Formula I, X is Cl⁻.

In some embodiments, in the compound represented by Formula I, R₁ and R₂ each independently are C₁₋₈ alkyl. In some embodiments, in the compound represented by Formula I, R₁ and R₂ each independently are C₁₋₆ alkyl. In some embodiments, in the compound represented by Formula I, R₁ and R₂ each independently are C₁₋₄ alkyl. In some embodiments, in the compound represented by Formula I, R₁ and R₂ each independently are methyl, ethyl, n-propyl, iso-propyl, n-butyl, iso-butyl, tert-butyl, n-pentyl, tert-pentyl, neopentyl, or n-hexyl.

In some embodiments, in the compound represented by Formula I, R₁ and R₂, together with the nitrogen atom to which they are linked, form 5- to 7-membered heterocycloalkyl, wherein the heterocycloalkyl contains one N atom and one, two or three other heteroatoms selected from the group consisting of N, O or S atom, the heterocyclyl is optionally substituted with one, two, three or four R', and each R' independently is H, C₁₋₈ alkyl, C₁₋₈ alkoxy, fluoro, chloro, bromo, iodo, hydroxy, cyano, amino or nitro. In some embodiments, in the compound represented by Formula I, R₁ and R₂, together with the nitrogen atom to which they are linked, form pyrrolidinyl, piperazinyl, piperidinyl, morpholinyl, or thiomorpholinyl. In some embodiments, in the compound represented by Formula I, R₁ and R₂, together with the nitrogen atom to which they are linked, form

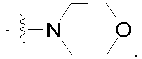

In some embodiments, in the compound represented by Formula I, R₁ and R₂, together with the nitrogen atom to which they are linked, form 5- to 7-membered heteroaryl, wherein the heteroaryl contains one N atom and one, two or three other heteroatoms selected from the group consisting of N, O or S atom, the heteroaryl is optionally substituted with one, two, three or four R', and each R' independently is H, C₁₋₈ alkyl, C₁₋₈ alkoxy, fluoro, chloro, bromo, iodo, hydroxy, cyano, amino or nitro. In some embodiments, in the compound represented by Formula I, R₁ and R₂, together with the nitrogen atom to which they are linked, form imidazolyl, triazolyl, indolyl, tetrazolyl, pyridyl, pteridinyl, pyrimidinyl, quinolinyl, isoquinolinyl, quinazolinyl, quinoxalinyl, or thiazinyl.

In some embodiments, in the compound represented by Formula I, R' is H, C₁₋₆ alkyl, C₁₋₆ alkoxy, fluoro, chloro, bromo, iodo, hydroxy, cyano, amino or nitro. In some embodiments, in the compound represented by Formula I, R' is H, C₁₋₄ alkyl, C₁₋₄ alkoxy, fluoro, chloro, bromo, iodo, hydroxy, cyano, amino or nitro. In some embodiments, in the compound represented by Formula I, R' is H. In some embodiments, in the compound represented by Formula I, R' is methyl, ethyl, n-propyl, iso-propyl, n-butyl, iso-butyl, tert-butyl, n-pentyl, tert-pentyl, neopentyl, or n-hexyl. In some embodiments, in the compound represented by Formula I, R' is methyl, ethyl or n-propyl. In some embodiments, in the compound represented by Formula I, R' is methoxy, ethoxy, propoxy or butoxy. In some embodiments, in the compound represented by Formula I, R' is fluoro, chloro, bromo or iodo. In some embodiments, in the compound represented by Formula I, R' is fluoro, chloro or bromo. In some embodiments, in the compound represented by Formula I, R' is cyano, amino or nitro.

In some embodiments, in the compound represented by Formula I, M is -(CH₂)ₒ-CH=CH-(CH₂)ₚ-, wherein o and p each independently are an integer from 0 to 9.

In some embodiments, in the compound represented by Formula I, o and p each independently are an integer from 1 to 6, such as 1, 2, 3, 4, 5 or 6.

In some embodiments, in the compound represented by Formula I, f is 0. In some embodiments, in the compound represented by Formula I, f is 1.

In some embodiments, in the compound represented by Formula I, R₈ is -(CH₂)ₐ-O-, -(CH₂)ₐ-S- or - (CH₂)ₐ-NH-. In some embodiments, in the compound represented by Formula I, R₅ is -(CH₂)ₐ-O-.

In some embodiments, in the compound represented by Formula I, a is an integer from 1 to 6, such as 1, 2, 3, 4, 5 or 6.

In some embodiments, in the compound represented by Formula I, R₉ is C₁₋₈ alkoxy, C₁₋₈ alkylthio or C₁₋₈ alkylamino. In some embodiments, in the compound represented by Formula I, R₉ is C₁₋₈ alkoxy. In some embodiments, in the compound represented by Formula I, R₉ is C₁₋₆ alkoxy. In some embodiments, in the compound represented by Formula I, R₉ is C₁₋₄ alkoxy. In some embodiments, in the compound represented by Formula I, R₉ is methoxy, ethoxy, n-propoxy, iso-propoxy, n-butoxy, tert-butoxy, sec-butoxy, n-pentyloxy, n-hexyloxy or 1,2-dimethylbutoxy. In some embodiments, in the compound represented by Formula I, R₉ is methoxy, ethoxy, n-propoxy or n-butoxy. In some embodiments, in the compound represented by Formula I, R₉ is C₁₋₆ alkylthio. In some embodiments, in the compound represented by Formula I, R₉ is C₁₋₄ alkylthio. In some embodiments, in the compound represented by Formula I, R₉ is methylthio, ethylthio, propylthio or butylthio. In some embodiments, in the compound represented by Formula I, R₉ is C₁₋₆ alkylamino. In some embodiments, in the compound represented by Formula I, R₉ is C₁₋₄ alkylamino. In some embodiments, in the compound represented by Formula I, R₉ is methylamino, ethylamino, propylamino or butylamino.

In some embodiments, in the compound represented by Formula I, R₁₀ and R₁₁ each independently are -CONR^{a}R^{b}, -CH₂NR^{a}R^{b} or -CH₂NR^{a}C(=O)-R^{b}. In some embodiments, in the compound represented by Formula I, R₁₀ and R₁₁ each independently are -CONR^{a}R^{b}.

In some embodiments, in the compound represented by Formula I, R^{a} and R^{b} each independently are H or C₁₋₄ alkyl. In some embodiments, in the compound represented by Formula I, R^{a} and R^{b} each independently are H. In some embodiments, in the compound represented by Formula I, R^{a} and R^{b} each independently are methyl, ethyl, n-propyl or n-butyl.

In some embodiments, in the compound represented by Formula I, R₁₂ and R₁₃ each independently are 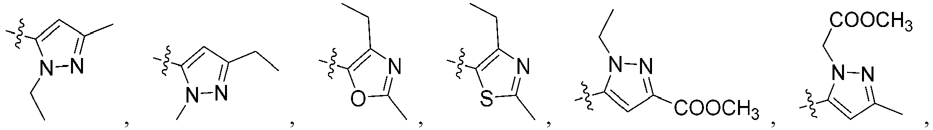 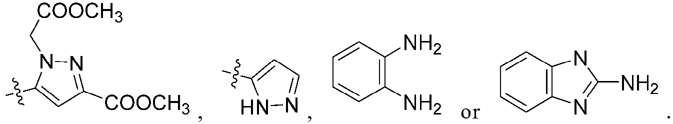 In some embodiments, in the compound represented by Formula I, R₁₂ and R₁₃ each independently are 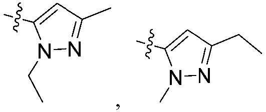 or 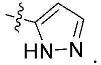 In some embodiments, in the compound represented by Formula I, R₁₂ and R₁₃ each independently are

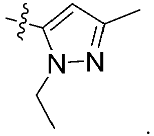

In some embodiments, in the compound represented by Formula I, B₀ is 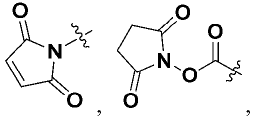 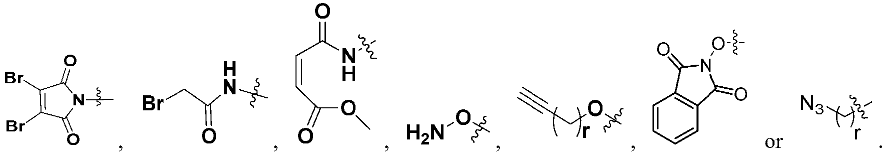 In some embodiments, in the compound represented by Formula I, B₀ is 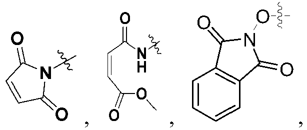 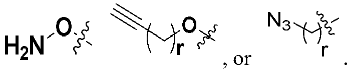 In some embodiments, in the compound represented by Formula I, B₀ is 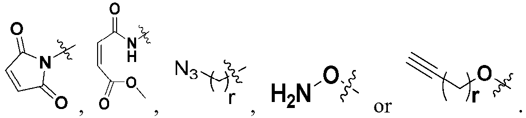 In some embodiments, in the compound represented by Formula I, B₀ is 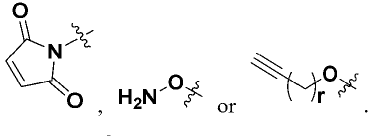 In some embodiments, in the compound represented by Formula I, B₀ is

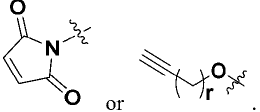

In some embodiments, in the compound represented by Formula I, r is 1, 2, 3 or 4. In some embodiments, in the compound represented by Formula I, r is 1 or 2;

In some embodiments, in the compound represented by Formula I, V is 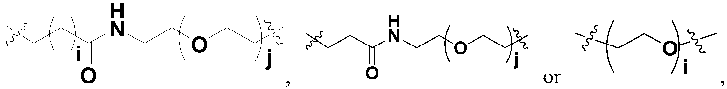 wherein i and j are as defined in any embodiment of the present application.

In some embodiments, in the compound represented by Formula I, i and j each independently are 1, 2, 3, 4, 5, 6, 7, 8, 9 or 10. In some embodiments, in the compound represented by Formula I, i and j each independently are 1, 2, 3, 4, 5, 6, 7 or 8. In some embodiments, in the compound represented by Formula I, i and j each independently are 1, 2, 3, 4, 5 or 6.

In some embodiments, in the compound represented by Formula I, Lis -(CH₂CH₂O)ₖ-(CH₂)₁-, -CHZ-, or -(CH₂)ₘ-, wherein Z is -(CH₂)ₙ-NHC(O)-(CH₂CH₂O)ₖ-CH₃, and k, l, m, and n are as defined in any embodiment of the present application.

In some embodiments, in the compound represented by Formula I, each k independently is an integer from 0 to 10, such as 0, 1, 2, 3, 4, 5, 6, 7, 8, 9 or 10.

In some embodiments, in the compound represented by Formula I, each 1 independently is an integer from 0 to 10, such as 0, 1, 2, 3, 4, 5, 6, 7, 8, 9 or 10.

In some embodiments, in the compound represented by Formula I, n is an integer from 0 to 10, such as 0, 1, 2, 3, 4, 5, 6, 7, 8, 9 or 10.

In some embodiments, in the compound represented by Formula I, m is an integer from 0 to 20. In some embodiments, in the compound represented by Formula I, m is an integer from 0 to 16, such as 0, 1, 2, 3, 4, 5, 6, 7, 8, 9, 10, 11, 12, 13, 14, 15, or 16.

In some embodiments, in the compound represented by Formula I, L is absent.

In some embodiments, in the compound represented by Formula I, W is 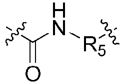 or 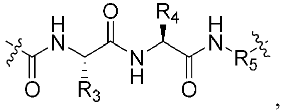 wherein R₃, R₄ and R₅ are as defined in any embodiment of the present application.

In some embodiments, in the compound represented by Formula I, R₃ and R₄ each independently are methyl, ethyl, n-propyl, iso-propyl or -(CH₂)_{c}-NHC(=O)NH₂, wherein c is as defined in any embodiment of the present application.

In some embodiments, in the compound represented by Formula I, R₅ is 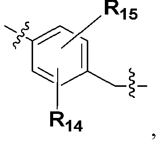 wherein R₁₄ and R₁₅ are as defined in any embodiment of the present application.

In some embodiments, in the compound represented by Formula I, R₃ and R₄ each independently are methyl, iso-propyl or -(CH₂)_{c}- NHC(=O)NH₂.

In some embodiments, in the compound represented by Formula I, c is an integer from 0 to 6, such as 0, 1, 2, 3, 4, 5, or 6.

In some embodiments, in the compound represented by Formula I, R₁₄ and R₁₅ each independently are H, methyl, ethyl, n-propyl, iso-propyl, n-butyl, sec-butyl, iso-butyl or tert-butyl. In some embodiments, in the compound represented by Formula I, R₁₄ and R₁₅ each independently are H, methyl, ethyl or n-propyl. In some embodiments, in the compound represented by Formula I, R₁₄ and R₁₅ each independently are H or methyl. In some embodiments, in the compound represented by Formula I, R₁₄ and R₁₅ each independently are H.

In some embodiments, in the compound represented by Formula I, -V-L-W- is 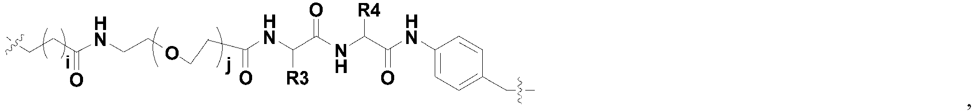 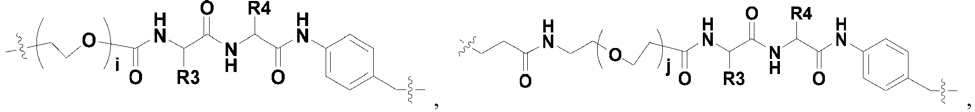 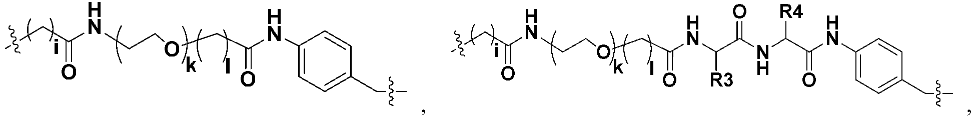 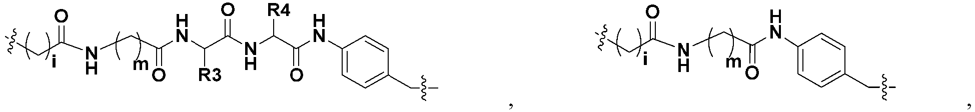 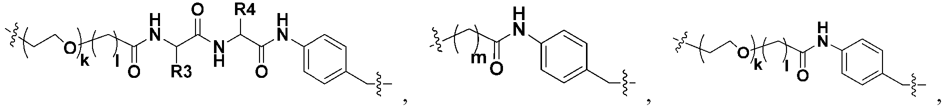 or 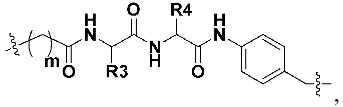 wherein i, j, k, l, m, R₃, and R₄ are as defined in any embodiment of the present application.

In some embodiments, in the compound represented by Formula I, -V-L-W- is 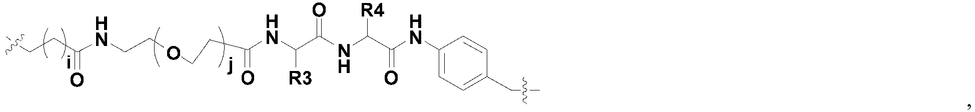 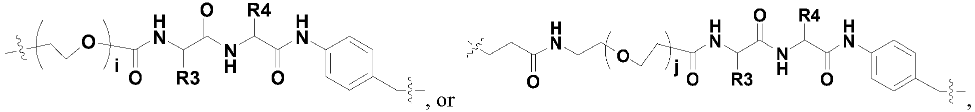 wherein i, j, k, l, m, R₃, and R₄ are as defined in any embodiment of the present application.

In some embodiments, the compound represented by Formula I has a structure presented by Formula I-2, 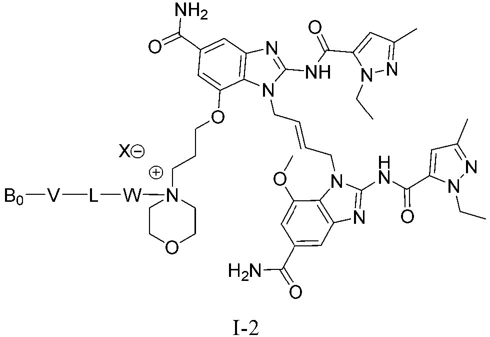 wherein B₀, X, V, L, and W are as defined in any embodiment of the present application.

In some embodiments, the compound represented by Formula I is selected from the group consisting of: 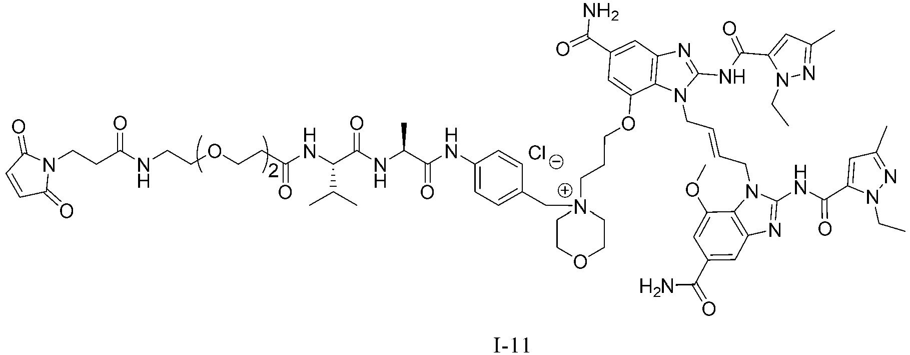 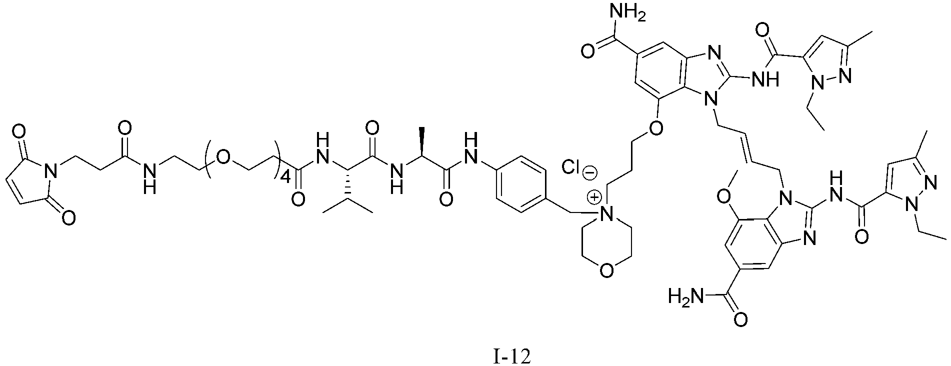 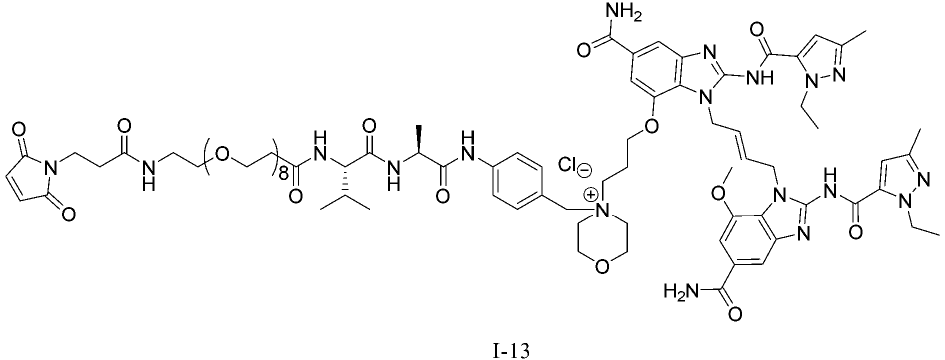 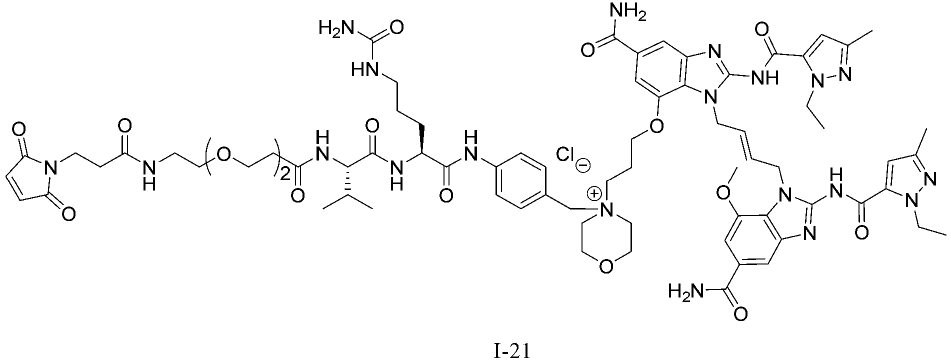 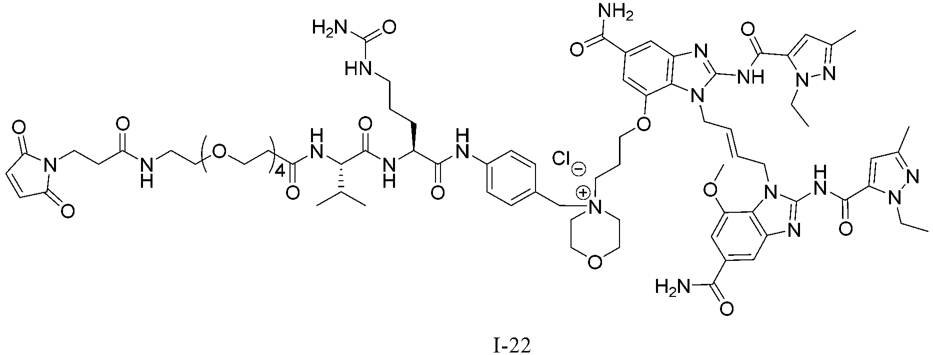 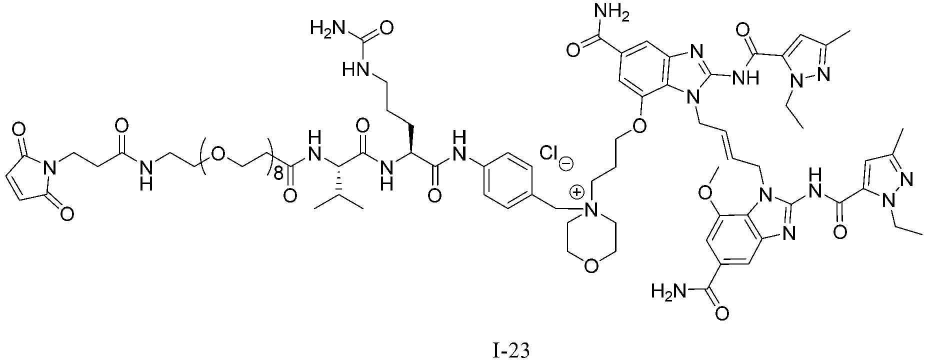 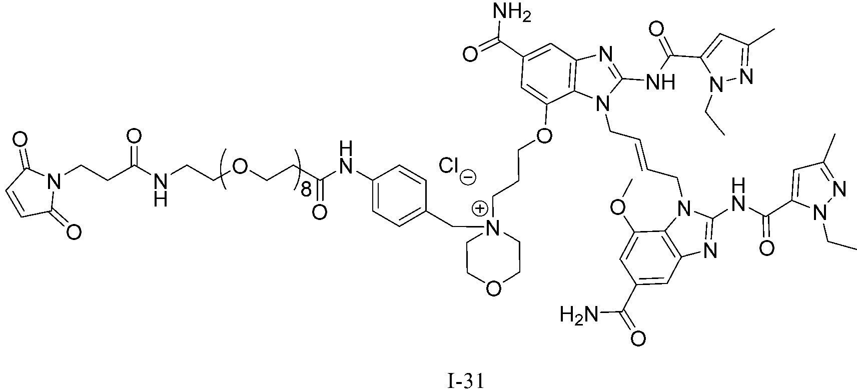 and

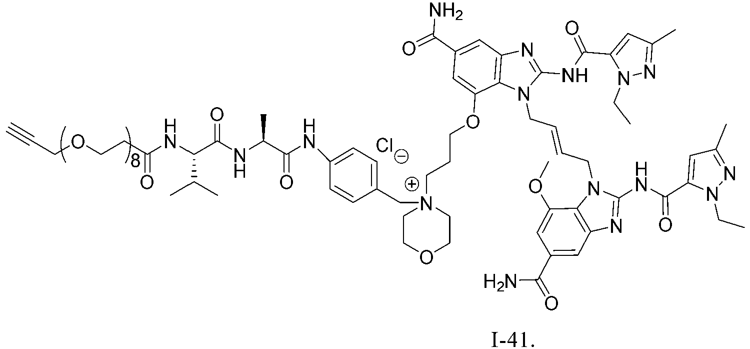

### Preparation Method

The third aspect of the present application relates to the use of the intermediate compound in the preparation of an antibody-drug conjugate (e.g., an immune-stimulating antibody conjugate).

The immune-stimulating antibody conjugate according to the present application may be prepared by conjugating the intermediate compound to a biological macromolecule or a fragment thereof represented by A by methods well known in the art.

In some embodiments, the immune-stimulating antibody conjugate may be illustratively prepared by the following synthetic route, 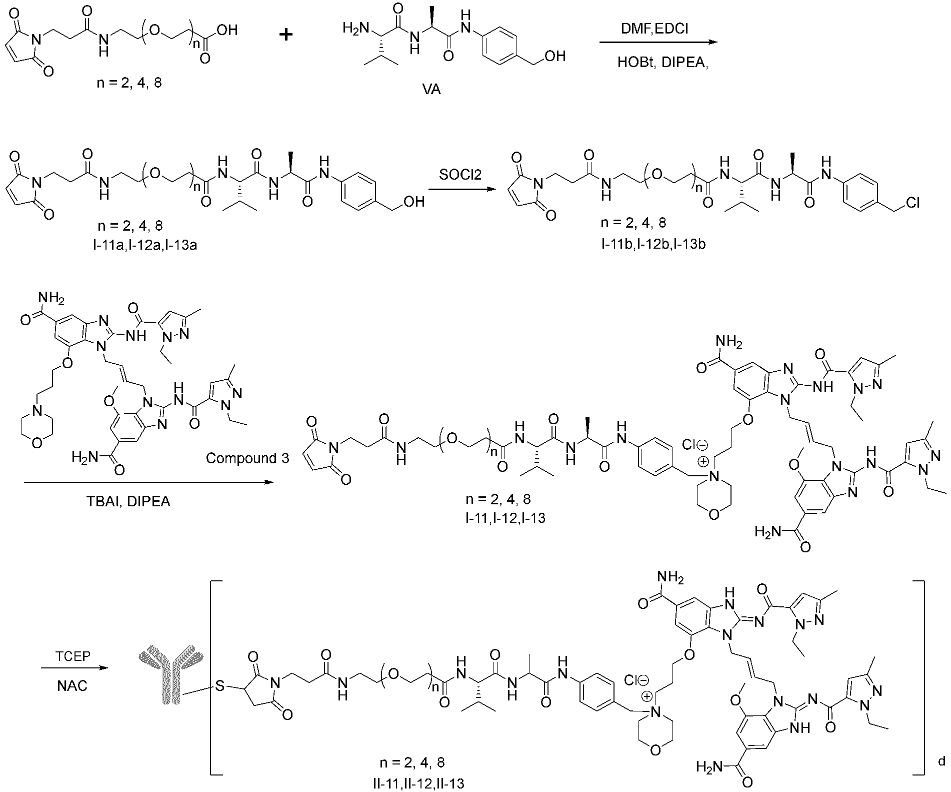 wherein the reaction reagents and the reaction conditions are as follows: (1) maleimide-polyethylene glycol-carboxylic acid and compound VA are used as starting materials, and anhydrous DMF is used as a solvent, the reaction is carried out in the presence of EDCI, HOBt and DIPEA to afford compounds I-11a, I-12a and I-13a; (2) compounds I-11a, I-12a and I-13a react with thionyl chloride, to obtain compounds I-11b, I-12b and I-13b; (3) compounds I-11b, I-12b and I-13b react with Compound 3 in anhydrous DMF under the action of DIPEA and TBAI, to obtain compounds I-11, I-12 and I-13; (4) compounds I-11, I-12 and I-13 are further conjugated to an antibody to obtain final ISAC products II-11, II-12 and II-13.

In some embodiments, the immune-stimulating antibody conjugate may also be illustratively prepared by the following synthetic route, 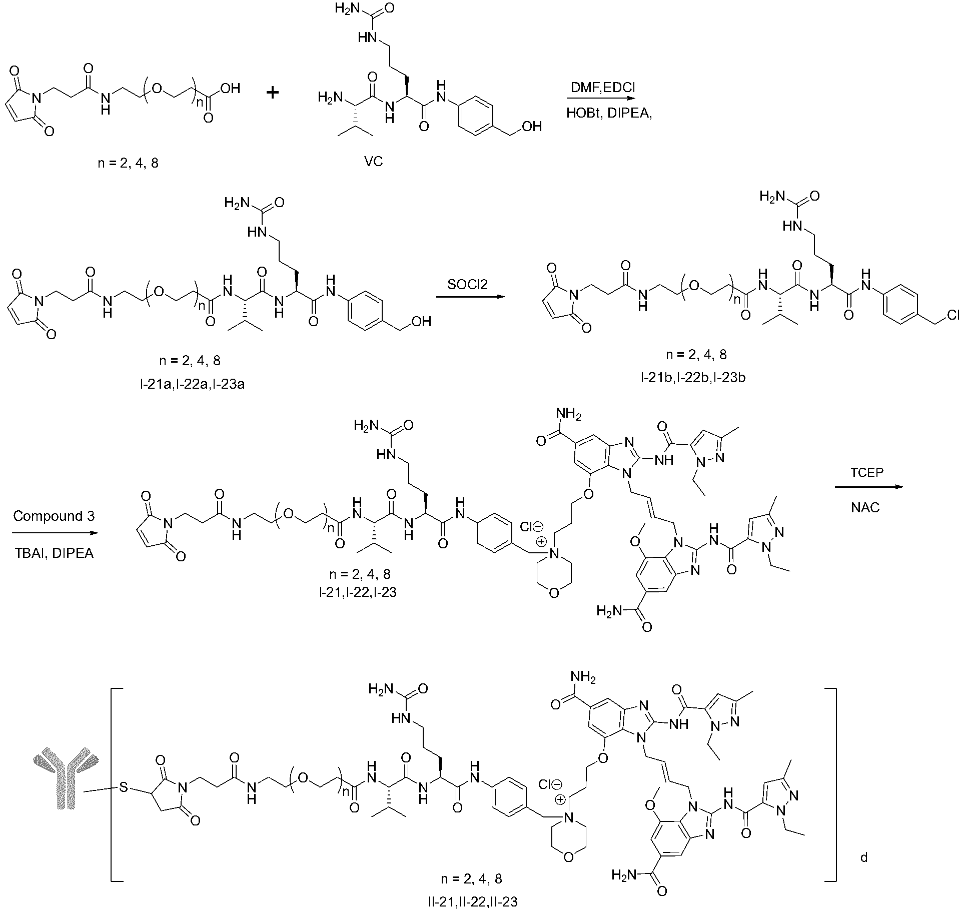 wherein the reaction reagents and the reaction conditions are as follows: (1) maleimide-polyethylene glycol-carboxylic acid and compound VC are used as starting materials, and anhydrous DMF as a solvent, the reaction is carried out in the presence of EDCI, HOBt and DIPEA, to afford compounds I-21a, I-22a and I-23a; (2) compounds I-21a, I-22a and I-23a react with thionyl chloride, to obtain compounds I-21b, I-22b and I-23b; (3) compounds I-21b, I-22b and I-23b react with Compound 3 in anhydrous DMF under the action of DIPEA and TBAI, to obtain compounds I-21, I-22 and I-23; (4) compounds I-21, I-22 and I-23 are further conjugated to an antibody to obtain final ISAC products II-21, II-22 and II-23.

In some embodiments, the immune-stimulating antibody conjugate may also be illustratively prepared by the following synthetic route, 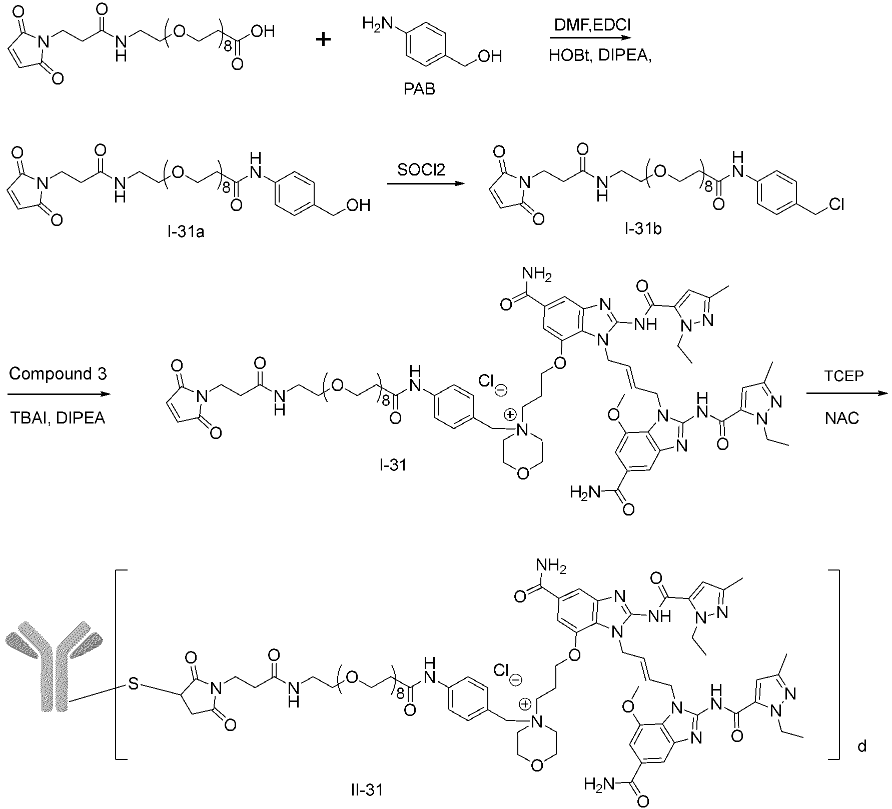 wherein the reaction reagents and the reaction conditions are as follows: (1) maleimide-polyethylene glycol-propanoic acid and compound PAB are used as starting materials, and anhydrous DMF is used as a solvent, the reaction is carried out in the presence of EDCI, HOBt and DIPEA, to afford compound I-31a; (2) compound I-31a reacts with thionyl chloride, to obtain compound I-31b; (3) compound I-31b reacts with Compound 3 in anhydrous DMF under the action of DIPEA and TBAI, to obtain compound I-31; (4) compound I-31 is further conjugated to an antibody to obtain final ISAC product II-31.

In some embodiments, the immune-stimulating antibody conjugate may also be illustratively prepared by the following synthetic route, 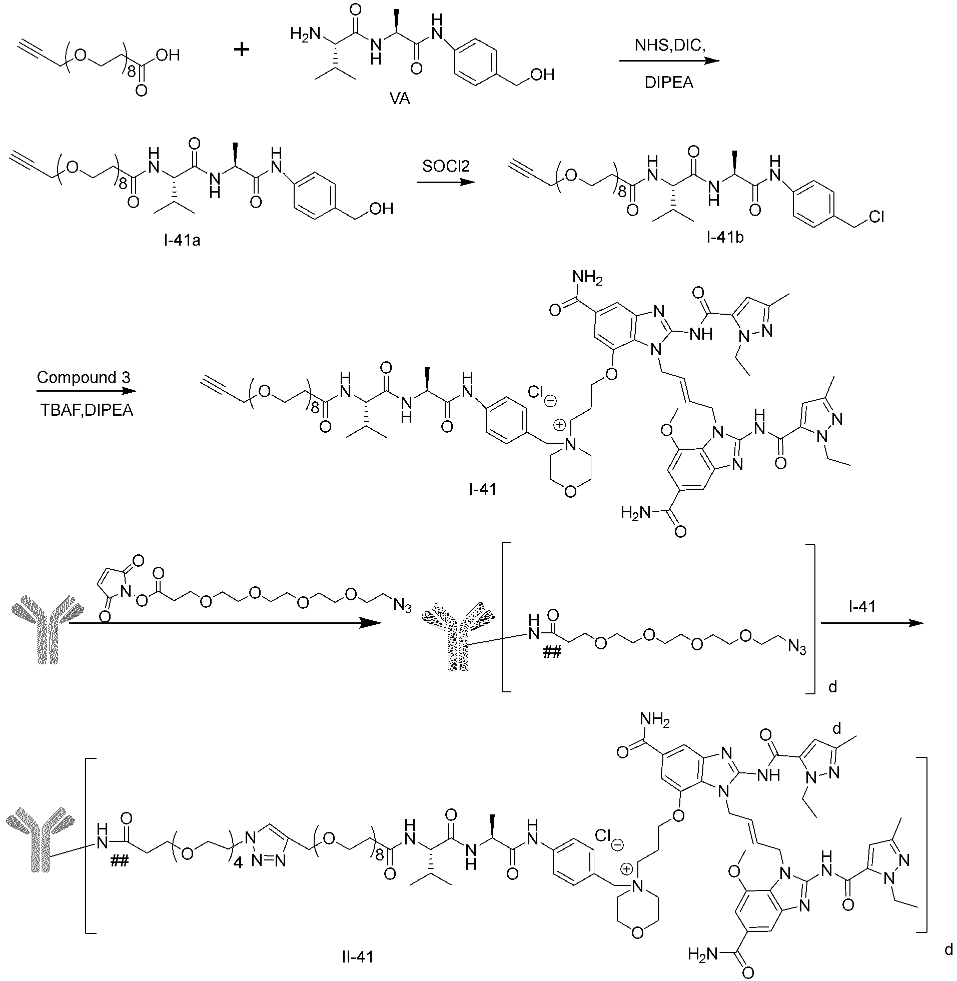 wherein the reaction reagents and the reaction conditions are as follows: (1) propynyl-octa(polyethylene glycol)-carboxylic acid and compound VA are used as starting materials, and anhydrous DMF is used as a solvent, the reaction is carried out in the presence of NHS, DIC and DIPEA, to afford compound I-41a; (2) compound I-41a reacts with thionyl chloride, to obtain compound I-41b; (3) compound I-41b reacts with Compound 3 in anhydrous DMF under the action of DIPEA and TBAI, to obtain compound I-41; (4) compound I-41 is further conjugated to an antibody via a click chemical reaction to obtain final ISAC product II-41.

### Pharmaceutical Composition or Vaccine

The fourth aspect of the present application relates to a pharmaceutical composition or vaccine, comprising the compound represented by Formula II, a tautomer, a geometric isomer, an optical isomer, a polymorph or a pharmaceutically acceptable hydrate or solvate thereof, and optionally one or more pharmaceutically acceptable carriers or excipients.

In some embodiments, in the pharmaceutical composition or vaccine, the compound represented by Formula II, a tautomer, a geometric isomer, an optical isomer, a polymorph or a pharmaceutically acceptable hydrate or solvate thereof is present in an effective amount, e.g., in a therapeutically effective amount, or in a prophylactically effective amount.

The pharmaceutical composition or vaccine may be administered via routes, such as oral or parenteral administration, including intravenous injection, intraperitoneal injection, intramuscular injection, subcutaneous injection and intranasal drip.

The pharmaceutical composition or vaccine may be formulated into various dosage forms by conventional methods known in the art, including but not being limited to tablets, capsules, solutions, suspensions, particles or injections.

### Uses

The immune-stimulating antibody conjugate according to the present application can be used for treating or preventing a STING-mediated disease or condition, specifically for treating and preventing a disease or condition or alleviating the severity of the disease or condition, wherein the disease or condition is selected from the group consisting of a tumor, a pathogen infection, a disease caused by a pathogen infection, a hematological disease, a metabolic disease, an inflammation, an allergic disease, an autoimmune disease, a precancerous syndrome, tumor metastasis, and a cardiovascular disease.

In some embodiments, the tumor is cancer, lymphoma, lymphoid neoplasm, blastoma, sarcoma, or leukemia.

In some embodiments, the pathogen infection is a bacterial infection or a viral infection.

In some embodiments, the inflammation is an inflammation of any tissue and organ of a body, including musculoskeletal inflammation, vascular inflammation, neuroinflammation, inflammation of the digestive system, ocular inflammation, inflammation of the reproductive system, and other inflammations.

In some embodiments, the viral infection is an infection caused by severe acute respiratory syndrome coronavirus (SARS-CoV), severe acute respiratory syndrome coronavirus 2 (SARS-CoV-2) or a variant strain thereof, influenza A virus, influenza B virus, parainfluenza virus, rhinovirus, dengue virus, Zika virus, yellow fever virus, or hepatitis B virus.

In some embodiments, the bacterial infection is an infection caused by a Gram-positive bacterium, a penicillin-resistant bacterium, *Staphylococcus aureus, mycobacterium tuberculosis,* drug-resistant *mycobacterium tuberculosis, clostridium difficile,* or *chlamydia trachomatis.*

In some embodiments, the disease caused by the viral infection is COVID-19.

In some embodiments, the disease caused by the pathogen infection is a disease caused by a bacterial infection or a viral infection.

In some embodiments, the disease caused by the bacterial infection is selected from the group consisting of bacterial pulmonary infection, sepsis caused by infections such as *Staphylococcus aureus* infection, *pyocyania* infection, or *clostridium difficile* infection, tuberculosis, bacterial ocular infection, infection of heart, brain or skin, gastrointestinal tract infection, bacterial meningitis, or abscess in any organ (such as muscle, liver, meninges, or lung), cellulitis, bacteremia, skin necrosis, eyelid infection, ocular infection, neonatal conjunctivitis, osteomyelitis, impetigo, ecthyma, scalded skin syndrome, food poisoning, pneumonia, surgical infection, urinary tract infection, burn wound infection, meningitis, endocarditis, septicemia, toxic shock syndrome, and septic arthritis.

In some embodiments, the cancer is selected from the group consisting of: breast cancer (e.g., HER2 positive breast cancer); squamous cell carcinoma (e.g., epithelial squamous cell carcinoma); lung cancer, including small cell lung cancer, non-small cell lung cancer, lung adenocarcinoma and lung squamous cell carcinoma; peritoneal cancer; liver cancer; gastric cancer; gastrointestinal cancer; pancreatic adenocarcinoma; glioblastoma; cervical cancer; ovarian cancer; bladder cancer; urethral carcinoma; hepatocellular carcinoma; intestinal cancer; colon cancer; rectal cancer; colorectal cancer; endometrial cancer; uterine cancer; salivary gland cancer; renal cancer; prostate cancer; vulvar cancer; thyroid cancer; anal cancer; penile cancer; melanoma; multiple myeloma and B-cell lymphoma; brain cancer; gallbladder cancer; esophageal cancer; cholangiocarcinoma; head and neck cancer and associated metastatic tumors.

In some embodiments, the autoimmune disease includes, but is not limited to, STING-associated vasculopathy with onset in infancy (SAVI), Aicardi Goutières Syndrome (AGS), chilblain lupus, ataxia telangiectasia (also known as Louis-Bar syndrome), retinal vasculopathy with cerebral leukoencephalopathy (RCVL), systemic lupus erythematosus (SLE), cutaneous lupus, lupus nephritis, psoriasis, diabetes mellitus (including insulin dependent diabetes mellitus (IDDM)), dermatomyositis, human immunodeficiency virus (HIV), AIDS, polymyositis, systemic sclerosis (scleroderma), and Sjogren's syndrome (SS), rheumatoid arthritis, psoriatic arthritis, polyarthritis, osteoarthritis, myasthenia gravis, polyarteritis nodosa, vasculitis, cutaneous vasculitis, anti-neutrophil cytoplasmic antibody (ANCA)-related vasculitis, Henoch-Schönlein purpura, autoimmune hepatitis, primary sclerosing cholangitis, granulomatosis with polyangiitis, microscopic polyangiitis, Behcet's disease, spondylitis, giant cell arteritis, polymyalgia rheumatica, Raynaud's phenomenon, primary biliary cirrhosis, primary vasculitis of the central nervous system, neuromyelitis optica, and mixed connective tissue disease.

### Definitions

As used herein, the term "linker" refers to a molecule having two reactive terminuses, one terminus of which may be conjugated to a macromolecule (e.g., an antibody or an antigen-binding fragment thereof, a polypeptide or a protein capable of specifically binding to a target ) and the other terminus of which is used for conjugating a small-molecule active compound, such as a cytotoxin or an immunomodulator. The macromolecule-conjugating terminus of the linker is generally a site capable of conjugating an antibody via the thiol group of cysteine residues or the amino group of lysine residues on the antibody, and the small-molecule active compound-conjugating terminus of the linker is generally an active site capable of reacting with a cytotoxin molecule via thiol, amino, carboxyl, hydroxyl or other functional group on the cytotoxin molecule. When the term "linker" is used to describe a linker in a conjugated form, since the linker has reacted with one or both of the antibody and the cytotoxin to form a covalent bond, it may no longer comprise one or both reactive terminus sites (such as a leaving group of a thiol-reactive group, and a leaving group of an amine-reactive group).

As used herein, the term "immunomodulator" refers to a substance capable of modulating the immune system, and exerting activating or inhibitory effect on the immune response of a body, which includes immunostimulants and immunosuppressants. Immunomodulators of particular interest in the present application include STING agonists, glucocorticoids, immunostimulating cytokines.

As used herein, the term "cytotoxin" refers to those molecules that exert cytotoxic effects on cancer cells once released inside the cancer cells. Cytotoxins of particular interest in the present application include monomethyl auristatin E (MMAE), auristatin, maytansine or a derivative thereof (such as maytansinoids, DM1, DM3, DM4), calicheamicin, duocarmycin, doxorubicin, camptothecin, or PBD cytotoxins.

As used herein, the term "antibody-drug conjugate" or "ADC" refers to a product formed by conjugating multiple (typically 1 to 8) molecules of cytotoxins to an antibody molecule via a linker, respectively. The antibody conjugated to one or more cytotoxins is generally a monoclonal antibody with selectivity for a specific antigen.

As used herein, the term "immune-stimulating antibody conjugate" or "ISAC" is a drug conjugate form. An ISAC consists of an antibody, a linker, and a small-molecule immune agonist (an innate immune agent or immunomodulator); the antibody is generally a monoclonal antibody with selectivity for a specific antigen.

As used herein, the term "antibody" refers to an immunoglobulin molecule generally consisting of two pairs of polypeptide chains (each pair having one "light" (L) chain and one "heavy" (H) chain). The light chains of an antibody can be classified as κ and λ light chains. The heavy chains of an antibody can be classified as µ, δ, γ, α or ε chains, which respectively define the antibody isotypes IgM, IgD, IgG, IgA, and IgE. Within both light and heavy chains, variable and constant regions are connected by a "J" region of about 12 or more amino acids, and heavy chains additionally contain a "D" region of about 3 or more amino acids. Each heavy chain consists of a heavy chain variable region (V_{H}) and a heavy chain constant region (C_{H}). The heavy chain constant region consists of 3 domains (CH1, CH2, and CH3). Each light chain consists of a light chain variable region (V_{L}) and a light chain constant region (C_{L}). The light chain constant region consists of one C_{L} domain. The constant region of an antibody may mediate the binding of the immunoglobulin to host tissues or factors, including various cells of the immune system (e.g., effector cells) and the first component (C1q) of the classical complement system. The V_{H} and V_{L} regions may also be subdivided into regions of high variability (referred to as complementarity determining regions (CDR), interspersed with more conservative regions, referred to as framework regions (FRs). Each V_{H} and each V_{L} consist of three CDR and four FR arranged from the amino terminus to the carboxy terminus in the following order: FR1, CDR1, FR2, CDR2, FR3, CDR3, FR4. The variable regions (V_{H} and V_{L}) of each heavy/light chain pair form antibody-binding sites, respectively. The assignment of amino acids to various regions or domains follows the definition of Kabat Sequences of Proteins of Immunological Interest (National Institutes of Health, Bethesda, Md. (1987 and 1991)), or Chothia & Lesk (1987) J. mol. biol. 196: 901-917; Chothia et al., (1989) Nature 342: 878-883. The term "antibody" is not limited by any particular method for producing antibodies. For example, it includes, a recombinant antibody, a monoclonal antibody and a polyclonal antibody. The antibody may be of different isotypes, such as IgG (e.g., IgG1, IgG2, IgG3 or IgG4 subtype), IgA1, IgA2, IgD, IgE or IgM antibody.

As used herein, the term "Complementarity Determining Region" or "CDR" refers to amino acid residues responsible for antigen binding within the variable region of an antibody. The precise boundaries of these amino acid residues may be defined according to various numbering systems known in the art, for example, according to definitions in the Kabat numbering system (Kabat et al, Sequences of Proteins of Immunological Interest, 5th Ed. Public Health Service, National Institutes of Health, Bethesda, Md., 1991), the Chothia numbering system (Chothia & Lesk (1987) J. mol. biol. 196:901-917; Chothia et al (1989) Nature 342: 878-883) or the IMGT numbering system (Lefranc et al., Dev. Comparat. Immunol. 27: 55-77, 2003). For a given antibody, one skilled in the art will readily identify the CDRs as defined by each numbering system. Furthermore, the correspondence relationship between the different numbering systems is well known to one skilled in the art (see, e.g., Lefranc et al., Dev. Comparat. Immunol. 27: 55-77, 2003).

The CDR contained in the antibody or antigen-binding fragment thereof of the present disclosure may be determined according to various numbering systems known in the art. In some embodiments, the CDR contained in the antibody or antigen-binding fragment thereof of the present disclosure is preferably determined by the Kabat, Chothia, or IMGT numbering system.

As used herein, the term "antigen-binding fragment" of an antibody refers to a polypeptide comprising a fragment of a full-length antibody, which retains the capacity to specifically bind to the same antigen to which the full-length antibody binds, and/or competes with the full-length antibody for the specific binding to the antigen, it is also referred to as an "antigen-binding portion". See generally Fundamental Immunology, Ch. 7 (Paul, W., ed., 2nd edition, Raven Press, N.Y. (1989), which is incorporated herein by reference in its entirety for all purposes. An antigen-binding fragment of an antibody can be produced through recombinant DNA technology or through enzymatic or chemical cleavage of an intact antibody. In some cases, antigen-binding fragments include Fab, Fab', F(ab')₂, Fd, Fv, dAb, and a complementarity determining region (CDR) fragments, single-chain antibodies (e.g., scFv), chimeric antibodies, diabodies, and polypeptides that comprise at least one portion of an antibody sufficient to confer a specific antigen-binding capacity to the polypeptide.

As used herein, the term "Fd fragment" means an antibody fragment consisting of a V_{H} domain and a CH1 domain; the term "Fv fragment" means an antibody fragment consisting of the V_{L} and V_{H} domains of a single arm of an antibody; the term "dAb fragment" means an antibody fragment consisting of a V_{H} domain (Ward et al, Nature 341: 544-546 (1989)); the term "Fab fragment" means an antibody fragment consisting of a V_{L} domain, a V_{H} domain, a CL domain and a CH1 domain; the term "F(ab')₂ fragment" means an antibody fragment comprising two Fab fragments connected by a disulfide bridge at the hinge region; the term "Fab' fragment" means one of two Fab' fragments formed by reducing a F(ab')₂ fragment to cleave the disulfide bond in the hinge region of the F(ab')₂.

In some instances, the antigen-binding fragment of an antibody is a single chain antibody (e.g., scFv), in which the V_{L} domain and V_{H} domain are paired to form a monovalent molecule through a linker that enables them to form a single polypeptide chain (see, e.g., Bird et al, Science 242: 423-426 (1988) and Huston et al, proc. natl. acad. sci. USA 85: 5879-5883 (1988)). Such scFv molecules may have the general structure: NH₂-V_{L}-linker-VH-COOH or NH₂-V_{H}-linker-VL-COOH. Suitable linkers for use in the prior art consist of repeated GGGGS amino acid sequences or variants thereof. For example, a linker having the amino acid sequence (GGGGS)₄ may be used, and variants thereof also may be used (Holliger et al (1993), Proc. Natl. Acad. Sci. USA 90: 6444-6448). Other linkers applicable to the present disclosure are described in Alfthan et al (1995), Protein Eng. 8: 725-731; Choi et al., (2001), Eur. J. Immunol. 31: 94-106; Hu et al., (1996), Cancer Res. 56:3055-3061, Kipriyanov et al., (1999), J. Mol. Biol. 293:41-56 and Roovers et al., (2001), Cancer Immunol.

In some instances, the antigen-binding fragment of an antibody is a diabody, i.e., a divalent antibody, which is a small antibody fragment having two antigen-binding sites. Such fragment comprises a heavy chain variable domain (V_{H}) and a light chain variable domain (V_{L}) linked thereto within an identical polypeptide chain (V_{H}-V_{L} or V_{L}-V_{H}). The V_{H} and V_{L} domains are expressed on a single polypeptide chain, however, the linker is too short to permit pairing between the two domains on the same chain, thereby forcing the two domains to pair with the complementarity domains from another chain, generating two antigen-binding sites (see, e.g., Holliger p. et al, proc. natl. acad. sci. USA 90: 64446448 (1993), and Poljak r. j. et al, Structure 2: 1121 1123 (1994)).

Antigen-binding fragments of antibodies (such as the antibody fragments described above) can be obtained from a given antibody (e.g., monoclonal antibodies ch13, Hu13H2L0, Hu13H2L3, Hu13H3L0, Hu13H3L3, and Hu13H3L5 provided by the present disclosure) using conventional techniques (e.g., recombinant DNA techniques or enzymatic or chemical cleavage) known to one skilled in the art, and the antigen-binding fragments of antibodies are screened for specificity in the same manner as those for intact antibodies.

Herein, unless clearly indicated otherwise in the context, when the term "antibody" is referred to, it includes not only intact antibodies, but also antigen-binding fragments of antibodies.

As used herein, the term "VHH", also referred to as Nanobody (Nb), is a heavy chain single domain antibody containing only a VHH region fragment. It is composed of the heavy chain variable region derived from camelidae animals (camels, llamas, alpacas and close relative species thereof). The crystals of a nanobody have a diameter of 2.5 nm, a length of 4 nm, and a molecular weight of about 12 kDa to 15 kDa.

As used herein, the term "probody" is a recombinant antibody-based drug that is activated by protease cleavage. It mainly consists of three modules: a monoclonal antibody having anti-cancer activity or a fragment of the variable region thereof, a masking peptide conjugated to the N-terminus of the light chain (which blockes the binding between the Fab region of the antibody and antigen), and an protease-cleavable polypeptide spacer connected between the masking polypeptide and the antibody. Probodies can be produced using conventional recombinant antibody manufacturing techniques. The mechanism of action of probody mainly takes advantage of the difference in protease activity between normal tissues and tumor tissues, enabling maximal therapeutic activity of the drug to be exerted in the tumor microenvironment rather than in the normal tissues. In normal tissues, the probody remains structurally intact. Due to the presence of a masking peptide, the antibody fails to bind antigen and exert its effect, meanwhile retaining a relatively long half-life. After the probody enters the tumor microenvironment, the proteases in which cleave the spacer to release the masking peptide, exposing the Fab region of the antibody for normal binding antigen.

As used herein, the term "mAb" or "monoclonal antibody" refers to an antibody or an antibody fragment derived from a population of highly homologous antibody molecules, i.e., a population of substantially identical antibody molecules except for naturally occurring spontaneous mutations. Monoclonal antibodies exhibit high specificity toward a single epitope on an antigen. In contrast to monoclonal antibodies, polyclonal antibodies generally contain at least two different antibodies that typically recognize different epitopes on an antigen. Monoclonal antibodies may be generally obtained by the hybridoma technology that was first reported by Kohler et al (Nature, 256:495, 1975), and may also be obtained by recombinant DNA technology (see, e.g., U.S.P. 4,816,567).

As used herein, the term "chimeric antibody" refers to an antibody, in which a portion of the light chain and/or heavy chain is derived from one antibody (which may originate from a specific species or belong to a specific antibody class or subclass), and another portion of the light chain and/or heavy chain is derived from another antibody (which may originate from the same or a different species or belong to the same or a different antibody class or subclass), nevertheless, such antibody retains binding activity against the target antigen (U.S.P 4,816,567 to Cabilly et al.; Morrison et al., Proc. Natl. Acad. Sci. USA, 81:6851-6855 (1984)).

As used herein, the term "humanized antibody" refers to an antibody or an antibody fragment obtained by substituting all or part of the CDRs of a humanized immunoglobulin (acceptor antibody) with the CDRs of a non-human antibody (donor antibody), wherein the donor antibody may be a non-human antibody (e.g., murine, rat, or rabbit antibody) having desired specificity, affinity, or reactivity. In addition, some amino acid residues of the framework region (FR) of the acceptor antibody may also be substituted with the corresponding amino acid residues of the non-human antibody or other antibodies, to further improve or optimize antibody performance. For additional details regarding humanized antibodies, see, for example, Jones et al., Nature, 321:522-525 (1986); Reichmann et al., Nature, 332:323-329 (1988); Presta, Curr. Op. Struct. Biol., 2:593-596 (1992); and Clark, Immunol. Today 21: 397-402 (2000).

As used herein, the term "multi-specific antibody" refers to an antibody having binding specificity for at least two different epitopes on the same antigen or on different antigens. A multi-specific antibody may be a full-length antibody or a fragment thereof. The term "bispecific antibody" refers to an antibody having binding specificity for two different antigens or capable of recognizing two different epitopes a single antigen.

As used herein, the term "human antibody" or "fully human antibody" includes antibodies having variable regions and constant regions (if present) derived from human germline immunoglobulin sequences. A human antibody may contain amino acid residues that are not encoded by human germline immunoglobulin sequences (e.g., mutations introduced via random or site-directed mutagenesis in vitro or somatic mutation in vivo). Nevertheless, the term "human antibody" excludes antibodies in which CDR sequences derived from the germline of another mammalian species (such as mouse) have been grafted onto human framework sequences (i.e., humanized antibodies). Fully human antibodies or human antibodies may be derived from transgenic mice bearing human antibody genes or from human cells.

As used herein, the term "specific binding", "specificity", or "specific for" refers to a non-random binding reaction between two molecules, such as a reaction between an antibody and its target antigen. In some embodiments, an antibody that specifically binds an antigen (or an antibody having specificity for an antigen) means that the antibody binds the antigen with an affinity dissociation constant (K_{D}) of less than about 10⁻⁵ M, such as less than about 10⁻⁶ M, 10⁻⁷ M, 10⁻⁸ M, 10⁻⁹ M, or 10⁻¹⁰ M or lower.

As used herein, the term "K_{D}" refers to the equilibrium dissociation constant of a specific antibody-antigen interaction, which is used to characterize the binding affinity between an antibody and an antigen. A lower equilibrium dissociation constant indicates tighter antibody-antigen binding and higher affinity between the antibody and the antigen. Typically, an antibody (e.g., the monoclonal antibodies ch13, Hu13H2L0, Hu13H2L3, Hu13H3L0, Hu13H3L3, and Hu13H3L5 disclosed herein) bind to an antigen (e.g., protein tyrosine kinase 7 (PTK7)) with an equilibrium dissociation constant (K_{D}) of less than about 10⁻⁵ M, e.g., less than about 10⁻⁶ M, 10⁻⁷ M, 10⁻⁸ M, 10⁻⁹ M, or 10⁻¹⁰ M or lower, as measured, for example, by surface plasmon resonance (SPR) on a BIACORE instrument.

As used herein, the terms "monoclonal antibody" and "mab" have the same meaning and are used interchangeably; the terms "polyclonal antibody" and "pAb" have the same meaning and are used interchangeably; the terms "polypeptide" and "protein" have the same meaning and are used interchangeably. In addition, in the present application, amino acids are generally represented by single-letter abbreviations and three-letter abbreviations well known in the art. For example, alanine may be represented by A or Ala.

As used herein, the term "antigen" refers to any substance capable of inducing an immune response in an organism, i.e., a substance that can be specifically recognized and bound by antigen receptors (TCR/BCR) on the surface of T/B lymphocytes, activate T/B lymphocytes to trigger their proliferation and differentiation, generate immune response products (sensitized lymphocytes or antibodies), and bind specifically to the corresponding immune products both in vivo and in vitro.

As used herein, the term "polypeptide" refers to a chain comprising at least two consecutively connected amino acid residues, with no upper limit on chain length. One or more amino acid residues in the polypeptide may comprise modifications, including but not limited to, optionally substituted polysarcosine residue modification, optionally substituted glycosylated polyethylene glycol modification, glycosylation modification, phosphorylation modification or disulfide bond modification. A "protein" may comprise one or more polypeptides.

As used herein, the term "tautomer" refers to a functional group isomer generated by rapid migration of a certain atom between two positions within a molecule, such as a ketone-enol tautomer, and an imine-enamine tautomer. If the compound of the present application has tautomers, such compounds may exist in the form of a single tautomer or a mixture thereof, preferably predominantly in the form of more stable tautomer.

The compound represented by Formula I or Formula II of the present application contains a group 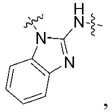 and thus there is an imine-enamine tautomer. The compound may also be present in the form of Formula I', Formula I", Formula I‴, Formula II', Formula II", or Formula II‴.

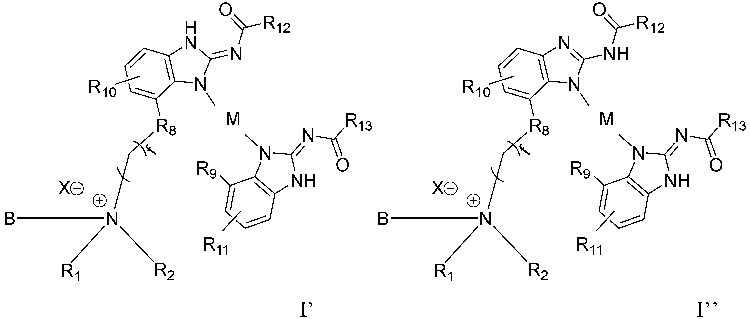

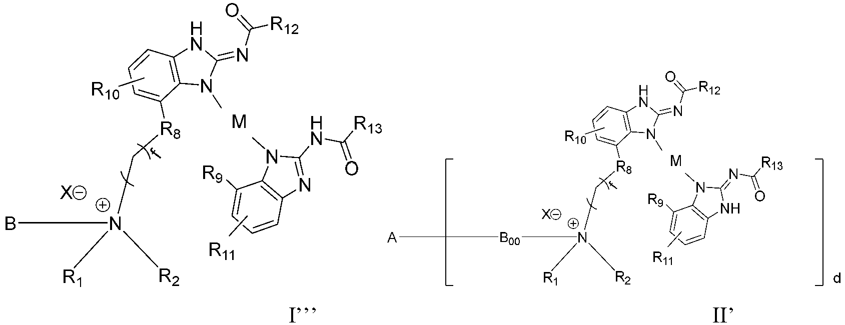

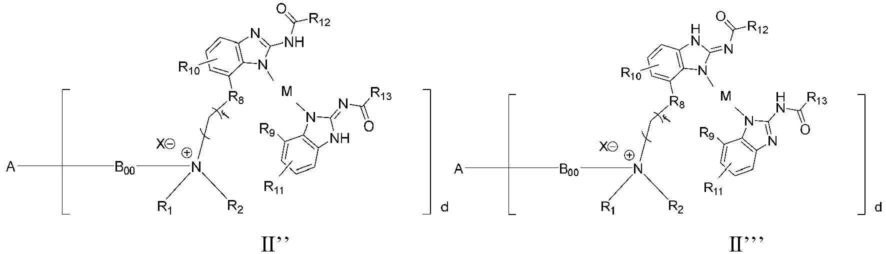

As used herein, the term "pharmaceutically acceptable carrier and/or excipient" refers to a carrier and/or excipient that is pharmacologically and/or physiologically compatible with subjects and active ingredients, which is well known in the art (see, for example, Remington's Pharmaceutical Sciences, Edited by Gennaro AR, 19th ed. Pennsylvania: Mack Publishing Company, 1995). Pharmaceutically acceptable carriers and/or excipients include, but are not limited to, pH regulators, surfactants, ionic strength enhancers, diluents, osmotic pressure maintaining agents, absorption-delaying agents, preservatives, and stabilizers. For example, the pH regulators include, but are not limited to, phosphate buffers. The surfactants include, but are not limited to, cationic, anionic or nonionic surfactants, such as Tween-80. The ionic strength enhancers include, but are not limited to, sodium chloride. The preservatives include, but are not limited to, various antibacterial agents and antifungal agents, such as parabens, chlorobutanol, phenol, sorbic acid and the like. The osmotic pressure maintaining agents include, but are not limited to, sugars, NaCl, and analogues thereof. The absorption-delaying agents include, but are not limited to, monostearate salts and gelatin. The diluents include, but are not limited to, water, aqueous buffers (e.g., buffered saline), alcohols and polyols (e.g., glycerol), and the like. The preservatives include, but are not limited to, various antibacterial agents and antifungal agents, such as thiomersal, 2-phenoxyethanol, parabens, chlorobutanol, phenol, sorbic acid and the like. The stabilizers have the meanings as commonly understood by one skilled in the art, and they can stabilize the desired activities of active ingredients in a drug, including, but being not limited to, sodium glutamate, gelatin, SPGA, sugars (such as sorbitol, mannitol, starch, sucrose, lactose, dextran, or glucose), amino acids (such as glutamic acid, or glycine), proteins (such as dried whey, albumin, or casein) or degradation products thereof (such as lactalbumin hydrolysate) and the like.

As used herein, the term "effective amount" refers to an amount sufficient to achieve or at least partially achieve a desired effect. For example, a prophylactically effective amount for a disease refers to an amount sufficient to prevent, inhibit or delay the onset of the disease; a therapeutically effective amount for a disease refers to an amount sufficient to cure or at least partially arrest the disease and its complications in a patient suffering from the disease. Determination of such effective amounts falls completely within the capability of one skilled in the art. For example, an effective amount for therapeutic applications depends on multiple factors including the severity of the diseases to be treated, the overall status of patient's own immune system, the patient's general conditions such as age, body weight and gender, administration route of the drug, and other concurrently administered therapies.

In addition, it should be indicated that the dosage and administration regimen of the compounds according to the present application depend on a variety of factors including the patient's age, body weight, gender, natural health status, nutritional status, activity intensity of a compound, administration time, metabolic rate, severity of disease, and subjective judgment of physician. The preferred dosage ranges from 0.001 mg/kg body weight/day to 1000 mg/kg body weight/day.

As used herein, the term "preventing" refers to a method implemented to inhibit or delay the onset of a disease, disorder or symptom (for example, a tumor, a pathogen infection, a disease caused by pathogen infection, a hematologic disease, a metabolic disease, an inflammation, an allergic disease, an autoimmune disease, a precancerous syndrome, a tumor metastasis, a cardiovascular disease) in a subject, or to minimize effects if such onset occurs. As used herein, the term "treating" refers to a method performed to achieve beneficial or desired clinical outcomes. The beneficial or desired clinical outcomes include, but are not limited to, lowering the rate of disease progression, ameliorating or alleviating disease conditions, and regressing lesions or improving prognosis, whether detectable or undetectable. The dosage of a therapeutic agent effective for alleviating symptoms of any specific disease may vary depending on factors such as the patient's disease status, age and body weight, and the capacity of the drug to elicit desired response in a subject. Whether the symptom of a disease is alleviated can be assessed by any clinical measurements that are routinely used by a physician or other skilled healthcare provider to evaluate the severity or progression state of the symptom.

As used herein, the term "patient" refers to any human or non-human animal, especially human, receiving prophylactic or therapeutic treatment. For example, the antibody, method, and composition described herein can be used for treating a subject suffering from cancer. The term "non-human animal" includes all vertebrates, e.g., mammals and non-mammals, such as non-human primates, sheep, dogs, cattle, horses, cats, chickens, mice, rats, amphibians, and reptiles.

As used herein, the term "about" can be understood to be within +/-20%, +/-18%, +/-15%, +/-12%, +/-10%, +/-9%, +/-8%, +/-7%, +/-6%, +/-5%, +/-4%, +/-3%, +/-2%, +/-1%, +/-0.5%, +/-0.4%, +/-0.3%, +/-0.2%, +/-0.1% of the mentioned value. Unless obvious from the context, all numerical values provided herein are modified by the term "about".

As used herein, "C₁₋₈ alkyl" refers to a saturated linear or branched monovalent hydrocarbon group having 1 to 8 carbon atoms, preferably having 1 to 6 carbon atoms, 1 to 4 carbon atoms, or 1 to 3 carbon atoms. Representative examples of the "C₁₋₈ alkyl" include, but are not limited to, methyl, ethyl, n-propyl, isopropyl, n-butyl, isobutyl, tert-butyl, n-pentyl, tert-pentyl, neopentyl, hexyl, heptyl, octyl and the like.

As used herein, the term "C₁₋₈ alkylamino" means a group C₁₋₈ alkyl-NH-, i.e., an amino group substituted with C₁₋₈ alkyl. Representative examples of the "C₁₋₈ alkylamino" include, but are not limited to, methylamino, ethylamino, propylamino, butylamino, pentylamino, hexylamino, heptylamino, octylamino, and the like.

As used herein, the term "C₁₋₈ alkylthio" means a group C₁₋₈ alkyl-S-. Representative examples of the "C₁₋₈ alkylthio" include, but are not limited to, methylthio, ethylthio, n-propylthio, isopropylthio, n-butylthio, tert-butylthio, sec-butylthio, n-pentylthio, n-hexylthio, 1,2-dimethylbutylthio, n-heptylthio, n-octylthio and the like.

As used herein, the term "C₁₋₈ alkoxy" means a group C₁₋₈ alkyl-O-. Representative examples of the "C₁₋₈ alkoxy" include, but are not limited to, methoxy, ethoxy, n-propoxy, isopropoxy, n-butoxy, tert-butoxy, sec-butoxy, n-pentyloxy, n-hexyloxy, 1,2-dimethylbutoxy, n-heptyloxy, n-octyloxy, and the like.

As used herein, the term "cycloalkyl" means a saturated cyclic hydrocarbon group having 4 to 8 carbon atoms and having a monocyclic, bicyclic or poly-fused ring system (including fused and bridged ring systems), preferably having 5 to 7 carbon atoms, or 5 to 6 carbon atoms. Representative examples of the "cycloalkyl" include, but are not limited to, monocyclic structure such as cyclopropyl, cyclobutyl, cyclopentyl, cyclohexyl, cycloheptyl, cyclooctyl, 1-methylcyclopropyl, 2-methylcyclopentyl, and 2-methylcyclooctyl; bicyclic structure, such as bicyclo[2.2.1]heptyl, and polycyclic structure, such as adamantyl.

As used herein, the term "heterocycloalkyl" means a cycloalkyl as defined herein containing one, two or more heteroatoms independently selected from the group consisting of N, O and S. Representative examples of the "heterocycloalkyl" include, but are not limited to, pyrrolidinyl, piperazinyl, piperidinyl, morpholinyl, and the like.

As used herein, the term "aryl" refers to an unsaturated aromatic carbocyclic group with 5 to 14 carbon atoms, which possesses a conjugated π-electron system and consists of monocyclic, bicyclic or poly-fused rings. The aryl preferably has 5 to 10, 5 to 8, 5 to 7 or 5 to 6 carbon atoms. Representative examples of the "aryl" include, but are not limited to, phenyl, naphthyl, anthryl, and the like.

As used herein, the term "heteroaryl" means a heteroaromatic ring group having 5 to 14 ring members, including monocyclic heteroaromatic rings and polycyclic aromatic rings formed by fusion of a monocyclic aromatic ring with one or more additional aromatic rings. The heteroaryl group contains one N atom and one or two or more other heteroatoms selected from the group consisting of O, S or N. The term "heteroaryl" as used herein also covers groups in which an aromatic ring is fused with one or more non-aromatic rings (carbocyclic rings or heterocyclic rings), with attachment sites located on the aromatic or non-aromatic ring. The heteroaryl group preferably has 5 to 10 ring members, further preferably 5 to 7 ring members, and more preferably 5 to 6 ring members. Representative examples of the "heteroaryl" include, but are not limited to, imidazolyl, triazolyl, indolyl, tetrazolyl, pyridyl, pteridinyl, pyrimidinyl, quinolinyl, isoquinolinyl, quinazolinyl, quinoxalinyl, thiazinyl, and the like.

As used herein, Compound 3 refers to the compound having the structure 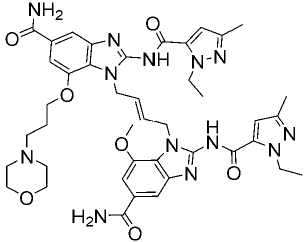 or a tautomer thereof.

When the names of the compounds used herein are inconsistent with the chemical structural formulas, the chemical structural formulas shall prevail.

The compound of the present application may be used either in their free form or in the form of a pharmaceutically acceptable hydrate or solvate thereof. The compound of the present application includes the compound represented by Formula II or Formula I and a pharmaceutically acceptable hydrate or solvate thereof.

### Abbreviations

ADC: Antibody-drug conjugate;
ISAC: Immune-stimulatory Antibody Conjugate;
CTSB: Cathepsin B;
DAR: Drug to antibody ratio;
DIPEA: *N,N*-Diisopropylethylamine;
DMAC: *N,N*-Dimethylacetamide;
DMF: *N,N*-Dimethylformamide;
DMSO: Dimethyl Sulphoxide;
Diox: 1,4-Dioxane;
DIC: Diisopropylcarbodiimide;
EDTA: Ethylenediamine tetraacetic acid;
EDCI: 1-(3-Dimethylaminopropyl)-3-ethylcarbodiimide Hydrochloride;
HOBT: 1-Hydroxybenzotriazole;
HCl: Hydrogen chloride;
HER2: Human epidermal growth factor receptor 2;
MAB: Monoclonal Antibody;
NHS: *N*-Hydroxy succinimide;
TCEP: Tris(2-carboxyethyl)phosphine;
PAB: P-Aminobenzylalcohol;
Tris: Tris(hydroxymethyl)aminomethane.

### Beneficial effects

The present application provides an immune-stimulating antibody conjugate containing quaternary ammonium salt, which has one or more of the following advantages:
1) High stability in the circulating system,
2) Improved solubility of effector molecules,
3) Capability to induce the upregulation of the gene expression of related immune factors,
4) Effective activation of immune responses in tumor tissues,
5) Capacity for targeted delivery of immunostimulants,
6) Effective reduction of systemic side effects induced by immune agonists,
7) Prominent in vitro and in vivo efficacy,
8) Favorable medication safety.

### Brief Description of the Drawings

FIG. 1 shows the hydrophobic interaction chromatography determination results of the ISACs represented by Formulas II-11, II-12, II-13, II-21, II-22, and II-23;
FIG. 2 shows the aggregation degree determination results of the ISACs represented by Formulas II-11, II-12, II-13, II-21, II-22, and II-23;
FIG. 3 shows the upregulation results of IFN-β and CXCL10 expression induced by the ISAC represented by Formula II-13;
FIG. 4 shows the evaluation results of stability of the ISAC represented by Formula II-13 in plasma;
FIG. 5 shows the pharmacodynamic experimental results of the ISAC represented by Formula II-41 in the SKOV3/THP-1 co-culture system;
FIG. 6 shows the release results of the compound represented by Formula I-13 under the action of cathepsin;
FIG. 7 shows the release results of the compound represented by Formula I-41 upon incubation with cell lysate;

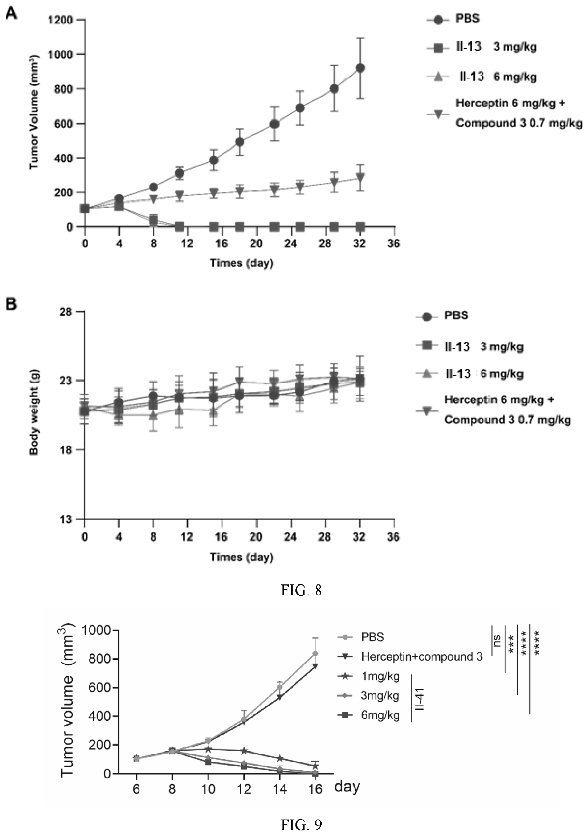

FIG. 8 shows the in I-13ivo pharmacodynamic evaluation results of the ISAC represented by Formula II-13 in the BALB/c mouse xenograft model bearing NCI-N87 human gastric carcinoma cells. Panel A shows the anti-tumor efficacy of the ISAC at various administration doses, and the results showed that sustained complete tumor regression was achieved in both dose groups, and the anti-tumor inhibitory activity exhibited dose dependence with statistically significant differences (**** representing P <0.0001); Panel B is a body weight curve of animals treated with ISAC at different administration doses, and the result showed that none of the animals in all test groups exhibited obvious treatment-related weight loss associated with poor drug tolerance;
FIG. 9 shows the tumor volume variation curves of the C57BL/6J mouse xenograft model bearing E0771-HER2 tumor cells after treatment with the ISAC represented by Formula II-41 at different administration doses; *** represents statistical significance at P<0.001, **** represents statistical significance at P <0.0001, and ns represents no statistical significance;
FIG. 10 shows the changes of tumor weight (g) in the C57BL/6J mouse xenograft model bearing E0771-HER2 tumor cells after treatment with the ISAC represented by Formula II-41 at different administration doses; *** represents statistical significance at P<0.001, **** represents statistical significance at P <0.0001, and ns represents no statistical significance;
FIG. 11 shows the body weight variation curve of the C57BL/6J mouse xenograft model bearing E0771-HER2 tumor cells after treatment with the ISAC represented by Formula II-41 at different administration doses, ns represents no statistical significance;
FIG. 12 shows the tumor volume variation curve of the C57BL/6J mouse xenograft model bearing E0771-HER2 tumor cells after treatment with the ISAC represented by Formula II-41 at different administration doses; * represents statistical significance at P<0.05, and *** represents statistical significance at P <0.001;
FIG. 13 shows the body weight variation curve of the C57BL/6J mouse xenograft model bearing E0771-HER2 tumor cells after treatment with the ISAC represented by Formula II-41 at different administration doses; ns represents no statistical significance;
FIG. 14 shows the variation curve of the number of green-labeled cancer cells over time.

### Detailed Description of the Preferred Embodiments

The embodiments of the present disclosure will be described in detail below with reference to the examples. However, one skilled in the art shall understand that the following examples are merely intended to illustrate the present application and not to be construed as limiting the scope of the present application. Where specific conditions are not indicated in the examples, the examples are carried out in accordance with the conventional conditions or the conditions recommended by manufacturers. All reagents and instruments without specified manufacturers are conventional commercially available products.

### Example 1: Preparation of Compounds I-11, I-12, and I-13, conjugates formed between valyl-alanine-containing linkers and STING agonists

### 1) Preparation of Intermediates I-11a, I-12a, and I-13a

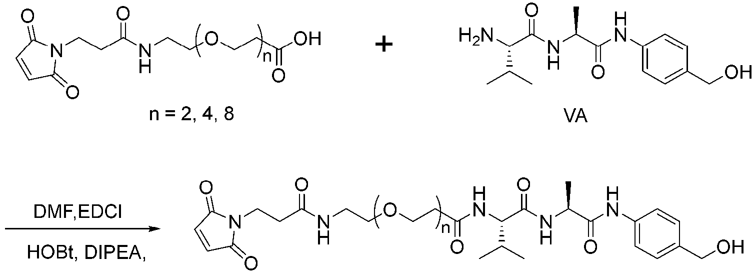

n = 2, 4, 8
I-11a,I-12a,I-13a

Maleimide-diethylene glycol-carboxylic acid (3-(2-{2-[3-(2,5-Dioxo-2,5-dihydro-pyrrol-1-yl)-propionylamino]-ethoxy}-ethoxy)-propionic acid) (500.00 mg, 1.52 mmol) as the starting material was added to a 100 mL eggplant-shaped flask and dissolved in 20 mL of anhydrous DMF. With stirring at room temperature, EDCI (437.08 mg, 2.28 mmol), HOBt (247.27 mg, 1.83 mmol) and DIPEA (294.44 mg, 2.28 mmol) were added sequentially. The resulting mixture was reacted for 2 h at room temperature under argon protection, and compound VA (Val-Ala) (445.91 mg, 1.52 mmol) was added to the reaction solution. The reaction was monitored with TLC. After reaction completion, the solvent was removed using a rotary evaporator, and the residue was purified by column chromatography, to obtain Intermediate I-13a as a colorless oil (773.98 mg, 84.35% yield) 1H-NMR (600 MHz, DMSO-d6) δ 9.99 (s, 1H), 8.21 (dd, J = 11.9, 7.0 Hz, 1H), 8.03 (t, J = 5.5 Hz, 1H), 7.90 (d, J = 8.7 Hz, 1H), 7.59 (dd, J = 16.4, 8.5 Hz, 2H), 7.36 (d, J = 8.5 Hz, 1H), 7.24 (d, J = 8.5 Hz, 1H), 7.00 (s, 2H), 4.71 (s, 1H), 4.41 - 4.36 (m, 1H), 4.33 (s, 1H), 4.23 - 4.19 (m, 1H), 3.61 - 3.57 (m, 4H), 3.48 - 3.45 (m, 4H), 3.34 (t, J = 5.9 Hz, 2H), 3.24 (s, 1H), 3.14 (q, J = 5.8 Hz, 2H), 2.46 (dd, J = 14.1, 7.2 Hz, 1H), 2.39 (dd, J = 13.6, 7.2 Hz, 1H), 2.33 (t, J = 7.3 Hz, 2H), 1.96 (dq, J = 13.6, 6.8 Hz, 1H), 1.31 (d, J = 7.1 Hz, 3H), 0.87 (d, J = 6.8 Hz, 3H), 0.83 (d, J = 6.8 Hz, 3H). Calcd. for C₂₉H₄₂N₅O₉⁺ [M+H]⁺ 604.6730; found 604.3130.

Maleimide-tetraethylene glycol-carboxylic acid (500.00 mg, 1.20 mmol) was used as the starting material, Intermediate I-12a was prepared according to the synthesis method of Compound I-11a to give a colorless oil (667.26 mg, 80.38% yield). 1H-NMR (600 MHz, DMSO-d6) δ 10.01 (s, 1H), 8.21 (dd, J = 11.8, 7.0 Hz, 1H), 8.04 (t, J = 5.5 Hz, 1H), 7.91 (d, J = 8.7 Hz, 1H), 7.60 (dd, J = 16.7, 8.5 Hz, 2H), 7.37 (d, J = 8.5 Hz, 1H), 7.24 (d, J = 8.5 Hz, 1H), 7.00 (s, 2H), 4.72 (s, 1H), 4.40 (p, J = 7.1 Hz, 1H), 4.33 (s, 1H), 4.21 (dd, J = 8.3, 7.0 Hz, 1H), 3.59 (dd, J = 8.2, 3.0 Hz, 8H), 3.51 - 3.46 (m, 12H), 3.36 (t, J = 5.9 Hz, 2H), 3.25 (s, 1H), 3.18 - 3.12 (m, 2H), 2.47 (dd, J = 14.2, 7.2 Hz, 1H), 2.42 - 2.35 (m, 1H), 2.33 (t, J = 7.3 Hz, 2H), 1.97 (dq, J = 13.5, 6.7 Hz, 1H), 1.31 (d, J = 7.1 Hz, 3H), 0.88 (d, J = 6.8 Hz, 3H), 0.84 (d, J = 6.8 Hz, 3H). Calcd. for C₃₃H₅₀N₅O₁₁⁺ [M+H]⁺ 692.7790; found 692.3657.

Maleimide-octaethylene glycol-carboxylic acid (500.00 mg, 0.84 mmol) was used as the starting material, Intermediate I-13a was prepared according to the synthesis method of Compound I-11a to give a colorless oil (560.32 mg, 76.85% yield). 1H-NMR (600 MHz, DMSO-d6) δ 9.98 (s, 1H), 8.25 - 8.14 (m, 1H), 8.03 (t, J = 5.4 Hz, 1H), 7.90 (d, J = 8.7 Hz, 1H), 7.60 (dd, J = 16.5, 8.5 Hz, 2H), 7.37 (d, J = 8.5 Hz, 1H), 7.24 (d, J = 8.4 Hz, 1H), 7.00 (s, 2H), 4.71 (s, 1H), 4.39 (t, J = 7.1 Hz, 1H), 4.33 (s, 1H), 4.21 (dd, J = 8.2, 7.2 Hz, 1H), 3.59 (t, J = 6.0 Hz, 4H), 3.49 (dd, J = 9.4, 4.5 Hz, 28H), 3.36 (t, J = 5.9 Hz, 2H), 3.25 (s, 1H), 3.15 (q, J = 5.8 Hz, 2H), 2.47 (dd, J = 14.2, 7.1 Hz, 1H), 2.41 - 2.36 (m, 1H), 2.33 (t, J = 7.3 Hz, 2H), 1.97 (dq, J = 13.6, 6.8 Hz, 1H), 1.31 (d, J = 7.1 Hz, 3H), 0.88 (d, J = 6.8 Hz, 3H), 0.84 (d, J = 6.8 Hz, 3H). Calcd. for C₄₁H₆₆N₅O₁₅⁺ [M+H]⁺ 868.9910; found 868.3524.

### 2) Preparation of Intermediates I-11b, I-12b, and I-13b:

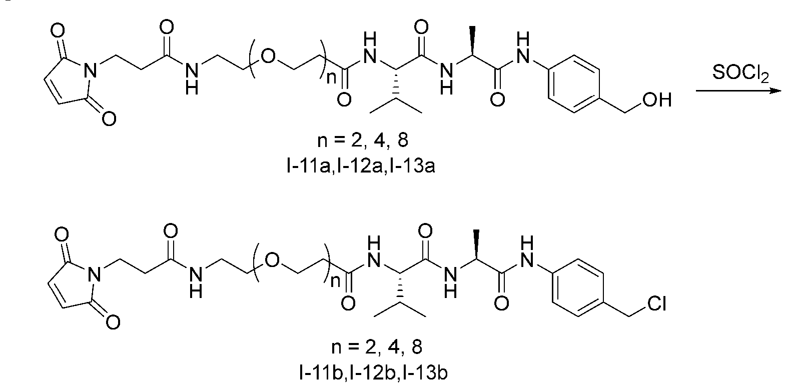

Intermediate I-11a (300.00 mg, 0.50 mmol) as the starting material was added to a 100 mL eggplant-shaped flask and dissolved in a mixed solvent of 5 mL of anhydrous DMF and 15 mL of anhydrous dichloromethane. The resulting mixture was cooled to -15°C. With stirring, thionyl chloride (178.46 mg, 1.50 mmol) dissolved in 5 mL of anhydrous dichloromethane was slowly added, and the obtained reaction mixture was kept at -15 °C and reacted for 30 min. The reaction was monitored with TLC. After reaction completion, the solvent was removed using a rotary evaporator and the resulting residue was purified by column chromatography, to obtain Intermediate I-11b as a pale-yellow oil (142.09 mg, 45.68% yield). 1H-NMR (600 MHz, DMSO-d6) δ 10.09 (s, 1H), 8.28 (d, J = 6.9 Hz, 1H), 8.08 (t, J = 5.6 Hz, 1H), 7.96 (d, J = 8.7 Hz, 1H), 7.66 (d, J = 8.5 Hz, 2H), 7.42 (d, J = 8.5 Hz, 2H), 7.06 (s, 2H), 4.77 (s, 2H), 4.45 (p, J = 7.1 Hz, 1H), 4.27 (dd, J = 8.5, 7.0 Hz, 1H), 3.68 - 3.62 (m, 5H), 3.57 - 3.50 (m, 6H), 3.40 (dd, J = 7.5, 4.2 Hz, 3H), 3.30 (s, 1H), 3.20 (dd, J = 11.4, 5.7 Hz, 3H), 2.52 (dd, J = 14.1, 7.2 Hz, 1H), 2.48 - 2.42 (m, 1H), 2.39 (t, J = 7.3 Hz, 2H), 2.02 (dq, J = 13.6, 6.7 Hz, 1H), 1.37 (d, J = 7.1 Hz, 3H), 0.93 (d, J = 6.8 Hz, 3H), 0.89 (d, J = 6.8 Hz, 3H). Calcd. for C₂₉H₄₁ClN₅O₈⁺ [M+H]⁺ 622.2565.; found 622.2618.

Intermediate I-12a (345.89 mg, 0.50 mmol) was used as the starting material, Intermediate I-12b was prepared according to the synthesis method of Intermediate I-11b to give a pale-yellow oil (147.80 mg, 41.62% yield). 1H-NMR (600 MHz, DMSO-d6) δ 9.99 (s, 1H), 8.21 (dd, J = 11.9, 7.0 Hz, 1H), 8.03 (t, J = 5.5 Hz, 1H), 7.90 (d, J = 8.7 Hz, 1H), 7.59 (dd, J = 16.4, 8.5 Hz, 2H), 7.36 (d, J = 8.5 Hz, 1H), 7.24 (d, J = 8.5 Hz, 1H), 7.00 (s, 2H), 4.71 (s, 1H), 4.42 - 4.36 (m, 1H), 4.33 (s, 1H), 4.24 - 4.17 (m, 1H), 3.62 - 3.55 (m, 4H), 3.50 - 3.43 (m, 4H), 3.34 (t, J = 5.9 Hz, 2H), 3.14 (q, J = 5.8 Hz, 2H), 2.51 - 2.49 (m, 1H), 2.46 (dd, J = 14.1, 7.2 Hz, 1H), 2.39 (dd, J = 13.6, 7.2 Hz, 1H), 2.33 (t, J = 7.3 Hz, 2H), 1.96 (dq, J = 13.6, 6.8 Hz, 1H), 1.31 (d, J = 7.1 Hz, 3H), 0.87 (d, J = 6.8 Hz, 3H), 0.83 (d, J = 6.8 Hz, 3H). Calcd. for C₃₃H₄₉ClN₅O₁₀⁺ [M+H]⁺ 710.3090.; found 710.3159.

Intermediate I-13a (434.00 mg, 0.50 mmol) was used as the starting material, Intermediate I-13b was prepared according to the synthesis method of Intermediate I-11b to give a pale-yellow oil (192.58 mg, 43.45% yield). 1H-NMR (600 MHz, DMSO-d6) δ 9.98 (s, 1H), 8.19 (dd, J = 11.6, 7.0 Hz, 1H), 8.02 (t, J = 5.5 Hz, 1H), 7.89 (d, J = 8.7 Hz, 1H), 7.59 (dd, J = 16.5, 8.5 Hz, 2H), 7.37 (d, J = 8.5 Hz, 1H), 7.24 (d, J = 8.4 Hz, 1H), 7.00 (s, 2H), 4.71 (s, 1H), 4.39 (p, J = 7.1 Hz, 1H), 4.21 (dd, J = 8.5, 6.9 Hz, 1H), 3.62 - 3.57 (m, 4H), 3.49 (dd, J = 9.4, 4.5 Hz, 28H), 3.36 (t, J = 5.9 Hz, 2H), 3.25 (s, 1H), 3.14 (q, J = 5.8 Hz, 2H), 2.46 (dd, J = 14.1, 7.2 Hz, 1H), 2.42 - 2.36 (m, 1H), 2.33 (t, J = 7.3 Hz, 2H), 1.97 (dq, J = 13.5, 6.8 Hz, 1H), 1.31 (d, J = 7.1 Hz, 3H), 0.87 (d, J = 6.8 Hz, 3H), 0.83 (d, J = 6.8 Hz, 3H). Calcd. for C₄₁H₆₅ClN₅O₁₄⁺ [M+H]⁺ 886.4138.; found 886.4213.

### 3) Preparation of Compounds I-11, I-12, and I-13:

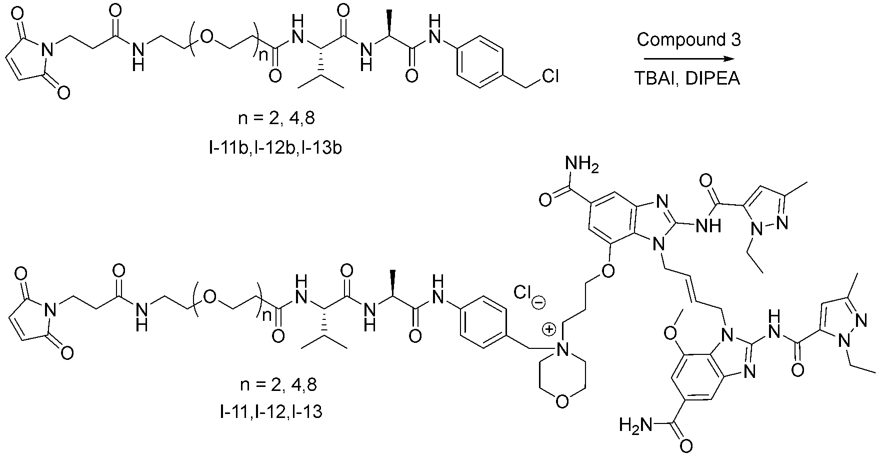

Compound 3 (20 mg, 23.53 mmol) and Intermediate I-11b (16.10 mg, 25.88 mmol) as the starting materials were added to a 1.5 mL microcentrifuge tube and dissolved in 300 µL of anhydrous DMF, then DIPEA (3.49 mg, 25.88 mmol) and TBAI (4.35 mg, 11.77 mmol) were added, and the resulting mixture was kept at 55 °C and reacted for 7 days. The reaction was monitored with TLC. After reaction completion, the solvent was removed using a rotary evaporator and the resulting residue was purified by column chromatography, to obtain Compound I-11 as a white solid (14.95 mg, 38.41% yield) with purify > 95%, Calcd. for C₇₁H₉₈N₁₈O₁₅⁺ [M+H]⁺ 1436.6906.; found 1435.6939.

Compound 3 (20 mg, 23.53 mmol) and IntermediateI-12b (18.38 mg, 25.88 mmol) were used as the starting materials, Compound I-12 was prepared according to the synthesis method of Compound I-11 to give a white solid (13.61 mg, 34.96% yield) with purify > 95%, Calcd. for C₇₅H₁₀₀N₁₈O₁₇⁷ [M+H]⁺ 1524.7430.; found 1523.7432.

Compound 3 (20 mg, 23.53 mmol) and IntermediateI-13b (22.94 mg, 25.88 mmol) were used as the starting materials, Compound I-13 was prepared according to the synthesis method of Compound I-11 to give a white solid (12.54 mg, 31.33% yield) with purify > 95%, Calcd. for Cₐ₃H₁₁₅N₁₃O₂₁⁺ [M+H]⁺ 1700.8479.; found 1700.8507. ¹H NMR (500 MHz, DMSO-*d*₆) *δ* ppm 12.44-13.03 (m, 1H), 10.22-10.28 (m, 1H), 8.47-8.53 (m, 1H), 8.26-8.35 (m, 1H), 8.02-8.07 (m, 1H), 7.94-8.01 (m, 2H), 7.87-7.91 (m, 1H), 7.69-7.76 (m, 2H), 7.66-7.68 (m, 1H), 7.61-7.65 (m, 1H), 7.32-7.42 (m, 4H), 7.29-7.32 (m, 1H), 7.24-7.29 (m, 1H), 6.97-7.03 (m, 2H), 6.51-6.56 (m, 1H), 6.44-6.51 (m, 1H), 5.69-5.82 (m, 2H), 4.78-4.99 (m, 4H), 4.55-4.65 (m, 2H), 4.46-4.55 (m, 4H), 4.33-4.40 (m, 1H), 4.16-4.23 (m, 1H), 3.93-4.10 (m, 3H), 3.78-3.92 (m, 2H), 3.61-3.68 (m, 3H), 3.55-3.61 (m, 4H), 3.47-3.52 (m, 31H), 3.12-3.18 (m, 4H), 2.45 (br d, *J =* 2.3 Hz, 1H), 2.38-2.41 (m, 1H), 2.30 (s, 3H), 2.11 (s, 3H), 2.09 (s, 3H), 1.91-1.95 (m, 1H), 1.83-1.90 (m, 1H), 1.53-1.59 (m, 1H), 1.21-1.35 (m, 13H), 0.92-0.96 (m, 2H), 0.86 (d, *J =* 6.7 Hz, 3 H), 0.81 (d, *J =* 6.9 Hz, 3H).

### Example 2: Preparation of compounds I-21, I-22, and I-23, conjugates formed between valyl-citrulline-containing linkers and STING agonists

### 1) Preparation of Intermediates I-21a, I-22a, and I-23a:

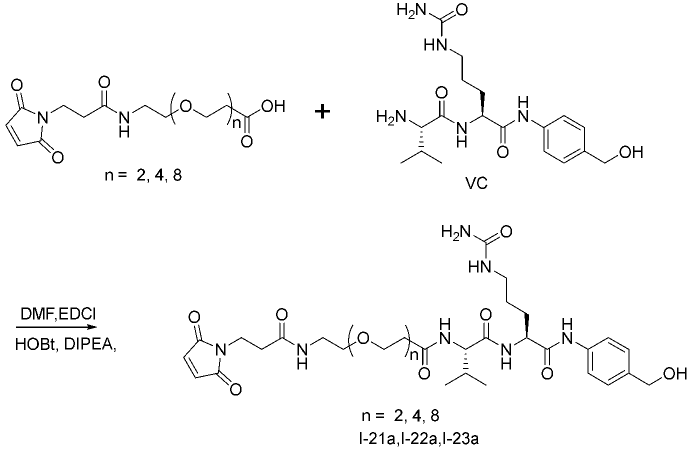

Maleimide-diethylene glycol-carboxylic acid (500.00 mg, 1.52 mmol) as the starting material was added to a 100 mL eggplant-shaped flask and dissolved in 20 mL of anhydrous DMF. With stirring at room temperature, EDCI (437.08 mg, 2.28 mmol), HOBt (247.27 mg, 1.83 mmol) and DIPEA (294.44 mg, 2.28 mmol) were added sequentially. The resulting mixture was reacted for 2 h at room temperature under argon protection, and L-Val-L-Cit-PAB (577.92 mg, 1.52 mmol) was added to the reaction solution. The reaction was monitored with TLC. After reaction completion, the solvent was removed using a rotary evaporator, and the resulting residue was purified by column chromatography, to obtain Intermediate I-21a as a colorless oil (926.31 mg, 88.35% yield). 1H-NMR (600 MHz, DMSO-d6) δ 9.93 (s, 1H), 8.11 (d, J = 7.6 Hz, 1H), 8.03 (t, J = 5.5 Hz, 1H), 7.89 (d, J = 8.7 Hz, 1H), 7.55 (d, J = 8.4 Hz, 2H), 7.23 (d, J = 8.4 Hz, 2H), 7.00 (s, 2H), 6.02 (s, 1H), 5.42 (s, 2H), 5.10 (t, J = 5.7 Hz, 1H), 4.42 (d, J = 5.5 Hz, 2H), 4.38 (dd, J = 13.6, 8.1 Hz, 1H), 4.23 (dd, J = 8.4, 7.0 Hz, 1H), 3.60 (dd, J = 14.3, 7.1 Hz, 4H), 3.47 (t, J = 5.1 Hz, 4H), 3.35 (t, J = 5.9 Hz, 2H), 3.14 (q, J = 5.8 Hz, 2H), 3.01 (dt, J = 13.1, 6.6 Hz, 1H), 2.95 (dt, J = 13.1, 6.5 Hz, 1H), 2.48 - 2.45 (m, 1H), 2.41 - 2.36 (m, 1H), 2.33 (t, J = 7.3 Hz, 2H), 1.96 (dd, J = 13.5, 6.8 Hz, 1H), 1.70 (dt, J = 14.7, 7.5 Hz, 1H), 1.62 - 1.55 (m, 1H), 1.46 - 1.40 (m, 1H), 1.39 - 1.32 (m, 1H), 0.86 (d, J = 6.8 Hz, 3H), 0.83 (d, J = 6.8 Hz, 3H). Calcd. for C₃₂H₄₈N₇O₁₀⁺ [M+H]⁺ 690.7670; found 690.3456.

Maleimide-tetraethylene glycol-carboxylic acid (500.00 mg, 1.20 mmol) was used as the starting material, Intermediate I-22a was prepared according to the synthesis method of Intermediate I-21a to give a colorless oil (788.20 mg, 84.44% yield). 1H-NMR (600 MHz, DMSO-d6) δ 9.94 (s, 1H), 8.11 (d, J = 7.6 Hz, 1H), 8.03 (t, J = 5.4 Hz, 1H), 7.89 (d, J = 8.7 Hz, 1H), 7.56 (d, J = 8.4 Hz, 2H), 7.23 (d, J = 8.4 Hz, 2H), 7.00 (s, 2H), 6.04 (s, 1H), 5.42 (s, 2H), 5.10 (t, J = 5.7 Hz, 1H), 4.42 (d, J = 5.6 Hz, 2H), 4.23 (dd, J = 8.5, 6.8 Hz, 1H), 4.03 (q, J = 7.1 Hz, 1H), 3.59 (t, J = 7.2 Hz, 4H), 3.51 - 3.46 (m, 12H), 3.36 (t, J = 5.9 Hz, 2H), 3.17 = 3.11 (m, 2H), 3.01 (dt, J = 13.1, 6.6 Hz, 1H), 2.94 (td, J = 12.9, 6.3 Hz, 1H), 2.47 (t, J = 6.9 Hz, 1H), 2.41 - 2.36 (m, 1H), 2.33 (t, J = 7.3 Hz, 2H), 1.99 (s, 1H), 1.71 (dt, J = 13.7, 6.0 Hz, 1H), 1.63 - 1.55 (m, 1H), 1.45 - 1.41 (m, 1H), 1.40 - 1.34 (m, 1H), 0.86 (d, J = 6.8 Hz, 3H), 0.83 (d, J = 6.8 Hz, 3H). Calcd. for C₃₆H₅₆N₇O₁₂⁺ [M+H]⁺ 778.8730; found 778.3977.

Maleimide-octaethylene glycol-carboxylic acid (500.00 mg, 0.84 mmol) was used as the starting material, Intermediate I-23a was prepared according to the synthesis method of Intermediate I-21a to give a colorless oil (649.00 mg, 80.98% yield). 1H NMR (600 MHz, DMSO-d6) δ 9.94 (s, 1H), 8.11 (s, 1H), 8.03 (t, J = 5.4 Hz, 1H), 7.89 (d, J = 8.7 Hz, 1H), 7.56 (d, J = 8.5 Hz, 2H), 7.23 (d, J = 8.4 Hz, 2H), 7.00 (s, 2H), 6.06 (s, 1H), 5.44 (s, 2H), 4.42 (s, 2H), 4.37 (s, 1H), 4.23 (dd, J = 8.5, 6.8 Hz, 1H), 3.59 (t, J = 6.2 Hz, 4H), 3.51 - 3.49 (m, 28H), 3.36 (s, 2H), 3.15 (d, J = 5.8 Hz, 2H), 3.03 - 2.99 (m, 1H), 2.97 - 2.93 (m, 1H), 2.47 (d, J = 6.9 Hz, 1H), 2.39 (dd, J = 9.0, 5.4 Hz, 1H), 2.33 (t, J = 7.3 Hz, 2H), 1.98 (dt, J = 13.5, 6.7 Hz, 1H), 1.71 (dt, J = 14.7, 7.4 Hz, 1H), 1.59 (ddd, J = 13.6, 8.9, 4.6 Hz, 1H), 1.46 - 1.42 (m, 1H), 1.39 - 1.35 (m, 1H), 0.86 (d, J = 6.8 Hz, 3H), 0.83 (d, J = 6.8 Hz, 3H). Calcd. for C₄₄H₇₂N₇O₁₆⁺ [M+H^{]+} 954.4957.; found 954.5015.

### 2) Preparation of Intermediates I-21b, I-22b, and I-23b:

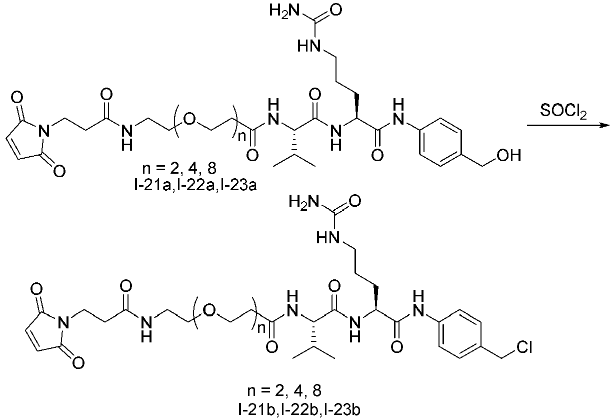

Intermediate I-21a (344.89 mg, 0.50 mmol) was used as the starting material, Intermediate I-21b was prepared according to the synthesis method of Intermediate I-11b to give a pale-yellow oil (154.53 mg, 43.64% yield). 1H-NMR (600 MHz, DMSO-d6) δ 10.10 (s, 1H), 8.15 (d, J = 7.5 Hz, 1H), 8.03 (t, J = 5.3 Hz, 1H), 7.90 (d, J = 8.6 Hz, 1H), 7.62 (d, J = 8.4 Hz, 2H), 7.36 (d, J = 8.5 Hz, 2H), 7.00 (s, 2H), 6.09 (s, 1H), 5.48 (s, 2H), 4.72 (s, 2H), 4.40 - 4.36 (m, 1H), 4.22 (d, J = 7.0 Hz, 1H), 3.60 - 3.57 (m, 4H), 3.47 (d, J = 4.1 Hz, 4H), 3.35 (t, J = 5.9 Hz, 2H), 3.15 - 3.12 (m, 2H), 3.01 (dd, J = 13.4, 6.7 Hz, 1H), 2.95 (dd, J = 13.3, 6.5 Hz, 1H), 2.47 (t, J = 7.0 Hz, 1H), 2.41 - 2.37 (m, 1H), 2.32 (d, J = 7.2 Hz, 2H), 1.97 (dd, J = 13.7, 6.9 Hz, 1H), 1.70 (dt, J = 14.1, 7.1 Hz, 1H), 1.62 - 1.57 (m, 1H), 1.45 (dd, J = 14.6, 7.5 Hz, 1H), 1.39 - 1.34 (m, 1H), 0.86 (d, J = 6.8 Hz, 3H), 0.83 (d, J = 6.8 Hz, 3H). Calcd. for C₃₂H₄₆ClN₇O₉⁺ [M+H]⁺ 708.3046.; found 708.3115.

Intermediate I-22a (389.94 mg, 0.50 mmol) was used as the starting material, intermediate I-22b was prepared according to the synthesis method of Intermediate I-11b to give a pale-yellow oil (158.55mg, 39.82% yield). 1H-NMR (600 MHz, DMSO-d6) δ 10.11 (s, 1H), 8.15 (d, J = 7.5 Hz, 1H), 8.04 (t, J = 5.2 Hz, 1H), 7.90 (d, J = 8.6 Hz, 1H), 7.62 (d, J = 8.5 Hz, 2H), 7.36 (d, J = 8.5 Hz, 2H), 7.00 (s, 2H), 6.11 (s, 1H), 5.42 (s, 2H), 4.72 (s, 2H), 4.38 (d, J = 5.4 Hz, 1H), 4.23 (d, J = 8.4 Hz, 1H), 3.59 (t, J = 7.1 Hz, 4H), 3.50 - 3.47 (m, 12H), 3.36 (t, J = 5.9 Hz, 2H), 3.16 - 3.13 (m, 2H), 3.03 - 2.99 (m, 1H), 2.96 (dd, J = 13.2, 6.5 Hz, 1H), 2.48 - 2.46 (m, 1H), 2.40 - 2.36 (m, 1H), 2.32 (d, J = 7.1 Hz, 2H), 1.97 (dd, J = 12.7, 6.0 Hz, 1H), 1.71 (dd, J = 14.4, 8.5 Hz, 1H), 1.62 - 1.58 (m, 1H), 1.45 (d, J = 7.3 Hz, 1H), 1.39 - 1.35 (m, 1H), 0.86 (d, J = 6.8 Hz, 3H), 0.83 (d, J = 6.8 Hz, 3H). Calcd. for C₃₆H₅₅ClN₇O₁₁⁺ [M+H]⁺ 796.3570.; found 796.3639.

Intermediate I-23a (477.05 mg, 0.50 mmol) was used as the starting material, intermediate I-23b was prepared according to the synthesis method of Intermediate I-11b to give a pale-yellow oil (198.54 mg, 40.83% yield). 1H-NMR (600 MHz, DMSO-d6) δ 10.17 (s, 1H), 8.17 (d, J = 7.6 Hz, 1H), 8.05 (t, J = 5.3 Hz, 1H), 7.93 (d, J = 8.7 Hz, 1H), 7.64 (d, J = 8.5 Hz, 2H), 7.36 (d, J = 8.5 Hz, 2H), 7.00 (s, 2H), 6.25 (s, 1H), 5.33 (s, 2H), 4.72 (s, 2H), 4.42 - 4.38 (m, 1H), 4.23 (d, J = 7.0 Hz, 1H), 3.60 (d, J = 6.9 Hz, 4H), 3.50 (d, J = 5.7 Hz, 28H), 3.37 (t, J = 5.9 Hz, 2H), 3.15 (d, J = 5.8 Hz, 2H), 3.04 - 3.00 (m, 1H), 2.99 - 2.96 (m, 1H), 2.50 - 2.46 (m, 1H), 2.40 (dd, J = 13.5, 7.1 Hz, 1H), 2.34 (t, J = 7.3 Hz, 2H), 1.99 (dd, J = 13.4, 6.7 Hz, 1H), 1.73 (dd, J = 14.5, 7.8 Hz, 1H), 1.62 (dd, J = 8.9, 4.6 Hz, 1H), 1.48 - 1.44 (m, 1H), 1.40 - 1.36 (m, 1H), 0.87 (d, J = 6.7 Hz, 3H), 0.84 (d, J = 6.7 Hz, 3H). Calcd. for C₄₄H₇₁ClN₇O₁₅⁺ [M+H]⁺ 972.4618.; found 972.4671.

### 3) Preparation of Compounds I-21, I-22, and I-23:

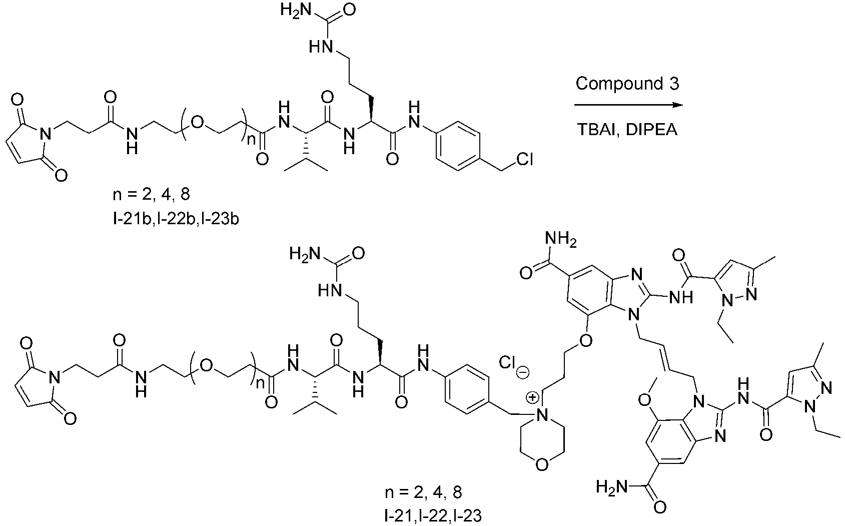

Compound 3 (20 mg, 23.53 mmol) and Intermediate I-21b (18.33 mg, 25.88 mmol) were used as the starting material, Compound I-21 was prepared according to the synthesis method of Compound I-11 to give a white solid (12.74 mg, 35.37% yield) with purity > 95%, Calcd. for C₇₄H₉₇N₂₀O₁₆⁺ [M+H]⁺ 1522.7386.; found 1521.7432.

Compound 3 (20 mg, 23.53 mmol) and Intermediate I-22b (20.61 mg, 25.88 mmol) were used as the starting material, Compound I-22 was prepared according to the synthesis method of Compound I-11 to give a white solid (12.19 mg, 32.16% yield) with purity > 95%, Calcd. for C₇₈H₁₀₅N₂₀O₁₈⁺ [M+H]⁺ 1610.7910.; found 1609.7896.

Compound 3 (20 mg, 23.53 mmol) and Intermediate I-23b (25.17 mg, 25.88 mmol) were used as the starting material, Compound I-23 was prepared according to a synthesis method similar to that of Compound I-11 to give a white solid (12.04 mg, 28.64% yield) with purity > 95%, Calcd. for C₈₆H₁₂₁N₂₀O₂₂²⁺ [M+2H]²⁺ 893.4480.; found 893.9559.

### Example 3: Preparation of Compound 1-31, a conjugate formed between a PEG8-PAB-containing linker and a STING agonist

### 1) Preparation of Intermediate I-31a

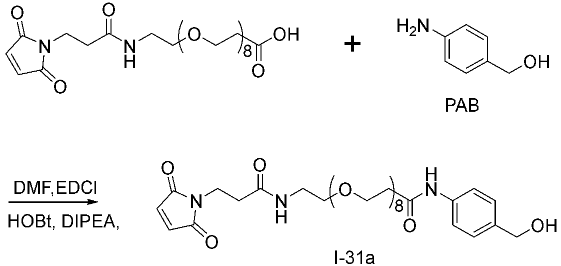

Maleimide-octaethylene glycol-carboxylic acid (500.00 mg, 0.84 mmol) as the raw material was added to a 100 mL eggplant-shaped flask and dissolved in 20 mL of anhydrous DMF. With stirring at room temperature, EDCI (241.54 mg, 1.26 mmol), HOBt (136.47 mg, 1.01 mmol) and DIPEA (162.72 mg, 1.26 mmol) were added sequentially. The resulting mixture was reacted for 2 h at room temperature under argon protection, and PAB (103.45 mg, 0.84 mmol) was added to the reaction solution. The reaction was monitored with TLC. After reaction completion, the solvent was removed using a rotary evaporator, and the resulting residue was purified by column chromatography, to obtain intermediate 1-3 1a as a colorless oil (500.85 mg, 85.45% yield). 1H-NMR (600 MHz, DMSO-d6) δ 10.02 (s, 1H), 8.02 (t, J = 5.5 Hz, 1H), 7.62 - 7.53 (m, 2H), 7.35 (d, J = 8.5 Hz, 1H), 7.22 (dd, J = 8.4, 4.4 Hz, 1H), 7.00 (s, 2H), 4.71 (s, 1H), 4.33 (s, 1H), 3.69 (t, J = 6.2 Hz, 2H), 3.59 (t, J = 7.3 Hz, 3H), 3.52 - 3.47 (m, 33H), 3.36 (t, J = 5.9 Hz, 3H), 3.25 (s, 1H), 3.14 (q, J = 5.8 Hz, 3H), 2.58 - 2.53 (m, 2H), 2.33 (t, J = 7.3 Hz, 2H). Calcd. for C₃₃H₅₂N₃O₁₃⁺ [M+H]⁺ 698.3422.; found 698.3390.

### 2) Preparation of Intermediate I-31b

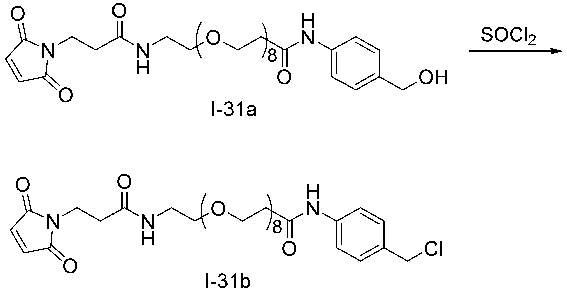

Intermediate I-31a (348.89 mg, 0.50 mmol) as the raw material was added to a 100 mL eggplant-shaped flask, and dissolved in a mixed solvent of 5 mL of anhydrous DMF and 15 mL of anhydrous dichloromethane. The resulting mixture was cooled to -15 °C. With stirring, thionyl chloride (178.46 mg, 1.50 mmol) dissolved in 5 mL of anhydrous dichloromethane was slowly added, and the resulting reaction mixture was kept at -15 °C and reacted for 30 min. The reaction was monitored with TLC. After reaction completion, the solvent was removed using a rotary evaporator and the resulting residue was purified by column chromatography, to obtain Intermediate I-31b as a pale-yellow oil (154.85 mg, 43.24% yield). 1H-NMR (600 MHz, DMSO-d6) δ 10.04 (s, 1H), 8.01 (t, J = 5.3 Hz, 1H), 7.60 (d, J = 8.4 Hz, 2H), 7.35 (d, J = 8.4 Hz, 2H), 7.00 (s, 2H), 4.71 (s, 1H), 3.69 (t, J = 6.2 Hz, 2H), 3.60 (d, J = 7.2 Hz, 2H), 3.49 (dd, J = 9.9, 4.6 Hz, 28H), 3.37 (d, J = 4.1 Hz, 2H), 3.25 (s, 1H), 3.14 (dd, J = 11.4, 5.7 Hz, 2H), 2.57 - 2.54 (m, 2H), 2.33 (t, J = 7.3 Hz, 2H). Calcd. for C₃₃H₅₀ClN₃O₁₂⁺ [M+H]⁺ 716.3083.; found 716.3153.

### 3) Preparation of Compound I-31

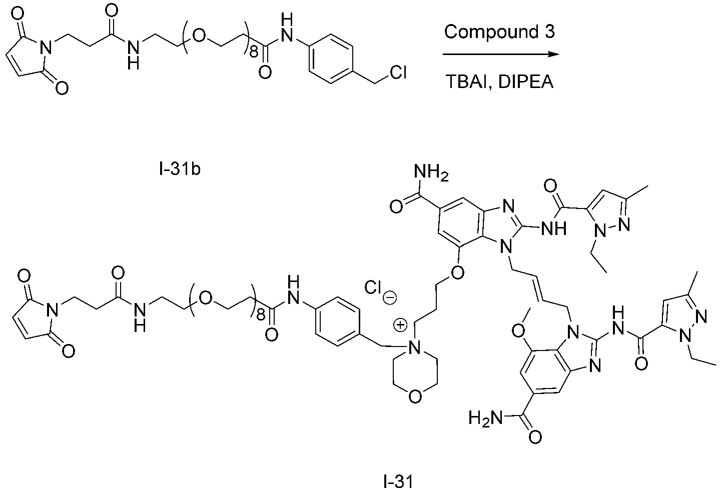

Compound 3 (20 mg, 23.53 mmol) and Intermediate I-31b (18.54 mg, 25.88 mmol) as the starting materials were added to a 1.5 mL microcentrifuge tube and dissolved in 300 µL of anhydrous DMF, then DIPEA (3.49 mg, 25.88 mmol) and TBAI (4.35 mg, 11.77 mmol) were added, and the resulting mixture was kept at 55 °C and reacted for 7 days. The reaction was monitored with TLC. After reaction completion, the solvent was removed using a rotary evaporator and the resulting residue was purified by column chromatography, to obtain Compound I-31 as a white solid (14.40 mg, 39.98% yield) with purity > 95%, Calcd. for C₇₅H₁₀₁N₁₆O₁₉⁺ [M+H]⁺ 1530.7423.; found [M+NH4]⁺1529.7416.

### Example 4: Preparation of Compound I-41, a conjugate formed between a linker and a STING agonist

### 1) Preparation of Intermediate I-41a

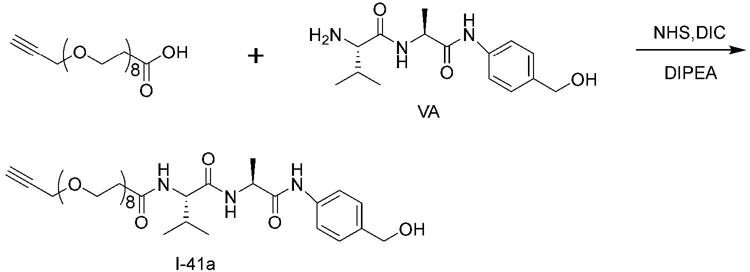

Propargyl-octaethylene glycol-carboxylic acid (500.00 mg) as the raw material was added to a 100 mL eggplant-shaped flask, and dissolved in 20 mL of anhydrous DMF. With stirring at room temperature, NHS (437.08 mg), DIC (247.27 mg) and DIPEA (294.44 mg) were added sequentially. The resulting mixture was reacted for 30 h at room temperature under argon protection, and compound VA (577.92 mg) was added to the reaction solution. The reaction was monitored with TLC. After reaction completion, the solvent was removed using a rotary evaporator, and the resulting residue was purified by column chromatography, to obtain intermediate I-41a as a colorless oil. MS (ESI) m/z: Calcd. for [M+H]⁺ 711.4, found. [M+NH₄]⁺ 729.4.

¹H NMR (600 MHz, Chloroform-d) data are as follows: δ 8.66 (s, 1H), 7.67 (d, J = 8.5 Hz, 2H), 7.32-7.23 (m, 3H), 7.07 (t, J = 6.0 Hz, 2H), 4.67 (p, J = 7.3 Hz, 1H), 4.63 (s, 2H), 4.24 (dd, J = 7.0, 5.6 Hz, 1H), 4.19 (d, J = 2.4 Hz, 2H), 3.88-3.78 (m, 1H), 3.71-3.57 (m, 28H), 2.69 (s, 0H), 2.64 (ddd, J = 14.8, 9.0, 4.1 Hz, 1H), 2.48 (ddd, J = 14.7, 5.8, 3.4 Hz, 1H), 2.44 (s, 0H), 2.26 (td, J = 7.1, 5.8 Hz, 1H), 2.02 (s, 3H), 1.45 (d, J = 7.2 Hz, 3H), 1.26 (s, 1H), 0.99 (dd, J = 15.6, 6.9 Hz, 6H). Calculated for C₃₅H₆₁N₄O₁₂⁺ [M+NH₄] ⁺ 729.4; found 729.4.

### 2) Preparation of IntermediateI-41b

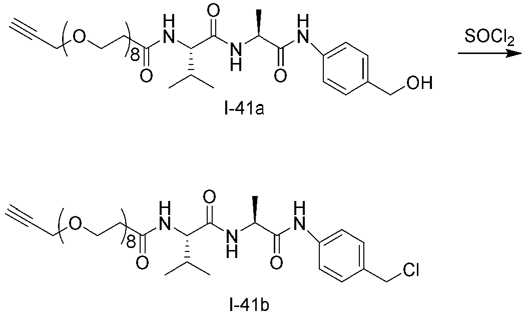

IntermediateI-41a (300.00 mg) as the raw material was added to a 100 mL eggplant-shaped flask, and dissolved in a mixed solvent of 5 mL of anhydrous DMF and 15 mL of anhydrous dichloromethane. The resulting mixture was cooled to -8 °C. With stirring, thionyl chloride (178.46 mg) dissolved in 5 mL of anhydrous dichloromethane was slowly added, and the resulting reaction mixture was kept at -8 °C and reacted for 30 min. The reaction was monitored with TLC. After reaction completion, the solvent was removed using a rotary evaporator and the resulting residue was purified by column chromatography, to obtain Intermediate I-41b as a pale-yellow oil. MS (ESI) m/z: [M+H]⁺729.4, found [M+NH₄]⁺747.3

¹H NMR (600 MHz, Chloroform-d) δ 8.70 (s, 1H), 7.73 (d, J = 8.2 Hz, 2H), 7.33-7.30 (m, 2H), 7.17-6.97 (m, 2H), 4.70-4.63 (m, 1H), 4.56 (s, 2H), 4.24-4.17 (m, 3H), 3.84 (dt, J = 9.8, 4.9 Hz, 1H), 3.72-3.52 (m, 28H), 3.35 (s, 0H), 2.67 (d, J = 11.1 Hz, 1H), 2.47 (ddd, J = 14.6, 5.5, 3.2 Hz, 1H), 2.43 (t, J = 2.4 Hz, 1H), 2.28 (h, J = 6.6 Hz, 1H), 1.45 (d, J = 7.2 Hz, 3H), 1.25 (s, 4H), 1.01 (dd, J = 15.9, 6.8 Hz, 6H). Calculated for C₃₅H₆₀ClN₄O₁₁⁺ [M+NH₄] ⁺ 747.3.; found 747.3.

### 3) Preparation of Compound I-41

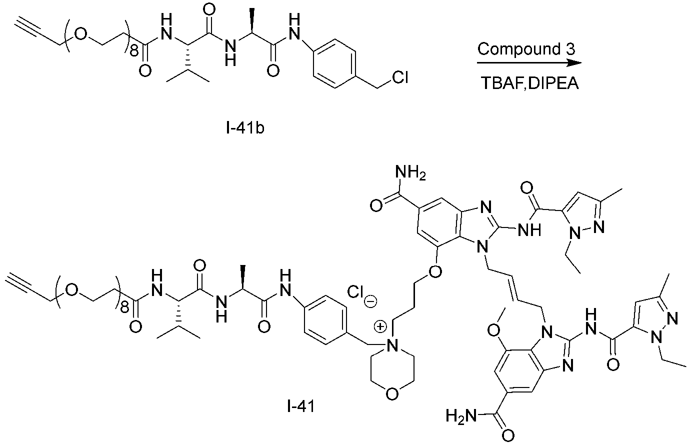

I-41b (25 mg) and Compound 3 (20 mg) were added to a 1.5 mL EP tube, to which 400 µL of anhydrous DMF were added to dissolve the solids. DIPEA (19 mg, 0.15 mmol) was dropwise added to the reaction system, the mixture was shaken at room temperature for 5 days. After reaction completion, the solvent was removed by concentration under reduced pressure, to obtain a crude product, which is purified by column chromatography to obtain the target product, being Compound I-41 as a black solid (10 mg, 20% yield). MS (ESI) m/z: [M+H]⁺1235.61, found [M+NH₄]⁺ 1543.8.

¹H-NMR (600 MHz, DMSO-d6): δ 10.289-10.191 (m, 1H), 8.235 (s, 1H), 7.873-7.861 (m, 2H), 7.761-7.735 (m, 3H), 7.506-7.303 (m, 4H), 6.647 (s, 1H), 6.513-6.435 (s, 1H), 5.325 (t, J = 4.8 Hz, 1H), 4.999-4.855 (m, 3H), 4.593-4.374 (m, 5H), 4.201-4.189 (m, 2H), 4.132 (s, 3H), 3.589-3.413 (m, 43H), 2.109-2.099 (m, 4H), 2.021-1.943 (m, 6H), 1.323-1.293 (m, 7H), 0.884-0.817 (m, 12H). Calculated for C₈₃H₁₁₅N₁₃O₂₁⁺ [M+ NH₄] ⁺ 1543.8; found 1543.778.

### Example 5: Preparation of antibody conjugates II-11, II-12, and II-13 based on valyl-alanine-containing linker-STING agonist conjugates

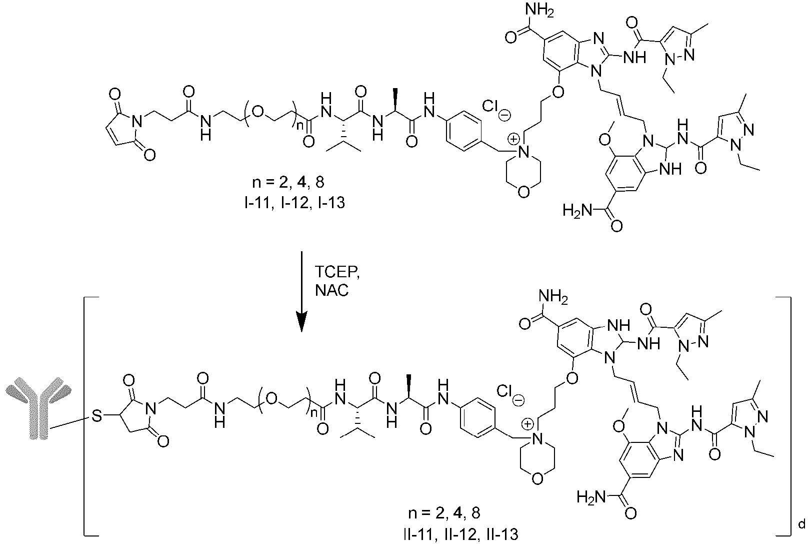

In the present example, Compounds I-11, I-12, and I-13 prepared in Example 1 were conjugated to humanized anti-HER2 monoclonal antibody.

### 1) Preparation of commonly used buffered salt solutions:

Buffered salt solution-1 (Buffer-1): 3.11 g of L-histidine was dissolved in 1L of double-distilled water, and after complete dissolution, the pH of the resulting solution was adjusted to about 5.50 (±0.05) with medical glacial acetic acid; the adjusted solution was sterile-filtered through a 0.22 µm membrane filter, dispensed into bottles, and stored temporarily at 4 °C for later use.

Buffered salt solution-2 (Buffer-2): 6.06 g of TRIS•base and 0.93 g of EDTA•2Na were weighed and dissolved, the resulting mixture was diluted to a final volume of 100 mL; 7.88 g of TRIS•HCl and 0.93 g of EDTA•2Na were further weighed and dissolved, the resulting mixture was diluted to a final volume of 100 mL; the TRIS•HCl solution was mixed with the TRIS•base solution to adjust the pH to 8.50 (+ 0.05). The resulting buffer solution was sterile-filtered through a 0.22 µm membrane filter, dispensed into bottles, and stored temporarily at 4 °C for later use.

Buffered salt solution-3 (Buffer-3): 1.715 mL of medical glacial acetic acid was pipetted and dissolved in 200 mL of double-distilled water, and after thorough mixing, the obtained solution was sterile-filtered through a 0.22 µm membrane filter, dispensed into bottles, and stored temporarily at 4 °C for later use.

### 2) Antibody Conjugation Reaction:

(1) Buffer exchange of pharmaceutical antibody: the original formulation solution of a humanized anti-HER2 monoclonal antibody Herceptin (purchased from Zhejiang Hisun Pharmaceutical Co., Ltd.) used for conjugation contained pharmaceutical excipients including 0.616 mg/ml of histidine hydrochloride (monohydrate), 0.364 mg/ml of L-histidine, 22.727 mg/ml of trehalose, and 100 mg/ml of Tween-20. To eliminate interference caused by the excipients, the freeze-thawed stock antibody solution was thawed slowly at room temperature, followed by buffer exchange into Buffer-1 using a G25 Sephadex column. After buffer exchange, the obtained antibody solution was concentrated by ultrafiltration and concentration (to a final concentration higher than 5 mg/mL. The antibody concentration was determined with an ultraviolet spectrophotometer.
(2) Preparation of conjugation reaction solutions: according to the required amount of antibody for conjugation (1 eq), the antibody in buffer-1 solution was precisely pipetted, and an appropriate volume buffer-1 was supplemented to bring the antibody concentration to about 10 mg/mL; the pH of the solution was adjusted to 6 to 8 with Buffer-2, and the solution was transferred to a clean reaction vial with a pipette.
(3) Reduction of antibody: the reaction solution in the vial was slowly stirred (at 100 rpm), to which 2 to 5 eq of TCEP•HCl solution with a concentration of 2.87 mg/mL were added, and after the addition was completed, the resulting mixture was slowly stirred at room temperature and reacted for 60 to 180 min.
(4) Conjugation of antibody: the volume of an organic solvent (DMAC or DMSO) was calculated and added to account for 5% - 15% of the total volume; the required mass of the ADC small-molecule payload (linker-STING agonist) was also calculated with a slight molar excess (typically 8 eq), and typically, the small-molecule payload should be slightly excessive (typically 8 eq), and the corresponding concentration of the payload in the organic solvent was determined accordingly. After precise preparation of the small-molecule payload solution, the solution was slowly dropwise added to the reduced antibody reaction solution. The resulting mixture was gently stirred at room temperature and reacted for 0.5 to 5 h depending on the conjugation progress.
(5) Reaction quenching: after the conjugation reaction reached the predetermined time, an excess amount of aqueous N-acetylcysteine solution (1.63 mg/mL), a water-soluble small molecule containing reactive thiol groups, was added to the reaction solution, and the mixture was continuously and gently stirred for 30 min.
(6) Preliminary purification of product: after completion of the quenching reaction, Buffer-3 was added to adjust the pH of the reaction solution to about 5.50; after the resulting reaction solution was filtered and preliminarily purified using a G25 Sephadex column. The early eluting fractions (about 80%) were collected, concentrated by ultrafiltration, sterile-filtered, and aliquoted. Partial samples reserved for product analysis were stored temporarily at 4 °C, the remaining products were stored at -80 °C for later use.

The structures of the prepared ISACs were listed in Table 1, wherein d (DAR value) may be determined with reference to the method described in the reference (J. Ouyang, in Antibody-Drug Conjugates, Vol. 1045 (Ed.: L. Ducry), 2013, pp. 275-283).

**Table 1 Structures of the synthesized ISACs II-11, II-12, and II-13**

| Designations | ISAC Structures |
|---|---|
| II-11 | 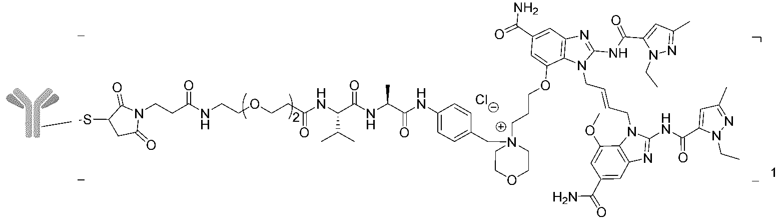 |
| II-12 | 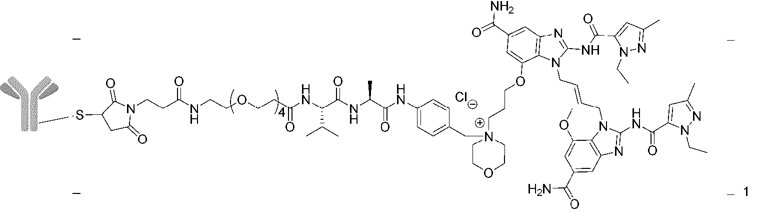 |
| II-13 | 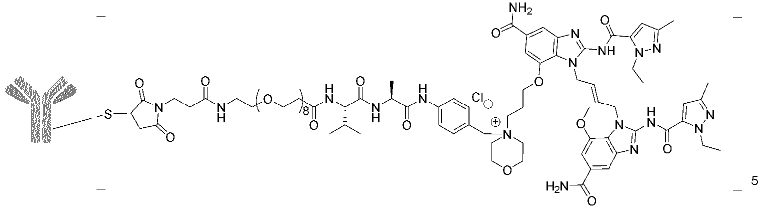 |

### Example 6: Preparation of antibody conjugates II-21, II-22, and II-23 based on valyl-citrulline-containing linker-STING agonist conjugates

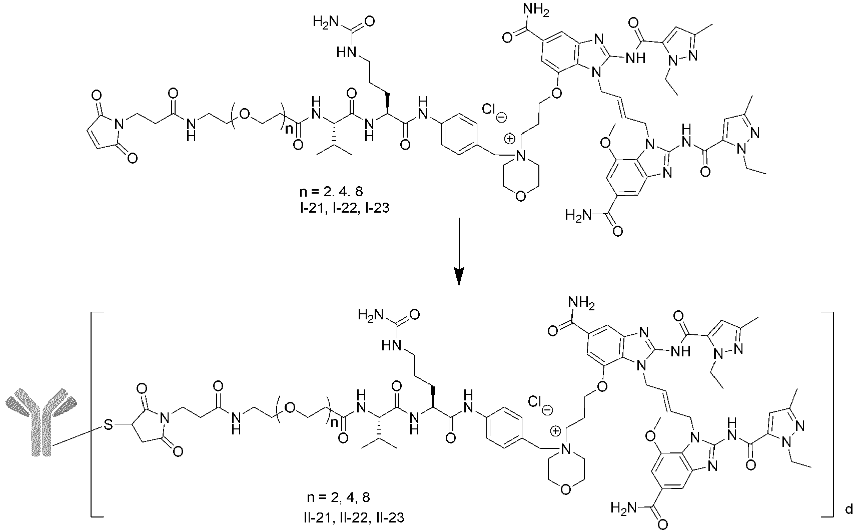

In the present example, Compounds I-21, I-22, and I-23 prepared in Example 2 were conjugated to the humanized anti-HER2 monoclonal antibody.

The conjugation method was the same as that in Example 5, and the structures of the prepared ISACs were listed in Table 2, wherein d (DAR value) may be determined with reference to the method described in the reference (J. Ouyang, in Antibody-Drug Conjugates, Vol. 1045 (Ed.: L. Ducry), 2013, pp. 275-283.).

**Table 2. Structures of the synthesized ISACs of II-21, II-22, and II-23**

| Designations | ISAC structures |
|---|---|
| II-21 | 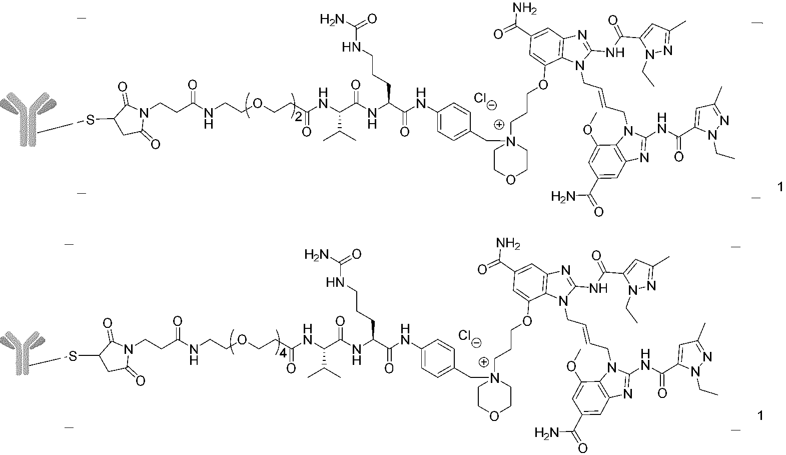 |
| II-22 | 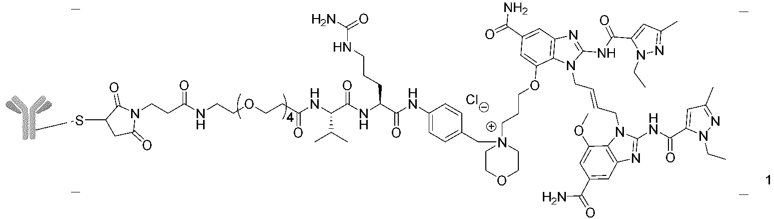 |
| II-23 | 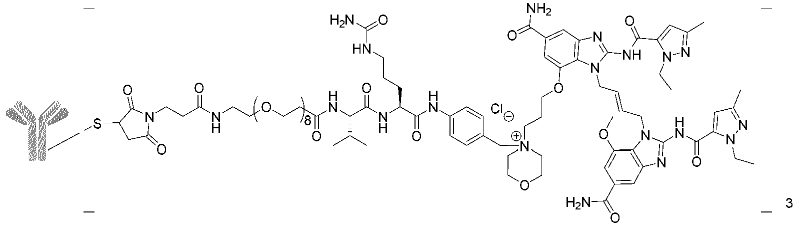 |

### Example 7: Preparation of an antibody conjugate II-31 based on PEG8-PAB linker-STING agonist conjugates

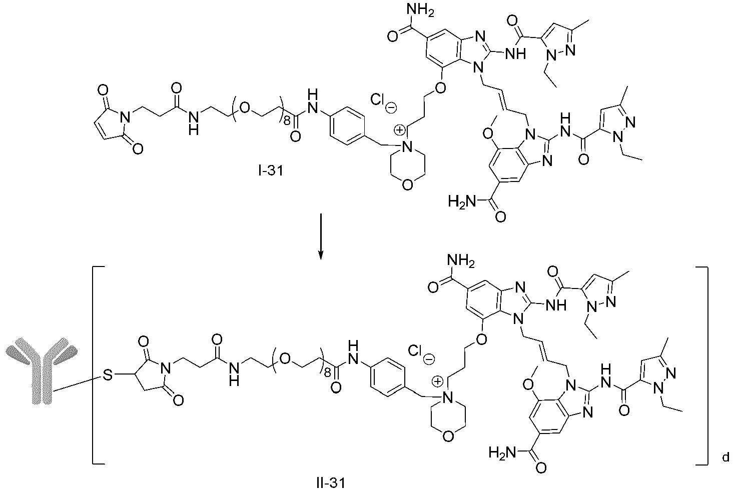

In the present example, Compound I-31 prepared in Example 3 was conjugated to the humanized anti-HER2 monoclonal antibody. The conjugation method was the same as that in Example 5, and the prepared ISAC was represented by Formula II-31, wherein d represented the DAR value, being about 2, and it may be determined with reference to the method as described in the reference (J. Ouyang, in Antibody-Drug Conjugates, Vol. 1045 (Ed.: L. Ducry), 2013, pp. 275-283.).

### Example 8: Preparation of an antibody conjugate II-41 containing STING agonist

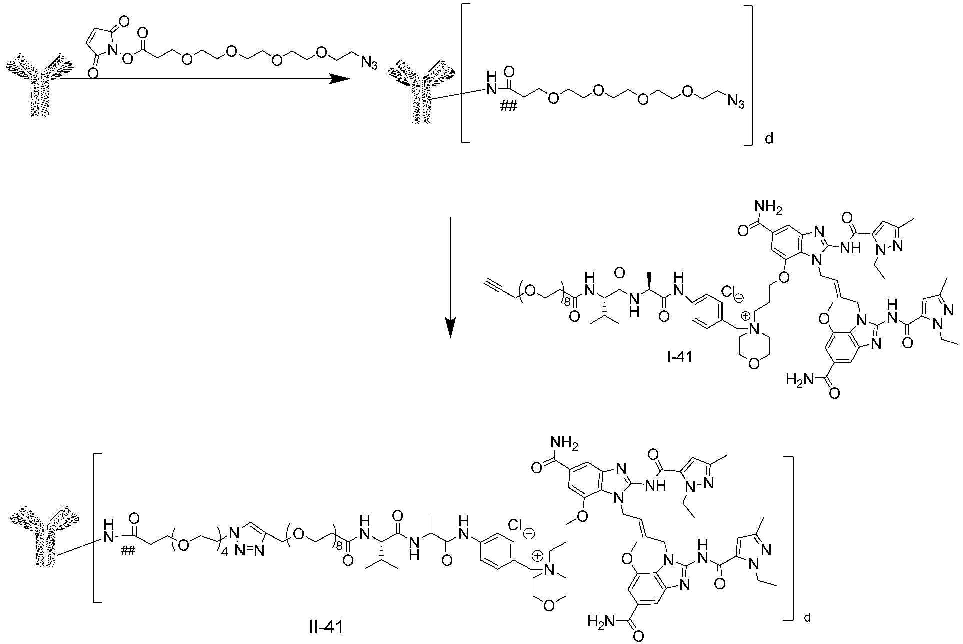

### 1) Preparation of antibody modified with azido group:

Antibody (26.25 mg, 1.75×10⁻⁴ mmol) was added to a 5 mL EP tube, to which a solution of compound 2,5-dioxopyrrolidin-1-yl 1-azido-3,6,9,12-tetraoxapentadecan-15-oate (0.54 mg, 1.4×10⁻³ mmol) in DMA was dropwise added. The resulting mixture was stirred and reacted at room temperature overnight, and the conjugated product was exchanged into PBS to obtain the azide-modified humanized anti-HER2 monoclonal antibody Herceptin, wherein 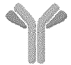 represents a humanized anti-HER2 monoclonal antibody Herceptin, d represents the DAR value, being about 2.

2) The azide-modified humanized HER2 monoclonal antibody (2.5 mg, 1.67×10⁻⁵ mmol) was added to a 2 mL EP tube, to which a DMA solution of compound I-41 (0.41 mg) prepared in Example 4 was added. Then, Aqueous solutions of CuSO₄ (0.13 mg, 5.01×10⁻⁴ mmol), THPTA (0.87 mg, 2.0×10⁻³ mmol) and sodium ascorbate (1.49 mg, 7.51×10⁻³ mmol) were sequentially added to the antibody solution, the resulting mixture was stirred and reacted at room temperature for 5 h, and the conjugated product was exchanged into PBS to obtain the target antibody-drug conjugate II-41, wherein d represents the DAR value, being about 2.

### Example 9: Determination of DAR values of ISACs represented by Formulae II-11, II-12, II-13, II-21, II-22, and II-23

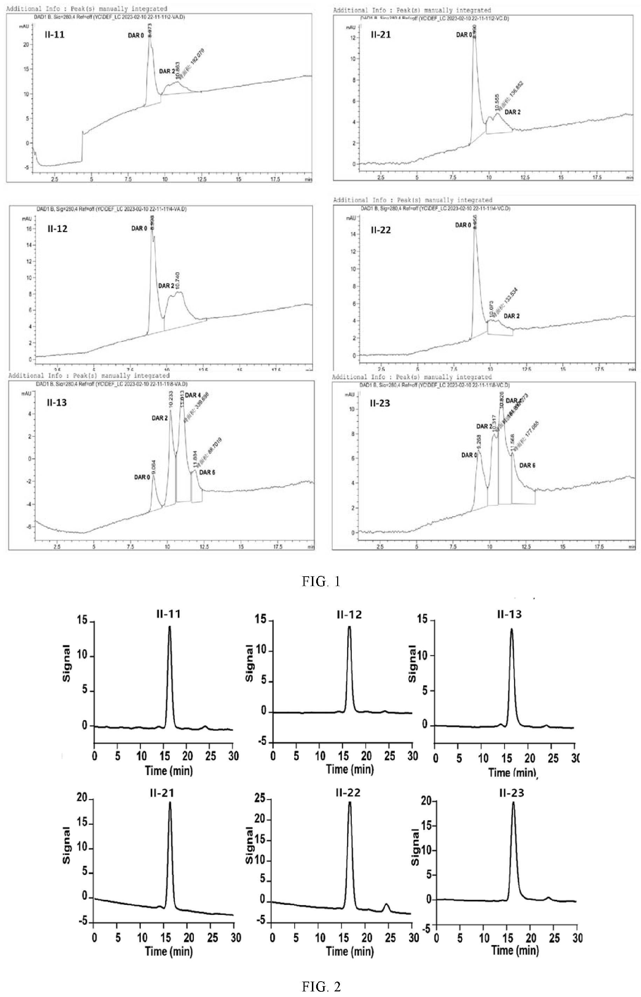

In the present example, the DAR values (i.e., the d in the formulas) of ISACs represented by Formulae II-11, II-12, II-13, II-21, II-22, and II-23 were evaluated. The DAR values of each conjugate were determined by hydrophobic interaction chromatography (FIG. 1), and the major peaks observed in the chromatogram corresponded to species conjugated with 0, 2, 4 and 6 small-molecule drugs, respectively. The determined DAR values of ISACs represented by Formulae II-11, II-12, II-13, II-21, II-22, and II-23 are shown in Table 3.

**Table 3. Determination results of DAR values of antibody-drug conjugates**

| Designations | PEG Length | Type of Linkers | DAR values |
|---|---|---|---|
| II-11 | 2 | Val-Ala dipeptide | ≈1 |
| II-12 | 4 | Val-Ala dipeptide | ≈1 |
| II-13 | 8 | Val-Ala dipeptide | ≈5 |
| II-21 | 2 | Val-Cit dipeptide | ≈1 |
| II-22 | 4 | Val-Cit dipeptide | ≈1 |
| II-23 | 8 | Val-Cit dipeptide | ≈3 |
| II-31 | 8 | PEG8-PAB | ≈2 |
| II-41 | 8 | Val-Cit dipeptide | ≈2 |

### Example 10: Determination of aggregation degree of ISACs represented by Formulae II-11, II-12, II-13, II-21, II-22, and II-23

In the present example, the aggregation degree of the ISACs represented by Formulae II-11, II-12, II-13, II-21, II-22, and II-23 were evaluated (FIG. 2). The monomers and aggregates of each antibody conjugate were detected using size-exclusion chromatography, and the results showed that ISACs represented by Formulae II-11, II-12, II-13, II-21, II-22, and II-23 existed almost entirely in monomeric form (with a purity of active monomers > 95%).

### Example 11: Stimulation of the ISAC represented by Formula II-13 on related cytokines

In the present example, the efficacy of the ISAC represented by Formula II-13 in regulating immune factor gene expression was evaluated via qPCR by detecting mRNA changes of interferon β (IFN-β) and C-X-C motif chemokine ligand 10 (CXCL10) in tumor cells treated with test drug. The cell lines used in the test were HER2-positive SKOV3 and SKBR3 cell lines (purchased from ATCC). The test drugs included vehicle control (PBS), Compound 3, the ISAC represented by Formula II-13. The IFN-β serves as a biomarker activated by downstream signaling of the cGAS-STING pathway, and the CXCL10 is induced by type I interferon and serves as a marker to promote recruitment of immune cells into tumor tissues. By evaluating the gene expression levels of the above cytokines, the degree of immune system activation in tumor tissues triggered by the ADC can be evaluated.

Reagents, instruments and consumables used in the experiment were listed in the following table.

| | |
|---|---|
| Reagents and consumables | Vendor |
| FBS | Invitrogen |
| Penicillin-Streptomycin | Invitrogen |
| DMEM | Invitrogen |
| SuperScript^{™} III CellsDirect^{™} cDNA | Invitrogen |
| Instruments | Vendor |
| PCR amplifier | Gene Max |
| RT-PCR primer sequence: | |

| Names | RNA sequence |
|---|---|
| IFNβ1F | GTCTCCTCCAAATTGCTCTC |
| IFNP1R | ACAGGAGCTTCTGACACTGA |
| CXCL10F | TGGCATTCAAGGAGTACCTC |
| CXCL10R | TTGTAGCAATGATCTCAACACG |
| IL6F | AGACAGCCACTCACCTCTTCAG |
| IL6R | GACTGTGCACTTGCTGGTGGAT |

The test procedure was described as follows:

### (1) Thawing Cell

Cryopreserved target cells were thawed by gentle agitation of the cryovial in a 37 °C water bath;
after thawing, the cryovial was removed from the water bath and decontaminated by spraying or immersing in 70% ethanol;
the cells in the cryovial were transferred into a centrifuge tube containing 9 mL of complete DMEM culture medium, followed by centrifugation at 200 g for 5 min;
the cells were resuspended with culture medium, and precipitated and seeded into a 75 cm² culture flask;
the cells were cultured in a Galaxy^{®} CO₂ incubator (48R, #CO48312044) under standard culture conditions of 37 °C, 20% oxygen and 5% carbon dioxide.

### (2) Spreading Cell

Cells were subcultured three times per week at a ratio of 1:4 in a culture medium supplemented with 10% FBS (heat inactivated) and a 1% Penicillin-Streptomycin solution;
for cell passaging, adherent cells were first rinsed with 3 mL of trypsin/EDTA solution (3 mL). Then, 3 mL of trypsin/EDTA solution was added to the T75 culture flask, and the flask was gently rotated to ensure uniform coverage of the cell. The cells were incubated at 37 °C until complete cell detachment was achieved. Cell detachment was verified under a microscope, then an equal volume of the cell culture medium was added to inactivate the trypsin, and the separated cells were collected, centrifuged at 200 g for 5 minutes, and then re-suspended in a fresh culture medium.

### (3) Inoculating cell

The cells were collected and counted;
100 µ L of a cell suspension with adjusted density were inoculated to a 6-well cell culture plate, and the final cell density was about from 5000 cells/well to 10000 cells/well;
The culture plate was covered and incubated in a 5% CO₂ incubator with 20% oxygen at 37 °C for 24 h.

### (4) Preparing compounds

A compound solution was serially diluted at a ratio of 1:3, wherein the maximum initial concentrations of the ISAC represented by Formula II-13 and Compound 3 were about 400 nM;

### (5) Co-incubation of cells with test compounds

The culture medium in the 6-well plate inoculated with cells was discarded, 150 µL of fresh culture medium was added to each well, and then 50 µL of the prepared test compounds were added to the 6-well plate. After the cells were co-incubated with the compounds in an incubator under conditions of 37 °C, 5% CO₂, and 0.1% O₂ for 24 h. After incubation, the drug-containing medium was removed, and the cells were rinsed with a fresh culture medium. Then the cells were placed in an incubator at 37 °C, 5% CO₂, and 20% O₂ and continuously cultured for an additional 72 h or 120 h.

### (6) Extraction of cell RNA

RNA extraction: cells were rinsed with PBS (three times), and Trizol reagent was added to lyse the sample, and the mixture was incubated at room temperature for 5 min. Chloroform was subsequently added, followed by vortexing for 30 s and standing at room temperature for another 5 min. After centrifugation at 4 °C and 12000 rpm for 15 min in a centrifuge, the upper aqueous phase was transferred into an EP tube. Isopropanol was added and mixed thoroughly; the resulting mixture was left to stand for 10 min at room temperature. A second round of centrifugation was carried out at 4 °C, 12000 rpm for 10 min, the supernatant was removed, and the precipitate was resuspended by adding 75% alcohol. After another centrifugation (4 °C, 12000 rpm, 5 min), the supernatant was removed again, the precipitate was air-dried at room temperature for 15 min and redissolved in DEPC water preheated at 65 °C, and the dissolved RNA was stored in a refrigerator at -20 °C for later use.

### (7) gDNA Removal and RNA reverse transcription:

1 µL of total RNA was mixed with 1 µL of gDNA Remover, 1 µL of Oligo Primer, 10 µL of 2×TS Reaction Mix, and 1 µL of TransScript RT/RI Enzyme Mix to reach a total volume of 20 µL, and the remaining volume was made up with RNase-free water. The PCR tube was incubated at 42 °C for 15 min, by which time RNA has been reverse transcribed into cDNA.

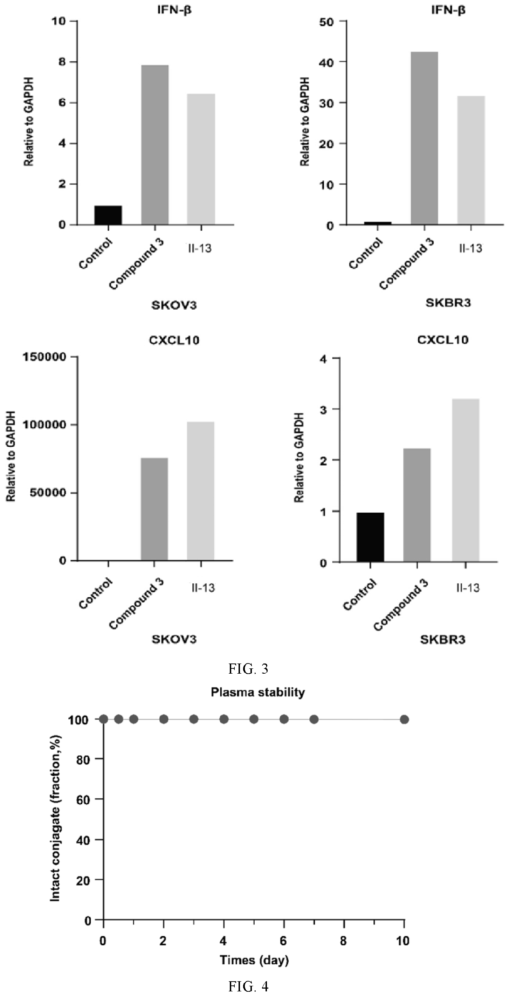

The experimental results showed (in FIG. 3) that the ISAC represented by Formula II-13 induced upregulating gene expression of two cytokines under identical experimental conditions, with efficacy superior or comparable to that of free payload Compound 3. The above results showed that the ISACs provided by the present application is capable of upregulating the gene expression of related immune factors, and efficiently activating the immune response of tumor tissues.

### Example 12: Evaluation of in vitro plasma stability of the ISAC represented by Formula II-13

To measure the stability of the conjugate in human plasma and PBS, released free payload Compound 3 was dissolved in 50% human plasma and PBS, respectively, to prepare standard samples, and test samples of conjugate were prepared using the same as that for the standard samples. Both samples were incubated at 37 °C for 10 days and aliquots were taken at predetermined time points (0 h, 12 h, 1 d, 2 d, 3 d, 4 d, 5 d, 6 d, 7 d and 10 d). Based on the standard curve of Compound 3 established with LC-MS, the concentration of the total free payload Compound 3 released from the conjugate at individual time points were quantified.

As shown in FIG. 4, the experimental results showed that after 10 days, the ISAC represented by Formula II-13 released 0.23% and 0.01% of Compound 3 in PBS and 50% plasma, respectively, indicating that the ISAC represented by Formula II-13 remained stable in PBS and 50% plasma at 37 °C.

### Example 13: Pharmacodynamic study of the ISAC represented by Formula II-41 in SKOV3/THP-1 co-culture cell line

The induction of the STING pathway in immune cells by the HER2-targeted ISAC represented by Formula II-41 was evaluated by a co-culture assay consisting of cancer cells SKOV3 and THP1-IRF 3-luciferase reporter cells. The human ovarian cancer SKOV3 cells were seeded into a 96-well CellBIND surface tissue culture plate (about 15000 cells / well) and allowed to adhere in a RPMI-1640 culture medium containing 10% FBS and 1% penicillin/streptomycin for 6 hours. Serial dilutions of the ISAC represented by Formula II-41 (starting from 100 nM, 3-fold serial dilutions were prepared in a growth culture medium) were added to each well and the plate was incubated at 37 °C for 20 min. Then, the THP 1 dual-reporter cells were added to each well (at a density of about 30,000 cells per well). The co-culture system was incubated for 20 hours at 37 °C under a humidified atmosphere of 5% CO₂. The cell culture supernatant (20 µL) from each incubated sample was mixted with resuspended QUANTI-Luc reagent (50 ul) and the luminescence signal was immediately measured by a SpectraMax M5 microplate reader (Molecular Devices). EC₅₀ values were calculated according to the dose-response curve.

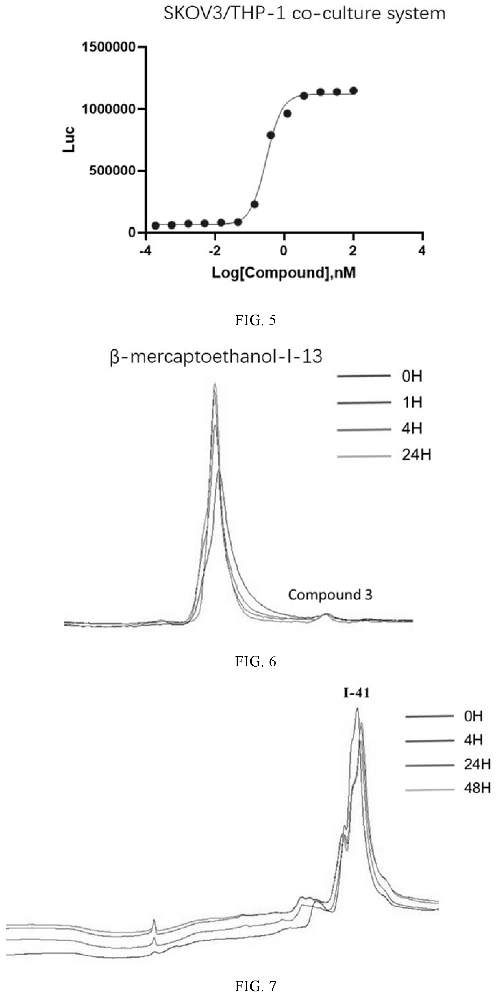

The experimental results were shown in FIG. 5. The results showed that the ISAC represented by Formula II-41 strongly activated STING signal pathway in THP-1 cells at a very low dosage, with an EC₅₀ value of 0.3 nM.

### Example 14: Release study of the compound represented by Formula I-13 under the action of cathepsin

1 mM of DMA stock solution of the compound represented by Formula I-13 was mixed with 1 mM of PBS stock solution of 2-mercaptoethanol at a volume ratio of 1:9, the resulting mixture was stirred at room temperature for 1 h to prepare a 2-mercaptoethanol-I-13 stock solution (100 µM). To 15 µL of 2-mercaptoethanol-I-13 solution, 30 µL of CTSB (15 UN/mL) were added, then 105 µL of active buffer (50 mM sodium acetate, 100 mM NaCl, 8 mM L-cysteine, 1 mM EDTA, pH 5.0) were added, and after well mixing, the mixture was incubated in a 37 °C incubator. After sampling at predetermined time points (t =0, 1h, 4h, 24 h), 600 µL of methanol was added to the samples, the protein was precipitated by centrifugation (12000 rpm, 4 °C, 20 min), and the supernatant was collected and analyzed via HPLC to quantify the released STING agonist Compound 3 and the intact parent compound represented by Formula I-13.

The experimental results were shown in FIG. 6. Unexpectedly, the results showed that 2-mercaptoethanol-I-13 was incubated with 3 UN CTSB for 24 h, almost all the compound remained in its intact parent form, and no obvious release of Compound 3 was observed. It was demonstrated that the dipeptide bonds in the ISACs provided by the present application were not cleaved by cathepsins under conventional conditions, and distinct from traditional cleavable ISACs, the ISACs provided in the present application exerts pharmacological effects via a non-cleavable quaternary ammonium salt structure.

### Example 15: Release study of the compound represented by Formula I-41 upon incubation with cell lysate

SKBR3 cells (purchased from Procell Life Science & Technology Co., Ltd., Wuhan, China) were collected at a total cell count of about 1.5×10⁶ cells and transferred into a 1.5 mL EP tube, and centrifuged (1000 rpm, 4 °C, 4 min), to remove the culture medium. To the collected cell samples, 500 µL of a RIPA lysate (product No.: P0013B, Beyotime) was added, and mixed well, and the resulting mixture was incubated on an ice-bath for 30 min, and centrifuged (14000 rcf, 4 °C, 15 min). 45 µL of the supernatant was mixed with 5 µL of a DMSO stock solution (100 µM) of the compound represented by Formula I-41, and the resulting mixture was incubated in an incubator at 37 °C. After sampling at predetermined time points (0 h, 4h, 24 h, 48 h), 150 µL of methanol was added to the sample, the protein was precipitated by centrifugation (12000 rpm, 4 °C, 20 min), and the supernatant was collected and analyzed via HPLC to quantify the released STING agonist Compound 3 and the intact parent compound represented by Formula I-41 were detected.

The experimental results were shown in FIG. 7. The results, similar to those in Example 14, showed that the compound represented by Formula I-41 was incubated with cell lysate for 48 h, the peak height and peak profile of the compound represented by Formula I-41 remained nearly unchanged, and no obvious release of Compound 3 was observed. It was demonstrated that the dipeptide bonds in the ISACs provided by the present application were not cleaved by cathepsins under conventional conditions, and distinct from traditional cleavable ISACs, the ISACs provided in the present application exerts pharmacological effects via a non-cleavable quaternary ammonium salt structure.

### Example 16: In vivo pharmacodynamic experiment of the ISAC represented by Formula II-13 in xenograft model

In the present example, the in vivo efficacy of the ISAC represented by Formula II-13 was evaluated in a xenograft animal model. The test drugs included II-13 at 3 mg/mL, II-13 at 6 mg/mL, the combination group of Herceptin (6 mg/mL) and compound 3 (0.7 mg/mL), and PBS (control group).

Female BALB/c nude mice aged 6 to 8 weeks were purchased from SPF (Beijing) Biotechnology Co., Ltd. Mice were housed in an SPF-grade animal care facility with 3 mice per cage. All animal studies followed ARRIVE guidelines. NCI-N87 cells (about 1×10⁷ cells, purchased from ATCC) were inoculated subcutaneously into mice. When tumor volumes reached about 120 mm³ or 180 mm³, mice bearing NCI-N87 xenograft were randomly grouped, with 6 mice per group. For mice bearing NCI-N87 xenograft, all test drugs were administered once per 4 days for a total of four administrations via tail vein injection. Starting on Day 0, the tumor size was measured with digital calipers twice weekly, and the general physical condition of the animals (including mental status, locomotor activity, hair appearance) were observed. The tumor volume was calculated by using the formula: 0.5 × L × W², wherein L and W represent the length and width of the tumor, respectively. At the termination of the experiment, mice were euthanized by cervical dislocation.

The experimental results were shown in FIG. 8. The results showed that both the two dose groups of the ISAC represented by Formula II-13 induced sustained complete tumor regression, and the antitumor inhibitory activity showed a dose-dependent relationship, the antitumor inhibitory activity exhibited dose dependence with statistically significant differences (**** P<0.0001). When the ISAC represented by Formula II-13 was administered at 3 mg/kg, the delivered payload dosage was 0.35 mg/kg, and compared with reported effective dosage of Compound 3 for tumor inhibition (1.5 mg/kg), the anti-tumor activity was markedly improved. Meanwhile, both two dose groups of the ISAC represented by Formula II-13 showed antitumor efficacy superior to that of the combination group (Herceptin 6mg/kg + Compound 30.7 mg/kg), further verifying its superior therapeutic efficacy relative to free small molecule drugs. In the present experiment, no obvious body weight loss was observed in all treated animals. These preliminary data demonstrated that due to the targeted property of the ISAC represented by Formula II-13, the systemic side effect of the payload was avoided, conferring favorable safety profiles.

### Example 17: In vivo pharmacodynamic experiment of the ISAC represented by Formula II-41 in xenograft model

In the present example the in vivo efficacy of the ISAC represented by Formula II-41 was evaluated in a xenograft animal model. The test drugs included II-41 at 6 mg/kg, II-41 at 3 mg/kg, II-41 at 1 mg/kg, the combination group of Herceptin (6 mg/kg) and compound 3 (0.7 mg/kg) combination, and PBS (control group).

Female C57BL/6J mice aged 6 to 8 weeks were purchased from China Nanjing Jeyao Technology Co., Ltd. All mice were housed under specific pathogen-free environment, and all the procedures were performed according to guidelines approved by Animal Ethics Committee, Institute of Medicinal Biotechnology, Chinese Academy of Medical Sciences (Ethics Approval Nos. IMB-20240912D801 and IMB-20241018D 801). In the in vivo experiment, E0771-HER2 tumor cells (2×10⁶ cells) were injected subcutaneously into the right abdomen of the C57BL/6J mice. Administration was initiated when the tumor volume reached 80 mm³, and PBS, TZ and dSA3 or II-41 (1 mg/kg, 3 mg/kg and 6 mg/kg) were intravenously injected once per week, for a total of 2 administrations. Tumor growth was monitored and recorded every 2 days, and mouse body weight was recorded every 3 days. The tumor volume was calculated by the formula: Volume = π/6 × tumor length × (tumor width)². At the termination of the experiment, the mice were euthanized by cervical dislocation.

The experimental results were shown in FIG. 9 to FIG. 11. The results showed that compared with the control group, the three dose groups of the ISAC represented by Formula II-41 produced significant tumor growth inhibition (*** P <0.001), and the antitumor inhibitory activity exhibited a dose-dependent relationship. Notably, administration of the ISAC represented by Formula II-41 at 6 mg/kg almost achieved complete tumor regression. Moreover, the three dose groups of the ISAC represented by Formula II-41 showed superior antitumor efficacy to the combination group (Herceptin 6mg/kg + Compound 30.7 mg/kg), which further proved that the ISAC represented by Formula I-41 exhibited superior therapeutic efficacy compared with free small molecule drugs. In the present experiment, no obvious body weight loss was observed in all treated animals. These preliminary data demonstrated that the ISAC represented by Formula II-41 had good tumor inhibitory effects with favorable safety profiles.

### Example 18: Immune memory effect of the ISAC represented by Formula II-41 in xenograft model

In the present example, the immunological memory effect of the ISAC represented by Formula II-41 was evaluated in an animal xenograft model. The test drugs included II-41 at 3 mg/kg, II-41 at 1 mg/kg, and PBS (control group).

In the immunological memory experiment, E0771-HER2 tumor cells were implanted into the right flank of mice, and when tumor size reached 50 mm³ to 60 mm³, mice were treated with the ISAC represented by Formula II-41. Administration was performed once per 3 days for a total of 3 administrations. On Day 10, complete tumor regression was observed in all ISAC treatment groups. For all mice in the control group, primary tumors on the right flank were surgically resected, followed by immediate surgical suture. On Day 14, 2×10⁶ E0771-HER2 cells were intravenously injected into the left flank of all mice to examine immune memory responses.

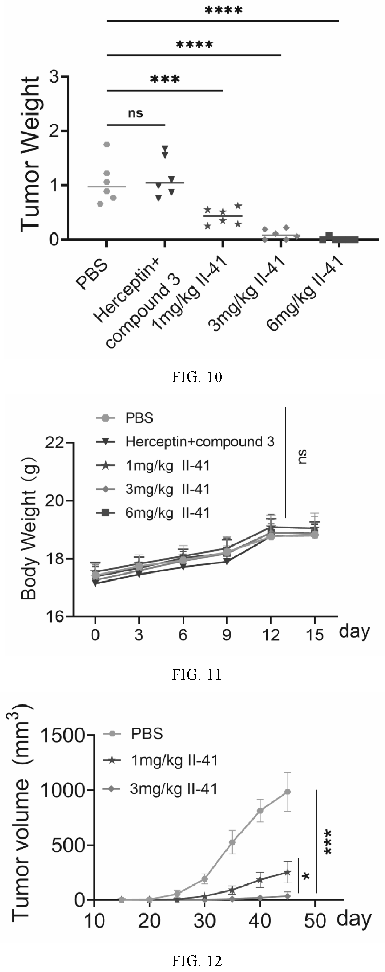

The experimental results were shown in FIG. 12 to FIG. 13. Compared with the control group, both ISAC treatment groups, including the II-41 at 3 mg/kg group and the III-41 at 1 mg/kg group, exhibited prominent immune memory effects, and after the second tumor cell inoculation, the tumor volumes of both treatment groups were markedly smaller than those of the PBS control group. Notably, the II-41 at 3 mg/kg group exhibited nearly no tumor recurrence after tumor rechallenge. No obvious body weight loss was observed in all animals of the treatment groups. The present example showed that mice receiving treatment with the ISAC represented by Formula II-41 developed immune memory, which effectively protects against tumor recurrence.

### Example 19: Pharmacodynamic experiment of the ISAC represented by Formula II-41 in SKOV3/PBMC co-culture cell line

In the present example, a co-culture assay consisting of SKOV3-GFP cancer cells and human peripheral blood mononuclear cells (PBMC) was adopted to evaluate the tumor-killing effect of the ISAC represented by Formula II-41, which functions by activating the STING pathway in PBMCs. The test drugs included II-41 at 16 nM, II-41 at 1.6 nM, Fab-blocked II-41 at 160 nM, Fc-blocked II-41 at 160 nM, compound 3 at 160 nM, compound 3 at 16 nM, and PBS (control group).

Cancer cell death following co-culture with immune cells was evaluated by the IncuCyte cancer cell killing assay. SKOV3-GFP cells were spread on a plate and incubated overnight, and on the next day, cells in each well were incubated with the test drugs at 37 ° C for 20 minutes, followed by addition of PBMCs (at a quantity twice the number of SKOV 3-GFP cells). The plate was then placed in an IncuCyte live cell analysis system housed within an incubator (37 °C, 5% CO₂). Scanning was performed every 2 hours for a total duration of 24 hours. The variation in the number of green fluorescent objects (cancer cells) over time was quantified by using IncuCyte S3 software (version 2022B).

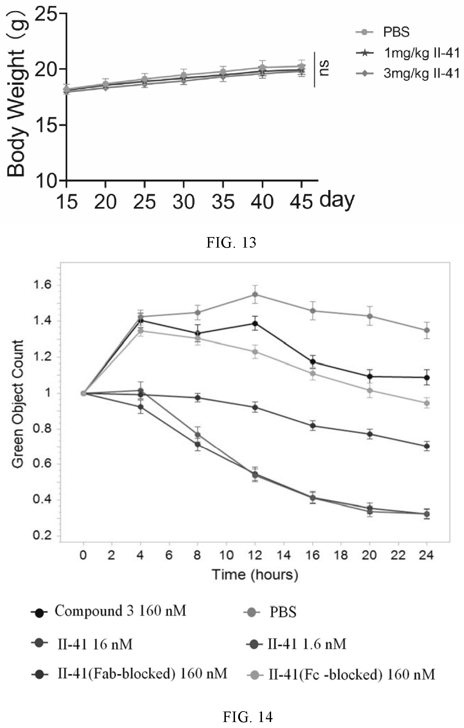

The experimental results were shown in FIG. 14. Compared to the control group, the ISAC treatment groups, including the II-41 at 16 nM group and the II-41 at 1.6 nM group, strongly activated PBMCs, and exhibited strong killing activity against SKOV3-GFP tumor cells (manifested by the loss of green fluorescence). Notably, the II-41 (1.6 nM) still exhibited markedly superior tumor cell killing activity, even though its concentration was 100-fold lower than that of compound 3 (160 nM), Fab-blocked II-41 (160 nM) and Fc-blocked II-41 (160 nM), indicating that the ISAC represented by Formula II-41 relies on combined immune activation mediated by its Fab and Fc domains, and its immune-stimulating effect was significantly better than that of free small molecules.

Directed to the problem of systemic side effects existing in the clinical application of immune agonists, cleavable ISACs were explored in the present application. An ISAC targeting the novel STING agonist diABZI-STING-agonist (Compound 3) was designed for the first time. The results of systemic evaluation experiments such as in vitro stability assessment of the ISAC (II-13), activation of downstream signaling of the cGAS-STING pathway, regulation of gene expression of related immune factors, and induction of Immunogenicity Cell Death (ICD), plasma stability, and in vivo anti-tumor drug efficacy in xenograft models, verified the application potentials of this drug release modality to a certain extent. The ISACs based on a cleavable linker have in vitro stability and immune activity which preliminarily meet the requirements of ADC drug development, accompanied by prominent in vivo and in vitro therapeutic efficacy and favorable safety profiles at therapeutic doses.

Finally, it should be illustrated that the above examples are intended only to illustrate the technical solution of the present application but not to limit it; although the present application has been described in detail with reference to preferred examples, one skill in the art should understand that modifications to the specific embodiments of the present application or equivalent substitutions to a part of the technical features may be made, and each of them, without departing from the spirit of the technical solution of the present application, should be covered in the scope of the technical solution as claimed in the present application.

## Claims

1. A compound represented by Formula II, a tautomer, a geometric isomer, an optical isomer, a polymorph or a pharmaceutically acceptable hydrate or solvate thereof, 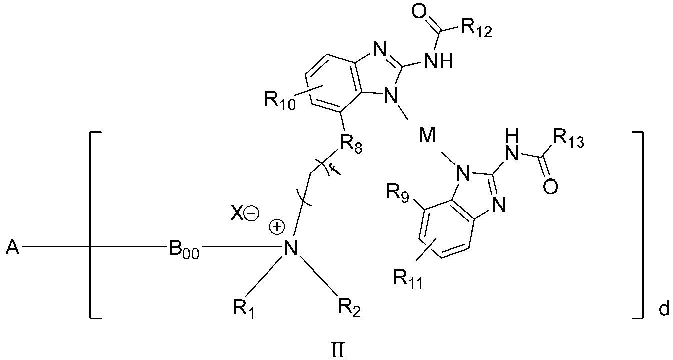 wherein:
A represents a biological macromolecule or a fragment thereof,
B₀₀ represents a linker, for conjugating A to the -NR₁R₂-group,
X represents a negatively charged anion,
R₁ and R₂ each independently are C₁₋₈ alkyl, C₁₋₈ alkyl cycloalkyl, C₄₋₈ cycloalkyl, 4- to 8-membered heterocycloalkyl, C₁₋₈ alkyl glycosyl, C₅₋₁₄ aryl, or 5- to 14-membered heteroaryl, or
R₁ and R₂, together with the nitrogen atom to which they are linked, form 5- to 7-membered heterocycloalkyl or heteroaryl, wherein the heterocycloalkyl contains one N atom and one, two or three other heteroatoms selected from the group consisting of N, O or S atom, the heterocyclyl or heteroaryl is optionally substituted with one, two, three or four R', and each R' independently is H, C₁₋₈ alkyl, C₁₋₈ alkoxy, fluoro, chloro, bromo, iodo, hydroxy, cyano, amino or nitro,
M is -(CH₂)ₒ-, -(CH₂)ₒ-CH=CH-(CH₂)ₚ-, -O-(CH₂)ₒ-, -NH-(CH₂)ₒ-, -O-(CH₂)ₒ-CH=CH-(CH₂)ₚ-O-, -NH-(CH₂)ₒ-CH=CH-(CH₂)ₚ-NH- or -CH₂-CHOH-CHOH-CH₂-, wherein o and p each independently are an integer from 0 to 9,
f is 0 or 1,
R₈ is -(CH₂)ₐ-, -(CH₂)ₐ-O-, -(CH₂)ₐ-S- or -(CH₂)ₐ-NH-, wherein a is an integer from 1 to 8,
R₉ is C₁₋₈ alkyl, C₁₋₈ alkoxy, C₁₋₈ alkylthio or C₁₋₈ alkylamino,
R₁₀ and R₁₁ each independently are C₁₋₄ alkyl, -CONR^{a}R^{b}, -CH₂NR^{a}R^{b}, -NR^{a}R^{b}, -NR^{a}C(=O)-R^{b}, - CH₂NR^{a}C(=O)-R^{b}, -C(=O)OCH₃ or -O-(CH₂)ₑ-OH, wherein R^{a} and R^{b} each independently are H, hydroxyl or C₁₋₄ alkyl, and e is an integer from 1 to 9,
R₁₂ and R₁₃ each independently are -(CH₂)_{g}-CH₃, -O-(CH₂)_{g}-OH, -O-(CH₂)_{g}-CH₃, -CONH₂, 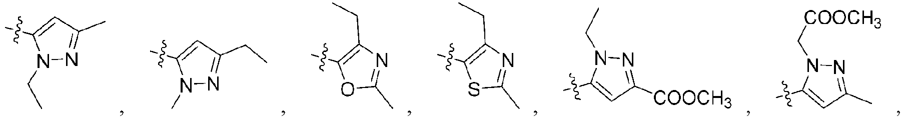 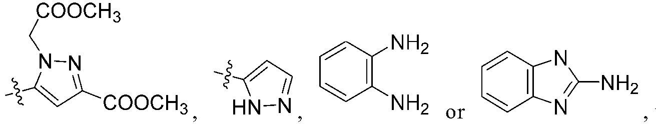 wherein g is a number from 1 to 9,
d is a number from 1 to 20.

2. The compound, a tautomer, a geometric isomer, an optical isomer, a polymorph or a pharmaceutically acceptable hydrate or solvate thereof according to claim 1, wherein R₁₀ is ortho, meta, or para of R₈; and/or R₁₁ is ortho, meta, or para of R₉;
preferably, R₁₀ is ortho of R₈, and R₁₁ is ortho of R₉.

3. The compound, a tautomer, a geometric isomer, an optical isomer, a polymorph or a pharmaceutically acceptable hydrate or solvate thereof according to claim 1 or 2, wherein the compound has a structure represented by Formula II-1, 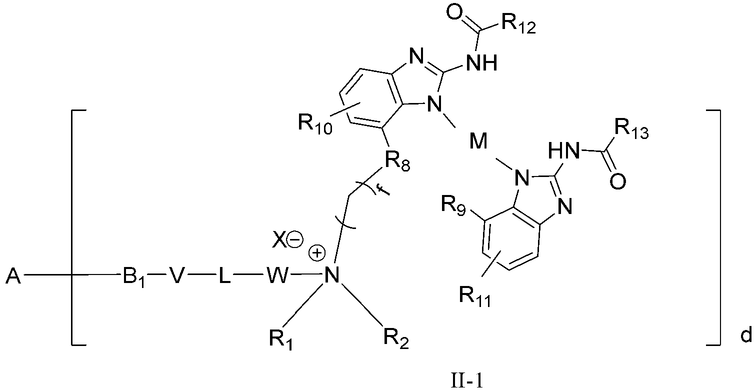
wherein A, X, R₁, R₂, f, R₈, R₉, R₁₀, R₁₁, R₁₂, R₁₃, and d are as defined in claim 1 or 2,
-B₁-V-L-W- represents a linker, for conjugating A to the -NR₁R₂- group, wherein B₁ is selected from the group consisting of 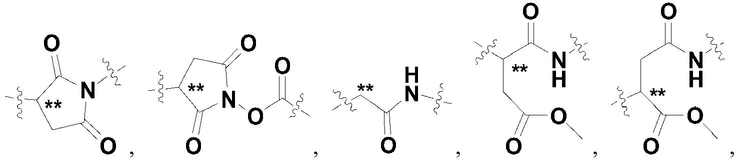 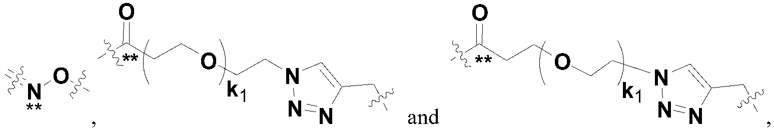 wherein k₁ is 1, 2, 3, 4, 5, 6, 7, 8, 9, 10, 11 or 12; V is 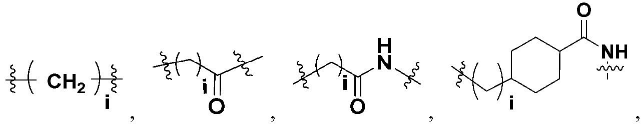 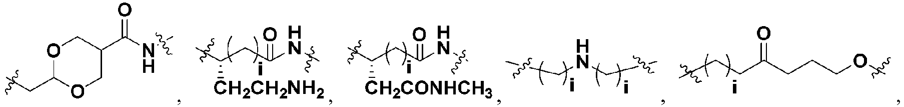 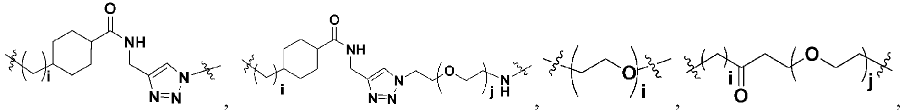 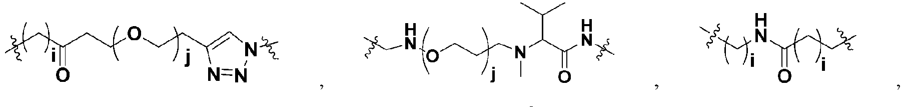 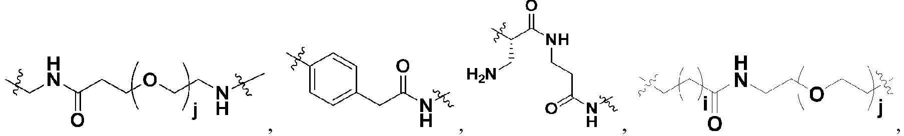 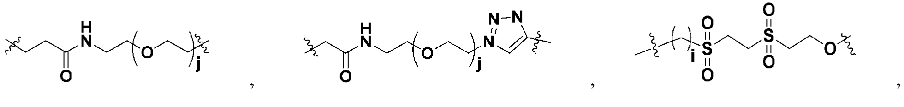 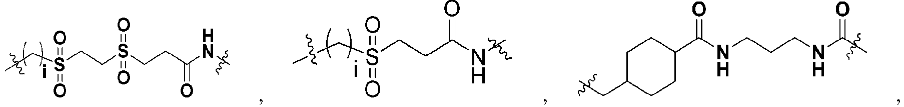 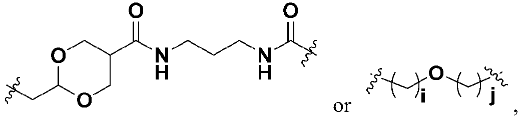 wherein each i independently is an integer from 0 to 12, each j independently is an integer from 0 to 12, or V is absent; L is -(CH₂CH₂O)ₖ-(CH₂)ₗ-, -CHZ-, -(CH₂)ₘ -, or 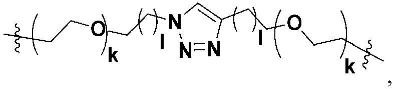 wherein Z is -(CH₂)ₙ-NHC(O)-(CH₂CH₂O)ₖ-CH₃, each k independently is an integer from 0 to 12, each l independently is an integer from 0 to 12, n is an integer from 0 to 12, m is an integer from 0 to 30, or L is absent; W is 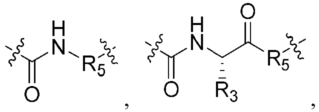 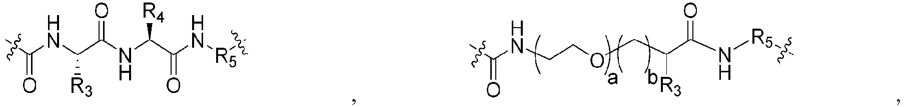 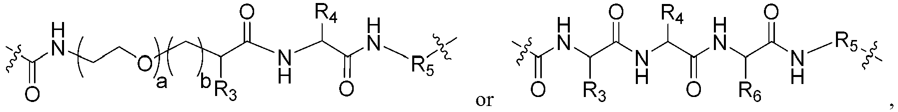 wherein R₃, R₄ and R₆ each independently are H, methyl, ethyl, n-propyl, iso-propyl, n-butyl, sec-butyl, iso-butyl, tert-butyl, phenyl, cyclobutyl, cyclopentyl, cyclohexyl, -CH₂(C₆H₅), -(CH2)_{c}-NH₂, -(CH₂)_{c}-NHC(NH)NH₂, -CHCH(CH₃)CH₃ or -(CH₂)_{c}-NHC(=O)NH₂, or 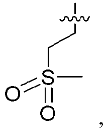 wherein c is a number from 0 to 8; R₅ is 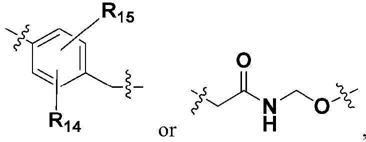 R₁₄ and R₁₅ each independently are H, methyl, ethyl, n-propyl, iso-propyl, n-butyl, sec-butyl, iso-butyl, tert-butyl, phenyl, cyclobutyl, cyclopentyl, cyclohexyl, - CH₂(C₆H₅), -(CH₂)ₕ-NH₂ or -(CH₂)ₕ, and each h independently is a number from 0 to 8,
A is conjugated to the position ** of the group B₁ via the S atom or the N atom in the biological macromolecule or a fragment thereof.

4. The compound, a tautomer, a geometric isomer, an optical isomer, a polymorph or a pharmaceutically acceptable hydrate or solvate thereof according to any one of claims 1 to 3, **characterized in** any one or more of the following i) to x):
i) X is Cl⁻, Br⁻, I⁻, NO₃⁻, HCOO⁻, CH₃COO⁻, CF₃COO⁻, SO₄²⁻, PO₄,³⁻, HPO₄²⁻ or H₂PO₄⁻; preferably, X is Cl⁻, Br⁻, I⁻, NO₃⁻, HCOO⁻, CH₃COO⁻, or CF₃COO⁻; preferably, X is Cl⁻, HCOO⁻, CH₃COO⁻, or CF₃COO⁻; preferably, X is Cl⁻, Br⁻, or I⁻; preferably, X is Cl⁻;
ii) R₁ and R₂ each independently is C₁₋₈ alkyl, or
R₁ and R₂, together with the nitrogen atom to which they are linked, form 5- to 7-membered heterocycloalkyl, wherein the heterocycloalkyl contains one N atom and one, two or three other heteroatoms selected from the group consisting of N, O or S atom, the heterocycloalkyl is optionally substituted with one, two, three or four R', and each R' independently is H, C₁₋₈ alkyl, C₁₋₈ alkoxy, fluoro, chloro, bromo, iodo, hydroxy, cyano, amino or nitro; preferably, R₁ and R₂, together with the nitrogen atom to which they are linked, form pyrrolidinyl, piperazinyl, piperidinyl, morpholinyl, or thiomorpholinyl. preferably, R₁ and R₂, together with the nitrogen atom to which they are linked, form 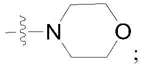 or
R₁ and R₂, together with the nitrogen atom to which they are linked, form 5- to 7-membered heteroaryl, wherein the heteroaryl contains one N atom and one, two or three other heteroatoms selected from the group consisting of N, O or S atom, the heteroaryl is optionally substituted with one, two, three or four R', and each R' independently is H, C₁₋₈ alkyl, C₁₋₈ alkoxy, fluoro, chloro, bromo, iodo, hydroxy, cyano, amino or nitro; preferably, R₁ and R₂, together with the nitrogen atom to which they are linked, form imidazolyl, triazolyl, indolyl, tetrazolyl, pyridyl, pteridinyl, pyrimidinyl, quinolinyl, isoquinolinyl, quinazolinyl, quinoxalinyl, or thiazinyl.
iii) M is -(CH₂)ₒ-CH=CH-(CH₂)ₚ-, wherein o and p each independently are an integer from 0 to 9; preferably, o and p each independently are an integer from 1 to 6, such as 1, 2, 3, 4, 5 or 6;
iv) f is 0, or f is 1;
v) R₈ is -(CH₂)ₐ-O-, -(CH₂)ₐ-S- or -(CH₂)ₐ-NH-; preferably, R₈ is -(CH₂)ₐ-O-, preferably, wherein a is as defined in claim 1; further preferably, a is an integer from 1 to 6, such as 1, 2, 3, 4, 5 or 6;
vi) R₉ is C₁₋₈ alkoxy, C₁₋₈ alkylthio or C₁₋₈ alkylamino; preferably, R₉ is C₁₋₈ alkoxy; preferably, R₉ is C₁₋₆ alkoxy; preferably, R₉ is C₁₋₄ alkoxy; preferably, R₉ is methoxy, ethoxy, n-propoxy, iso-propoxy, n-butoxy, tert-butoxy, sec-butoxy, n-pentyloxy, n-hexyloxy or 1,2-dimethylbutoxy; preferably, R₉ is methoxy, ethoxy, n-propoxy or n-butoxy; preferably, R₉ is C₁₋₆ alkylthio ; preferably, R₉ is C₁₋₄ alkylthio; preferably, R₉ is methylthio, ethylthio, propylthio or butylthio; preferably, R₉ is C₁₋₆ alkylamino; preferably, R₉ is C₁₋₄ alkylamino; preferably, R₉ is methylamino, ethylamino, propylamino or butylamino;
vii) R₁₀ and R₁₁ each independently are -CONR^{a}R^{b}, -CH₂NR^{a}R^{b} or -CH₂NR^{a}C(=O)-R^{b}, preferably, R₁₀ and R₁₁ each independently are -CONR^{a}R^{b}, wherein R^{a} and R^{b}are as defined in claim 1; preferably, R^{a} and R^{b} each independently are H or C₁₋₄ alkyl; preferably, R^{a} and R^{b} each independently are H; preferably, R^{a} and R^{b} each independently are methyl, ethyl, n-propyl or n-butyl;
viii) R₁₂ and R₁₃ each independently are 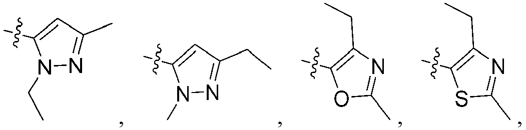 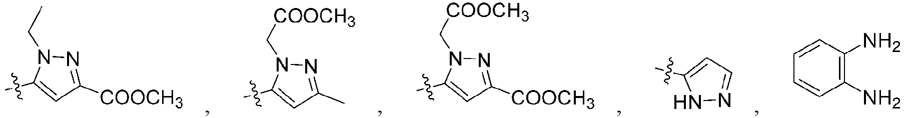 or 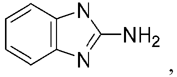 preferably, R₁₂ and R₁₃ each independently are 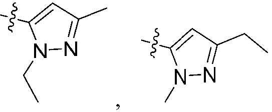 or 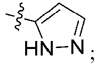 preferably, R₁₂ and R₁₃ each independently are 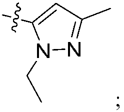
ix) d is a number from 1 to 8; preferably, d is a number from 1 to 6, such as about 1, about 2, about 3, about 4, about 5 or about 6;
x) A is an antibody or an antigen-binding fragment thereof, a polypeptide, a protein or an antigen;
preferably, A is an antibody or an antigen-binding fragment thereof, a protein or an antigen;
preferably, A is an antibody or an antigen-binding fragment thereof, a polypeptide or a protein capable of specifically binding to a target;
preferably, A is an antibody or an antigen-binding fragment thereof or a protein capable of specifically binding to a target;
preferably, A is an antibody or an antigen-binding fragment thereof capable of specifically binding to a target;
preferably, A is an antigen;
preferably, A is a protein capable of specifically binding to a target;
preferably, the antibody is selected from the group consisting of a monoclonal antibody (MAB), a polyclonal antibody, an IgG antibody, a murine antibody, a rabbit antibody, a humanized antibody, a fully human antibody, a chimeric antibody (e.g., a human-mouse chimeric antibody), a bispecific antibody, a multispecific antibody, and a probody;
preferably, the antibody-binding fragment is selected from the group consisting of a single chain antibody, dAb, a complementarity determining region fragment, Fv, single chain Fv (scfv), Fd, Fab, Fab', F(ab')₂ and VHH (a nanometer antibody, Nanobody or Nb);
preferably, the protein is albumin, preferably is human serum albumin;
preferably, A is a monoclonal antibody or an antigen-binding fragment thereof;
preferably, A is a monoclonal antibody or an antigen-binding fragment thereof with thiol or amino group as conjugating sites, or a site-directed mutated or modified monoclonal antibody or an antigen-binding fragment thereof with thiol or amino group as conjugating sites;
preferably, A is selected from the group consisting of: humanized anti-HER2 monoclonal antibody mil40, trastuzumab (HERCEPTIN), pertuzumab (PERJETA), cetuximab (ERBITUX), panitumumab (VECTIBIX), rituximab (RITUXAN), alemtuzumab (CAMPATH), ibritumomab tiuxetan (ZEVALIN), tositumomab (BEXXAR), ofatumumab (ARZERRA), bevacizumab (AVASTIN), ipilimumab (YERVOY), denosumab (XGEVA), pembrolizumab (KEYTRUDA), nivolumab (Opdivo), avelumab (Bavencio), atezolizumab (Tecentriq), durvalumab (Imfinzi), sacituzumab, rovalpituzumab, antibacterial antibody, a biological analog thereof or an antigen-binding fragment thereof, and the above monoclonal antibodies or an antigen-binding fragment thereof that are site-directed mutated or modified;
preferably, A is trastuzumab or an antigen-binding fragment thereof.

5. The compound, a tautomer, a geometric isomer, an optical isomer, a polymorph or a pharmaceutically acceptable hydrate or solvate thereof according to claim 3 or 4, **characterized in** any one or more of the following i) to iv):
i) B₁ is 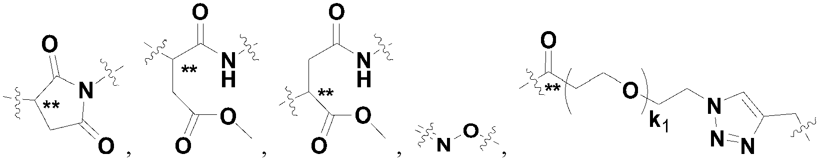 or 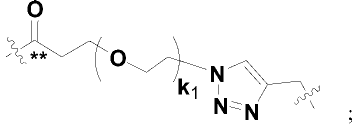 preferably, B₁ is 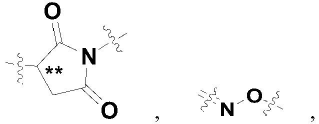 or 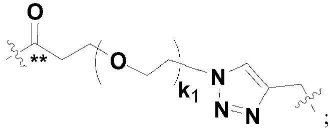 preferably, B₁ is 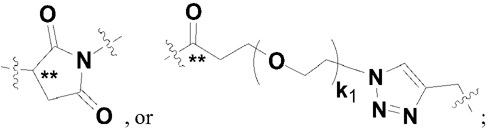 wherein, k₁ is as defined in claim 3; preferably, k₁ is 1, 2, 3, 4, 5, 6, 7, 8, 9 or 10; further preferably, k₁ is 1, 2, 3, 4, 5, 6, 7 or 8; more further preferably, k₁ is 2, 3, 4, 5 or 6;
ii) V is 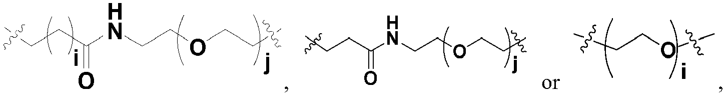 wherein i and j are as defined in claim 3; preferably, i and j each independently are 1, 2, 3, 4, 5, 6, 7, 8, 9 or 10; further preferably, i and j each independently are 1, 2, 3, 4, 5, 6, 7 or 8; more further preferably, i and j each independently are 1, 2, 3, 4, 5 or 6;
iii) L is -(CH₂CH₂O)ₖ-(CH₂)₁-, -CHZ-, or -(CH₂)ₘ -, or L is absent, wherein Z is -(CH₂)ₙ-NHC(O)-(CH₂CH₂O)ₖ-CH₃, and k, l, m, and n are as defined in claim 3; preferably, each k independently is an integer from 0 to 10 (such as 0, 1, 2, 3, 4, 5, 6, 7, 8, 9 or 10), each l independently is an integer from 0 to 10 (such as 0, 1, 2, 3, 4, 5, 6, 7, 8, 9 or 10), n is an integer from 0 to 10 (such as 0, 1, 2, 3, 4, 5, 6, 7, 8, 9 or 10), and m is an integer from 0 to 20 (preferably m is an integer from 0 to 16 (such as 0, 1, 2, 3, 4, 5, 6, 7, 8, 9, 10, 11, 12, 13, 14, 15, 16)); preferably, L is absent;
iv) W is 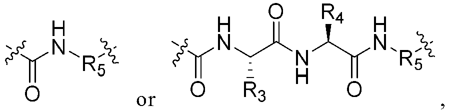 wherein R₃, R₄ and R₅ are as defined in claim 3; preferably, R₃ and R₄ each independently are methyl, ethyl, n-propyl, iso-propyl or -(CH₂)_{c}-NHC(=O)NH₂, wherein c is as defined in claim 3; preferably, R₅ is 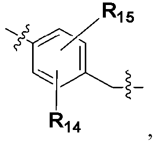 wherein R₁₄ and R₁₅ are as defined in claim 3;
preferably, R₃ and R₄ each independently are methyl, iso-propyl or -(CH₂)_{c}- NHC(=O)NH₂;
preferably, c is an integer from 0 to 6, such as 0, 1, 2, 3, 4, 5, or 6;
preferably, R₁₄ and R₁₅ each independently are H, methyl, ethyl, n-propyl, iso-propyl, n-butyl, sec-butyl, iso-butyl or tert-butyl; preferably, R₁₄ and R₁₅ each independently are H, methyl, ethyl or n-propyl; preferably, R₁₄ and R₁₅ each independently are H or methyl; preferably, R₁₄ and R₁₅ each independently are H;
preferably, -V-L-W- is 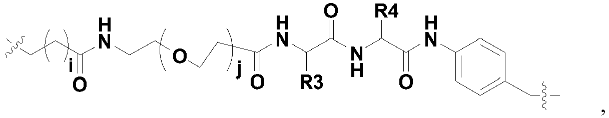 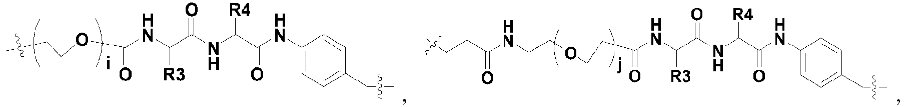 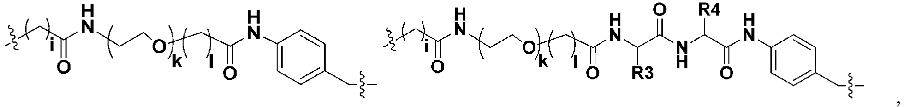 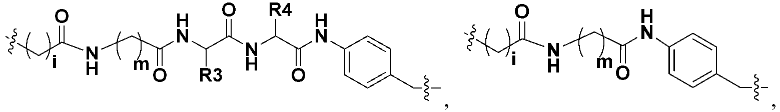 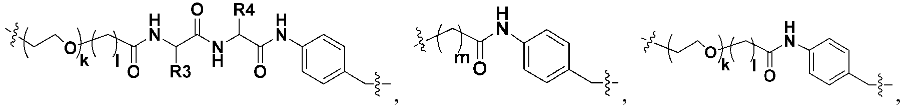 or 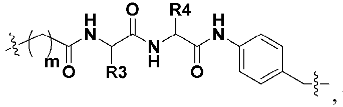 wherein i, j, k, l, m, R₃, and R₄ are as defined above;
preferably, -V-L-W- is 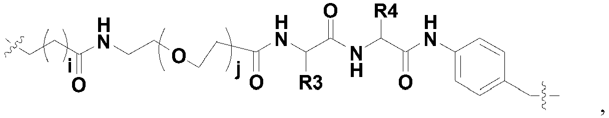 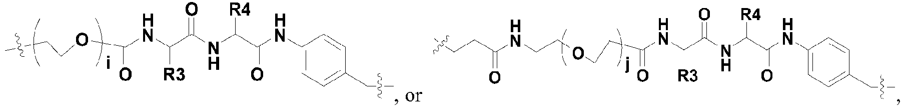 wherein i, j, k, l, m, R₃, and R₄ are as defined above.

6. The compound, a tautomer, a geometric isomer, an optical isomer, a polymorph or a pharmaceutically acceptable hydrate or solvate thereof according to any one of claims 3 to 5, wherein the compound has a structure represented by Formula II-2 or II-3, 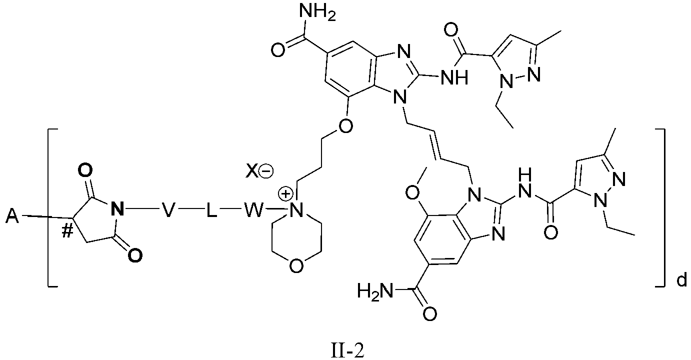 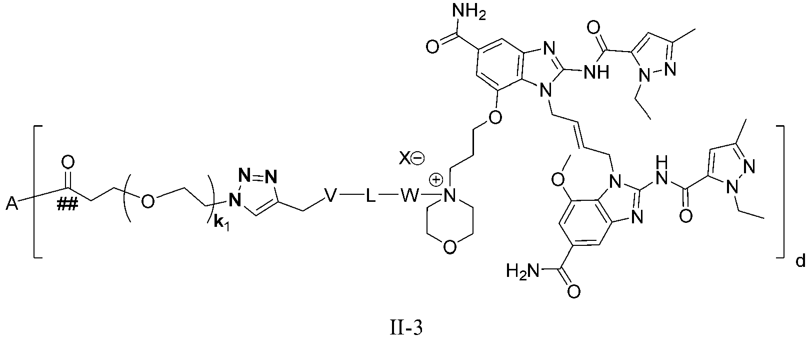 wherein A, X, V, L, W, and k₁ are as defined in any one of claims 3 to 5, A is conjugated to the position # via the S atom in the biological macromolecule or a fragment thereof, or A is conjugated to a position ## via the N atom in the biological macromolecule or a fragment thereof.

7. The compound, a tautomer, a geometric isomer, an optical isomer, a polymorph or a pharmaceutically acceptable hydrate or solvate thereof according to any one of claims 1 to 6, wherein the compound is selected from the group consisting of: 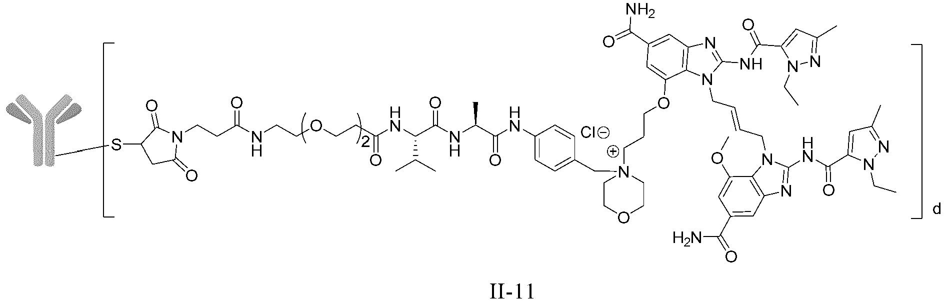 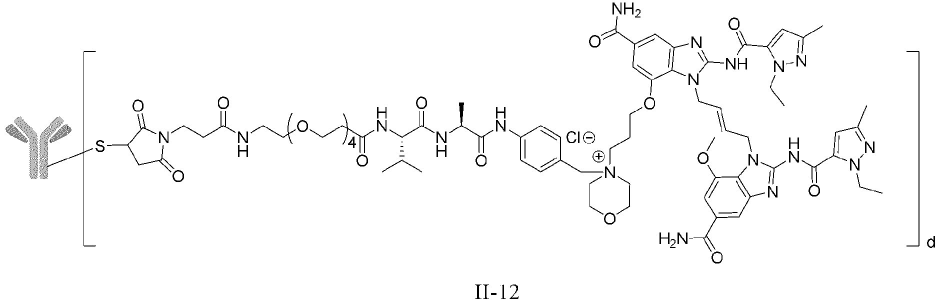 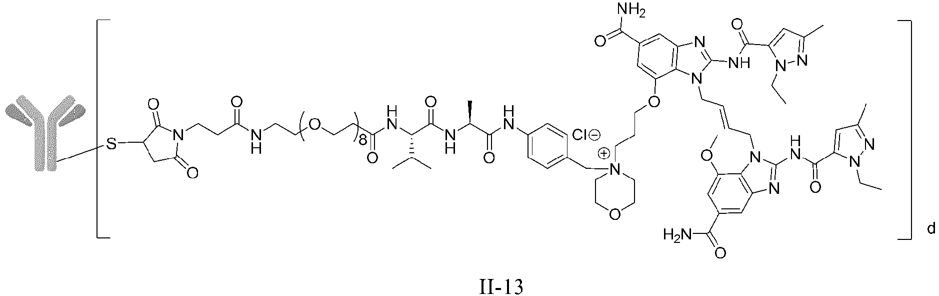 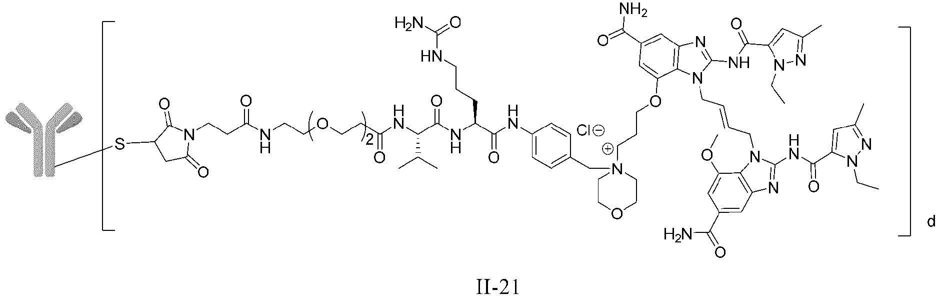 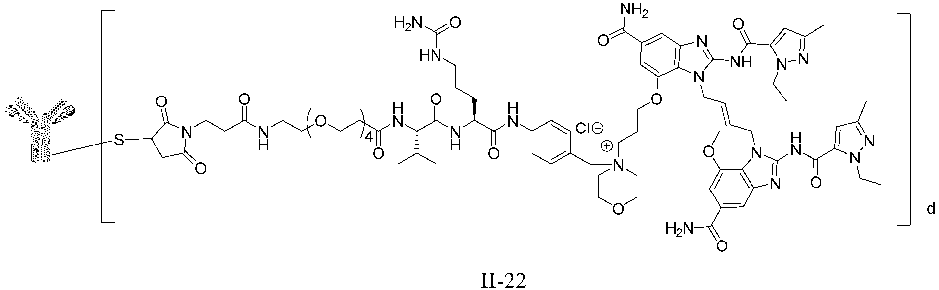 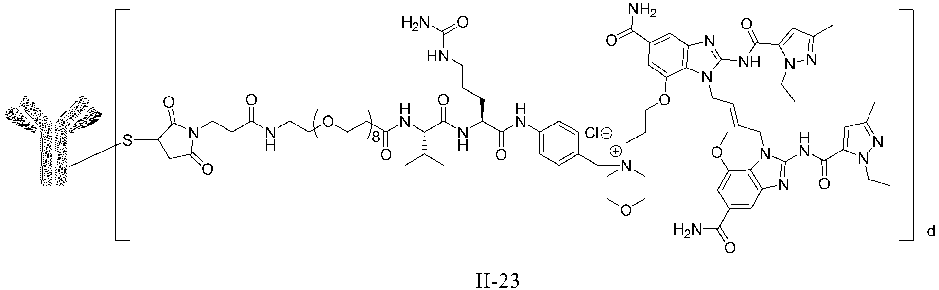 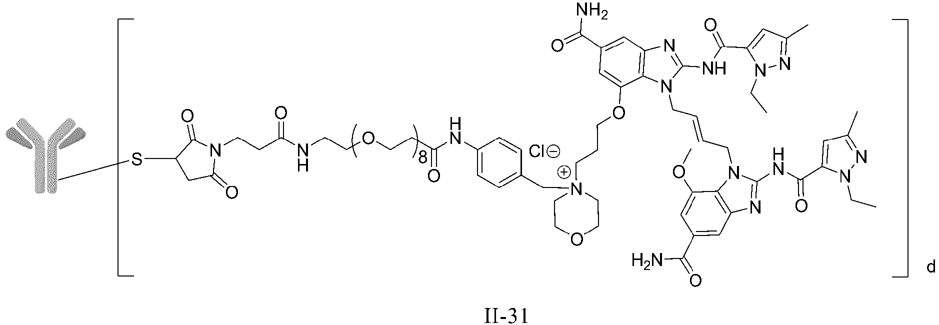 and 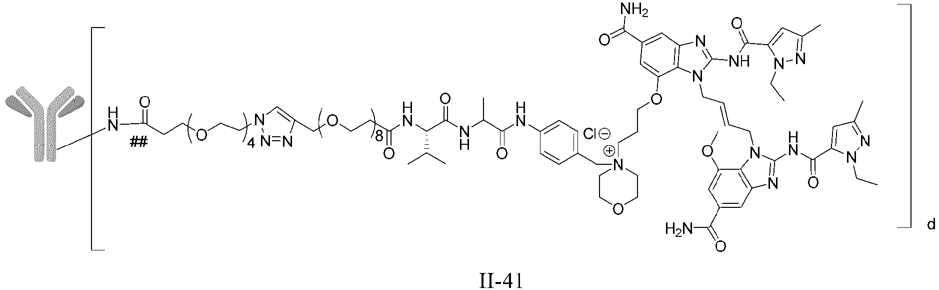 wherein, 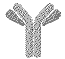 represents A, A and d are as defined in any one of claims 1 to 6, preferably, the S atom linked to A is derived from a sulfur atom in A (e.g., a sulfur atom in a cysteine residue), and the - NH- group linked to A is derived from an amino group in A (e.g., a side-chain amino group of lysine, arginine, asparagine, or glutamine residue).

8. A compound represented by Formula I, a tautomer, a geometric isomer, an optical isomer, a polymorph or a pharmaceutically acceptable hydrate or solvate thereof, 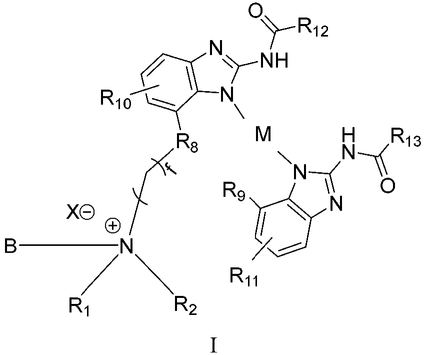 wherein:
B represents a linker, for conjugating the -NR₁R₂- group to a biological macromolecule or a fragment thereof,
X represents a negatively charged anion,
R₁ and R₂ each independently are C₁₋₈ alkyl, C₁₋₈ alkyl cycloalkyl, C₄₋₈ cycloalkyl, 4- to 8-membered heterocycloalkyl, C₁₋₈ alkyl glycosyl, C₅₋₁₄ aryl, 5- to 14-membered heteroaryl, or
R₁ and R₂, together with the nitrogen atom to which they are linked, form 5- to 7-membered heterocycloalkyl or heteroaryl, wherein the heterocycloalkyl contains one N atom and one, two or three other heteroatoms selected from the group consisting of N, O or S atom, the heterocyclyl or heteroaryl is optionally substituted with one, two, three or four R', and each R' independently is H, C₁₋₈ alkyl, C₁₋₈ alkoxy, fluoro, chloro, bromo, iodo, hydroxy, cyano, amino or nitro,
M is -(CH₂)ₒ-, -(CH₂)ₒ-CH=CH-(CH₂)ₚ-, -O-(CH₂)ₒ-, -NH-(CH₂)ₒ-, -O-(CH₂)ₒ-CH=CH-(CH₂)ₚ-O-, -NH-(CH₂)ₒ-CH=CH-(CH₂)ₚ-NH- or -CH₂-CHOH-CHOH-CH₂-, wherein o and p each independently are an integer from 0 to 9,
f is 0 or 1,
R₈ is -(CH₂)ₐ-, -(CH₂)ₐ-O-, -(CH₂)ₐ-S- or -(CH₂)ₐ-NH-, wherein a is an integer from 1 to 8,
R₉ is C₁₋₈ alkyl, C₁₋₈ alkoxy, C₁₋₈ alkylthio or C₁₋₈ alkylamino,
R₁₀ and R₁₁ each independently are C₁₋₄ alkyl, -CONR^{a}R^{b}, -CH₂NR^{a}R^{b}, -NR^{a}R^{b}, -NR^{a}C(=O)-R^{b}, - CH₂NR^{a}C(=O)-R^{b}, -C(=O)OCH₃ or -O-(CH₂)ₑ-OH, wherein R^{a} and R^{b} each independently are H, hydroxyl or C₁₋₄ alkyl, and e is an integer from 1 to 9,
R₁₂ and R₁₃ each independently are -(CH₂)_{g}-CH₃, -O-(CH₂)_{g}-OH, -O-(CH₂)_{g}-CH₃, -CONH₂, 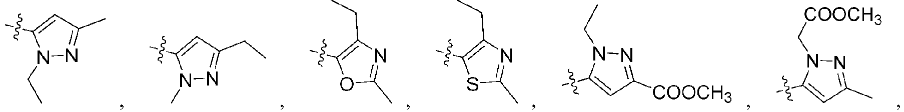 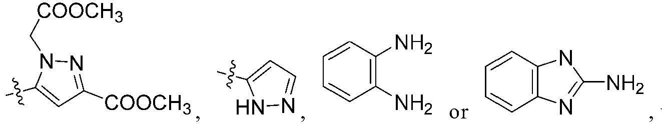 wherein g is a number from 1 to 9.

9. The compound, a tautomer, a geometric isomer, an optical isomer, a polymorph or a pharmaceutically acceptable hydrate or solvate thereof according to claim 8, wherein R₁₀ is ortho, meta, or para of R₈; and/or R₁₁ is ortho, meta, or para of R₉;
preferably, R₁₀ is ortho of R₈, and R₁₁ is ortho of R₉.

10. The compound, a tautomer, a geometric isomer, an optical isomer, a polymorph or a pharmaceutically acceptable hydrate or solvate thereof according to claim 8 or 9, wherein the compound has a structure represented by Formula I-1, 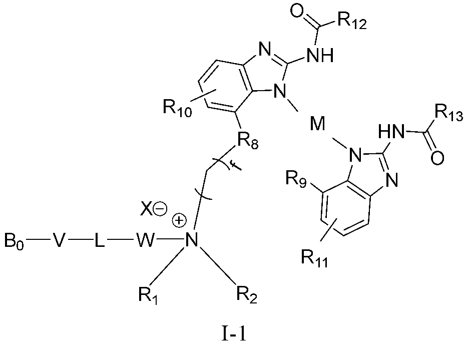
wherein X, R₁, R₂, f, R₈, R₉, R₁₀, R₁₁, R₁₂, R₁₃, and d are as defined in claim 8 or 9,
B₀-V-L-W- represents a linker, for conjugating the -NR₁R₂- group to a biological macromolecule or a fragment thereof, wherein B₀ is selected from the group consisting of 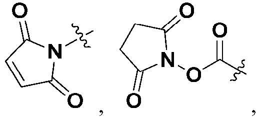 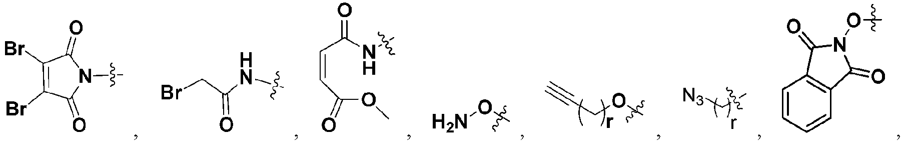 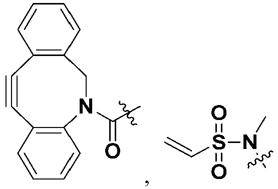 or H, wherein r is an integer from 1 to 4; V is 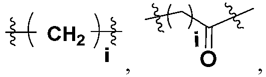 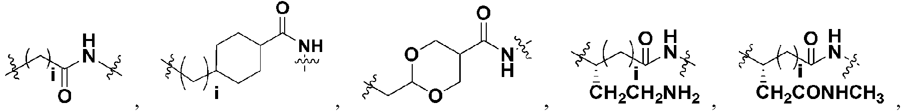 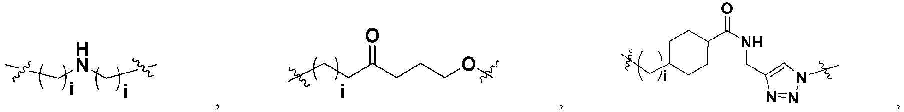 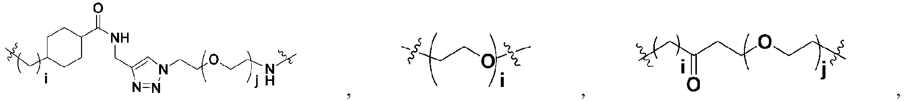 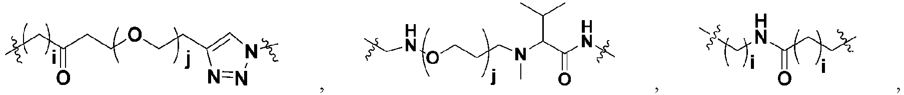 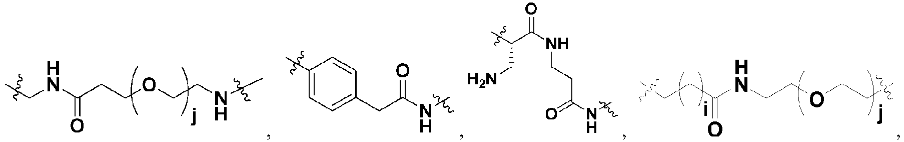 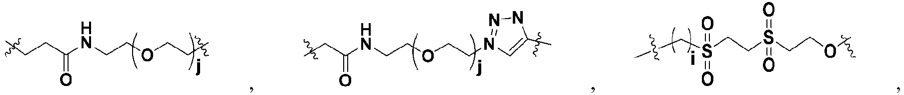 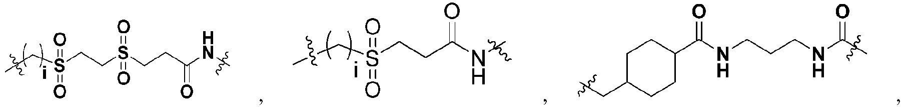 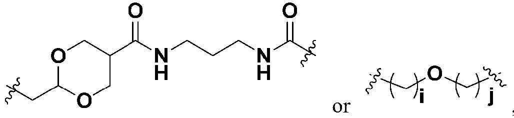 wherein each i independently is an integer from 0 to 12, each j independently is an integer from 0 to 12, or V is absent; L is -(CH₂CH₂O)ₖ-(CH₂)₁-, -CHZ-, -(CH₂)ₘ -, or 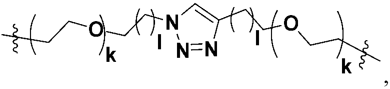 wherein Z is -(CH₂)ₙ-NHC(O)-(CH₂CH₂O)ₖ-CH₃, each k independently is an integer from 0 to 12, each l independently is an integer from 0 to 12, n is an integer from 0 to 12, m is an integer from 0 to 30, or L is absent; W is 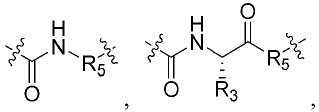 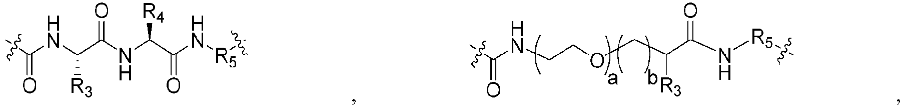 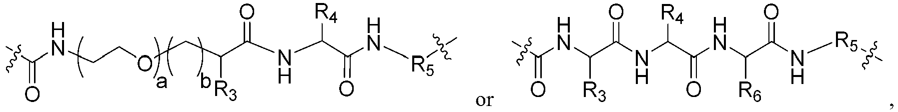 wherein R₃, R₄ and R₆ each independently are H, methyl, ethyl, n-propyl, iso-propyl, n-butyl, sec-butyl, iso-butyl, tert-butyl, phenyl, cyclobutyl, cyclopentyl, cyclohexyl, -CH₂(C₆H₅), -(CH₂)_{c}-NH₂, -(CH₂)_{c}-NHC(NH)NH₂, -CHCH(CH₃)CH₃ or -(CH₂)_{c}-NHC(=O)NH₂, or 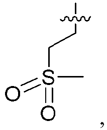 wherein c is a number from 0 to 8; R₅ is 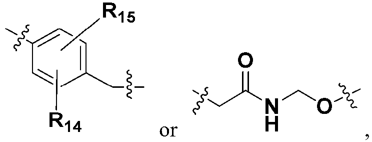 R₁₄ and R₁₅ each independently are H, methyl, ethyl, n-propyl, iso-propyl, n-butyl, sec-butyl, iso-butyl, tert-butyl, phenyl, cyclobutyl, cyclopentyl, cyclohexyl, - CH₂(C₆H₅), -(CH₂)ₕ-NH₂ or -(CH₂)ₕ, and each h independently is a number from 0 to 8.

11. The compound, a tautomer, a geometric isomer, an optical isomer, a polymorph or a pharmaceutically acceptable hydrate or solvate thereof according to any one of claims 8 to 10, **characterized in** any one or more of the following i) to viii):
i) X is Cl⁻, Br⁻, I⁻, NO₃⁻, HCOO⁻, CH₃COO⁻, CF₃COO⁻, SO₄²⁻, PO₄,³⁻, HPO₄²⁻ or H₂PO₄⁻; preferably, X is Cl⁻, Br⁻, I⁻, NO₃⁻, HCOO⁻, CH₃COO⁻, or CF₃COO⁻; preferably, X is Cl⁻, HCOO⁻, CH₃COO⁻, or CF₃COO⁻; preferably, X is Cl⁻, Br⁻, or I⁻; preferably, X is Cl⁻;
ii) R₁ and R₂ each independently are C₁₋₈ alkyl, or
R₁ and R₂, together with the nitrogen atom to which they are linked, form 5- to 7-membered heterocycloalkyl, wherein the heterocycloalkyl contains one N atom and one, two or three other heteroatoms selected from the group consisting of N, O or S atom, the heterocycloalkyl is optionally substituted with one, two, three or four R', and each R' independently is H, C₁₋₈ alkyl, C₁₋₈ alkoxy, fluoro, chloro, bromo, iodo, hydroxy, cyano, amino or nitro; preferably, R₁ and R₂, together with the nitrogen atom to which they are linked, form pyrrolidinyl, piperazinyl, piperidinyl, morpholinyl, or thiomorpholinyl; preferably, R₁ and R₂, together with the nitrogen atom to which they are linked, form 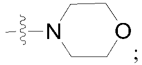 or
R₁ and R₂, together with the nitrogen atom to which they are linked, form 5- to 7-membered heteroaryl, wherein the heteroaryl contains one N atom and one, two or three other heteroatoms selected from the group consisting of N, O or S atom, the heteroaryl is optionally substituted with one, two, three or four R', and each R' independently is H, C₁₋₈ alkyl, C₁₋₈ alkoxy, fluoro, chloro, bromo, iodo, hydroxy, cyano, amino or nitro; preferably, R₁ and R₂, together with the nitrogen atom to which they are linked, form imidazolyl, triazolyl, indolyl, tetrazolyl, pyridyl, pteridinyl, pyrimidinyl, quinolinyl, isoquinolinyl, quinazolinyl, quinoxalinyl, or thiazinyl;
iii) M is -(CH₂)ₒ-CH=CH-(CH₂)ₚ-, wherein o and p each independently are an integer from 0 to 9; preferably, o and p each independently are an integer from 1 to 6, such as 1, 2, 3, 4, 5 or 6;
iv) f is 0, or f is 1;
v) R₈ is -(CH₂)ₐ-O-, -(CH₂)ₐ-S- or -(CH₂)ₐ-NH-; preferably, R₈ is -(CH₂)ₐ-O-, preferably, wherein a is as defined in claim 1; further preferably, a is an integer from 1 to 6, such as 1, 2, 3, 4, 5 or 6;
vi) R₉ is C₁₋₈ alkoxy, C₁₋₈ alkylthio or C₁₋₈ alkylamino; preferably, R₉ is C₁₋₈ alkoxy; preferably, R₉ is C₁₋₆ alkoxy; preferably, R₉ is C₁₋₄ alkoxy; preferably, R₉ is methoxy, ethoxy, n-propoxy, iso-propoxy, n-butoxy, tert-butoxy, sec-butoxy, n-pentyloxy, n-hexyloxy or 1,2-dimethylbutoxy; preferably, R₉ is methoxy, ethoxy, n-propoxy or n-butoxy; preferably, R₉ is C₁₋₆ alkylthio; preferably, R₉ is C₁₋₄ alkylthio; preferably, R₉ is methylthio, ethylthio, propylthio or butylthio; preferably, R₉ is C₁₋₆ alkylamino; preferably, R₉ is C₁₋₄ alkylamino; preferably, R₉ is methylamino, ethylamino, propylamino or butylamino;
vii) R₁₀ and R₁₁ each independently are -CONR^{a}R^{b}, -CH₂NR^{a}R^{b} or -CH₂NR^{a}C(=O)-R^{b}, preferably, R₁₀ and R₁₁ each independently are -CONR^{a}R^{b}, wherein R^{a} and R^{b} are as defined in claim 1; preferably, R^{a} and R^{b} each independently are H or C₁₋₄ alkyl; preferably, R^{a} and R^{b} each independently are H; preferably, R^{a} and R^{b} each independently are methyl, ethyl, n-propyl or n-butyl;
viii) R₁₂ and R₁₃ each independently are 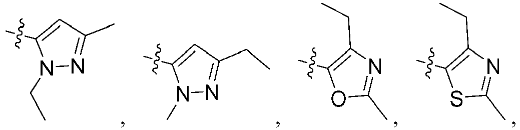 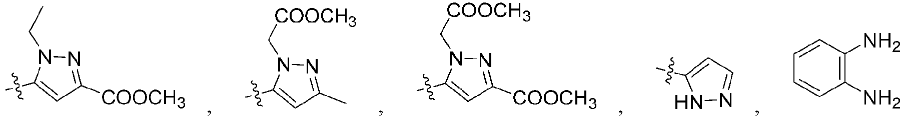 or 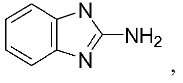 preferably, R₁₂ and R₁₃ each independently are 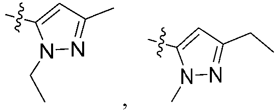 or 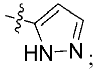 preferably, R₁₂ and R₁₃ each independently are

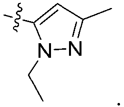

12. The compound, a tautomer, a geometric isomer, an optical isomer, a polymorph or a pharmaceutically acceptable hydrate or solvate thereof according to claim 10 or 11, **characterized in** any one or more of the following i) to iv):
i) B₀ is 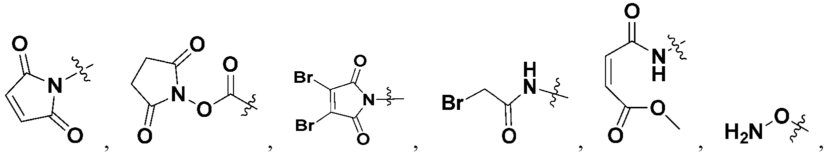 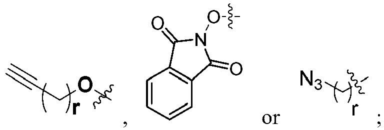 preferably, B₀ is 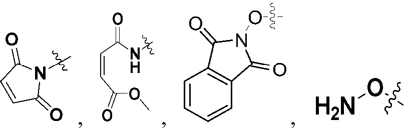 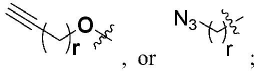 preferably, B₀ is 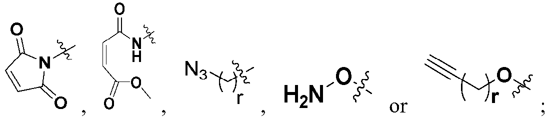 preferably, B₀ is 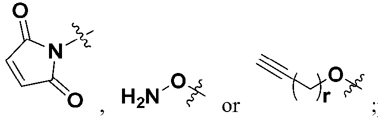 preferably, B₀ is 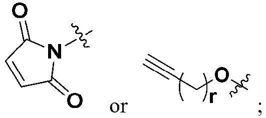 wherein, r is as defined in claim 10; preferably, r is 1, 2, 3 or 4; preferably, r is 1 or 2;
ii) V is 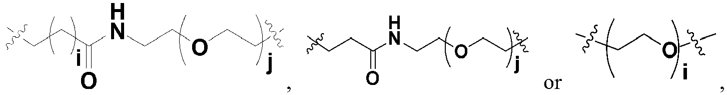 wherein i and j are as defined in claim 3; preferably, i and j each independently are 1, 2, 3, 4, 5, 6, 7, 8, 9 or 10; further preferably, i and j each independently are 1, 2, 3, 4, 5, 6, 7 or 8; more further preferably, i and j each independently are 1, 2, 3, 4, 5 or 6;
iii) L is -(CH₂CH₂O)ₖ-(CH₂)₁-, -CHZ-, or -(CH₂)ₘ -, or L is absent, wherein Z is -(CH₂)ₙ-NHC(O)-(CH₂CH₂O)ₖ-CH₃, and k, l, m, and n are as defined in claim 3; preferably, each k independently is an integer from 0 to 10 (such as 0, 1, 2, 3, 4, 5, 6, 7, 8, 9 or 10), each l independently is an integer from 0 to 10 (such as 0, 1, 2, 3, 4, 5, 6, 7, 8, 9 or 10), n is an integer from 0 to 10 (such as 0, 1, 2, 3, 4, 5, 6, 7, 8, 9 or 10), and m is an integer from 0 to 20 (preferably m is an integer from 0 to 16 (such as 0, 1, 2, 3, 4, 5, 6, 7, 8, 9, 10, 11, 12, 13, 14, 15, or 16)); preferably, L is absent;
iv) W is 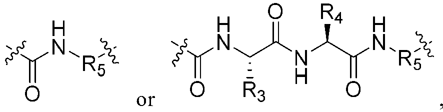 wherein R₃, R₄ and R₅ are as defined in claim 3; preferably, R₃, R₄ each independently are methyl, ethyl, n-propyl, iso-propyl or -(CH₂)_{c}-NHC(=O)NH₂, wherein c is as defined in claim 3; preferably, R₅ is 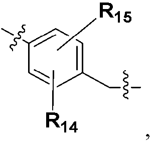 wherein R₁₄ and R₁₅ are as defined in claim 3;
preferably, R₃ and R₄ each independently are methyl, iso-propyl or -(CH₂)_{c}-NHC(=O)NH₂;
preferably, c is an integer from 0 to 6, such as 0, 1, 2, 3, 4, 5, or 6;
preferably, R₁₄ and R₁₅ each independently are H, methyl, ethyl, n-propyl, iso-propyl, n-butyl, sec-butyl, iso-butyl or tert-butyl; preferably, R₁₄ and R₁₅ each independently are H, methyl, ethyl or n-propyl; preferably, R₁₄ and R₁₅ each independently are H or methyl; preferably, R₁₄ and R₁₅ each independently are H;
preferably, -V-L-W- is 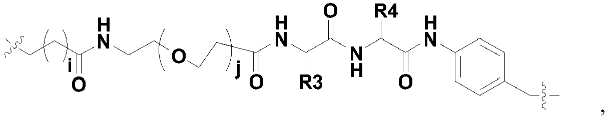 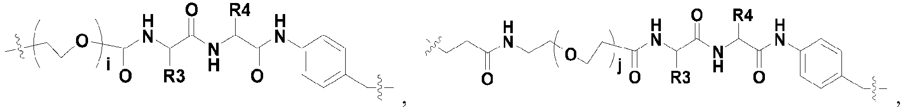 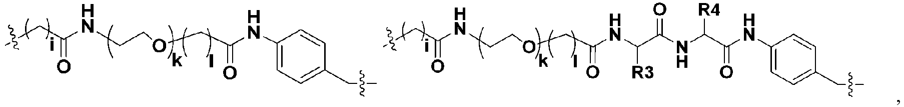 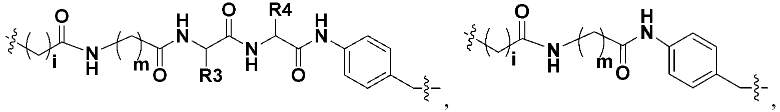 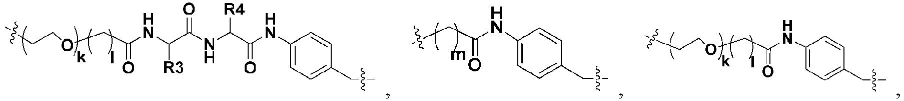 or 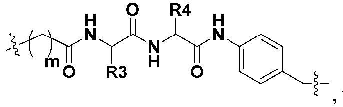 wherein i, j, k, l, m, R₃, and R₄ are as defined above;
preferably, -V-L-W- is 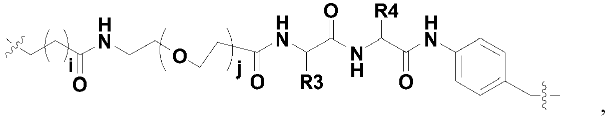 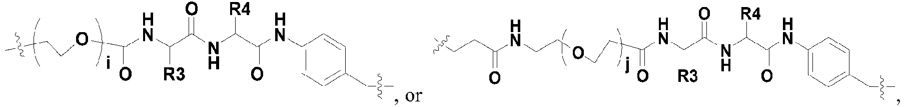 wherein i, j, k, l, m, R₃, and R₄ are as defined above.

13. The compound, a tautomer, a geometric isomer, an optical isomer, a polymorph or a pharmaceutically acceptable hydrate or solvate thereof according to any one of claims 10 to 12, wherein the compound has a structure represented by Formula I-2, 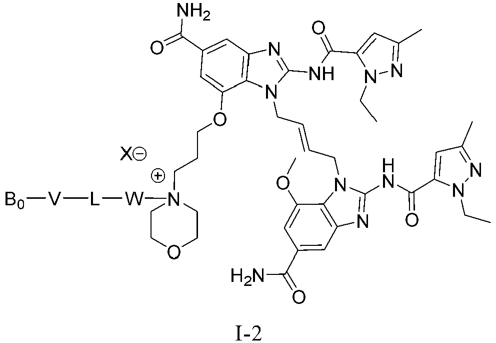 wherein B₀, X, V, L, and W are as defined in any one of claims 10 to 12.

14. The compound, a tautomer, a geometric isomer, an optical isomer, a polymorph or a pharmaceutically acceptable hydrate or solvate thereof according to any one of claims 8 to 13, wherein the compound is selected from the group consisting of: 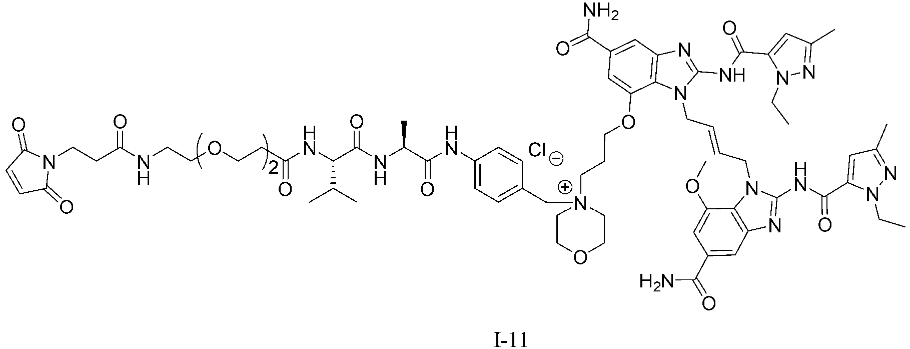 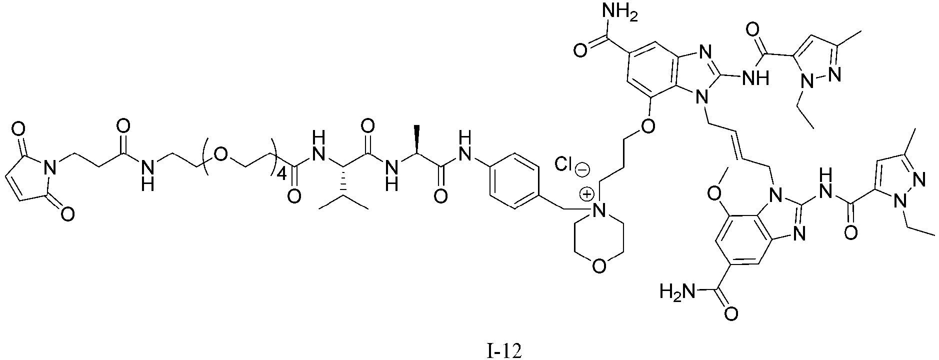 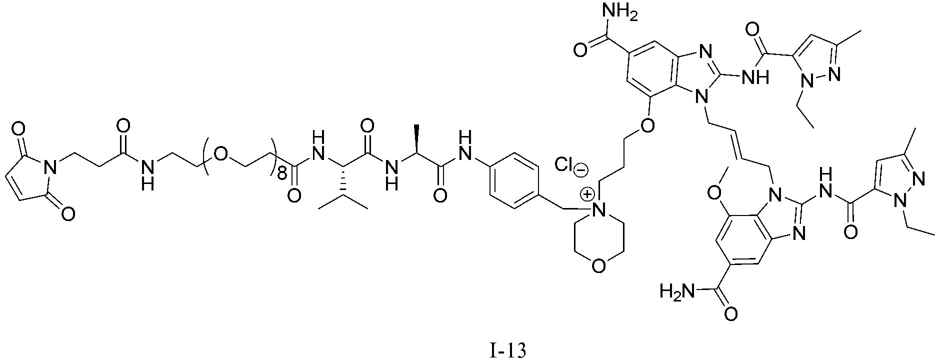 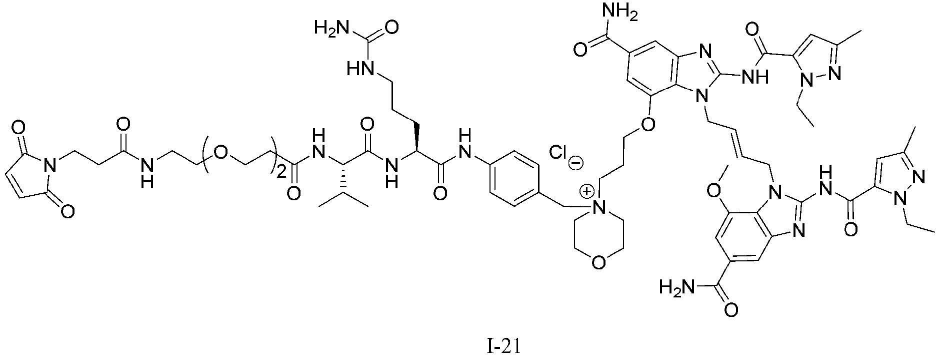 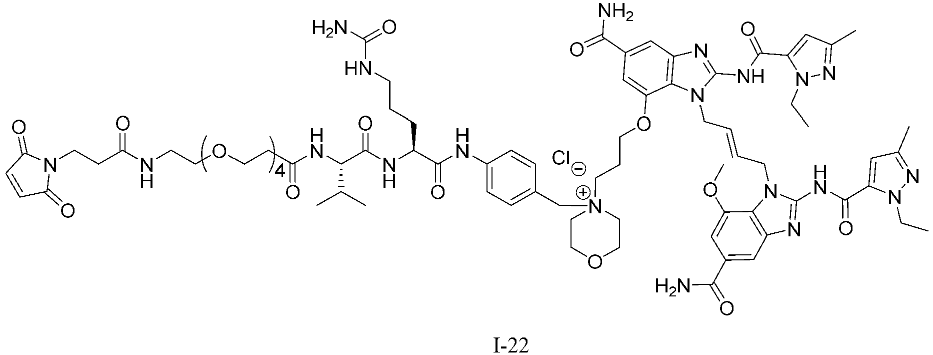 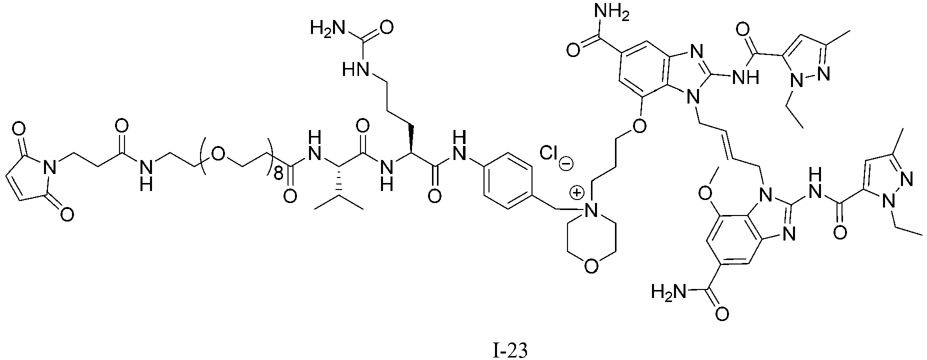 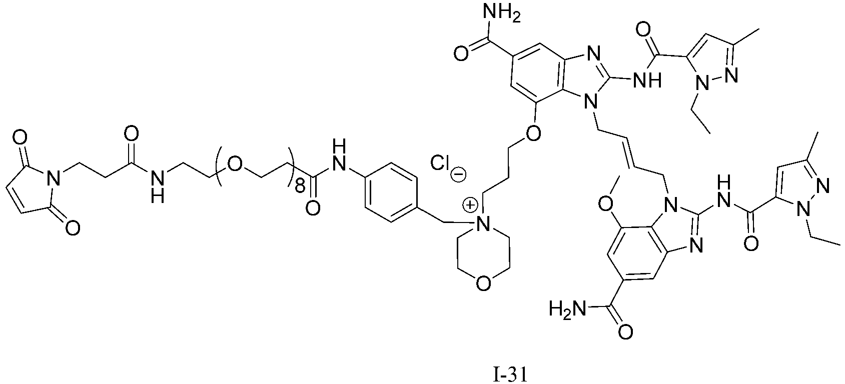 and

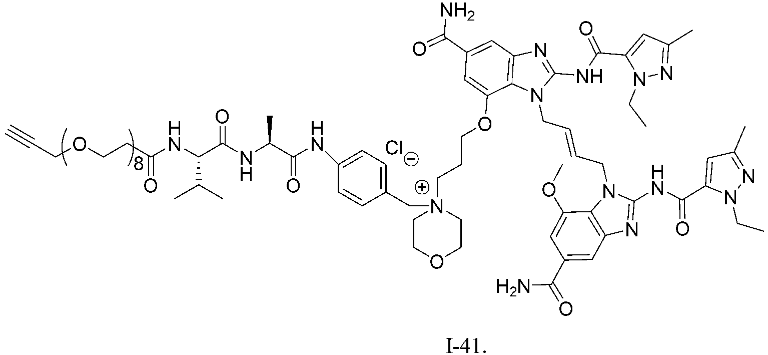

15. Use of the compound, a tautomer, a geometric isomer, an optical isomer, a polymorph or a pharmaceutically acceptable hydrate or solvate thereof according to any one of claims 8 to 14 in the preparation of an antibody-drug conjugate (e.g., an immune-stimulating antibody conjugate).

16. A pharmaceutical composition or vaccine, comprising the compound, a tautomer, a geometric isomer, an optical isomer, a polymorph or a pharmaceutically acceptable hydrate or solvate thereof according to any one of claims 1 to 7, and optionally one or more pharmaceutically acceptable carriers or excipients.

17. Use of the compound, a tautomer, a geometric isomer, an optical isomer, a polymorph or a pharmaceutically acceptable hydrate or solvate thereof according to any one of claims 1 to 7 in the manufacture of a medicament for treating and/or preventing a disease or condition or alleviating the severity of the disease or condition, wherein the disease or condition is selected from the group consisting of a tumor, a pathogen infection, a disease caused by a pathogen infection, a hematological disease, a metabolic disease, an inflammation, an allergic disease, an autoimmune disease, a precancerous syndrome, tumor metastasis, and a cardiovascular disease;
preferably, the tumor is cancer, lymphoma, lymphoid neoplasm, blastoma, sarcoma, or leukemia;
preferably, the pathogen infection is a bacterial infection or a viral infection;
preferably, the inflammation is an inflammation of any tissue and organ of a body, including musculoskeletal inflammation, vascular inflammation, neuroinflammation, inflammation of the digestive system, ocular inflammation, inflammation of the reproductive system, and other inflammations;
preferably, the viral infection is an infection caused by severe acute respiratory syndrome coronavirus (SARS-CoV), severe acute respiratory syndrome coronavirus 2 (SARS-CoV-2), or a variant strain thereof, influenza A virus, influenza B virus, parainfluenza virus, rhinovirus, dengue virus, Zika virus, yellow fever virus, or hepatitis B virus;
preferably, the bacterial infection is an infection caused by a Gram-positive bacterium, a penicillin-resistant bacterium, *Staphylococcus aureus, mycobacterium tuberculosis,* drug-resistant *mycobacterium tuberculosis, clostridium difficile,* or *chlamydia trachomatis;*
preferably, the disease caused by the viral infection is COVID-19;
preferably, the disease caused by the pathogen infection is a disease caused by a bacterial infection or a viral infection;
preferably, the disease caused by the bacterial infection is selected from the group consisting of bacterial pulmonary infection, sepsis caused by infections such as infections caused by *Staphylococcus aureus* infection, *pyocyania* infection, or *clostridium difficile* infection, tuberculosis, bacterial ocular infection, infection of heart, brain or skin, gastrointestinal tract infection, bacterial meningitis, or abscess in any organ (such as muscle, liver, meninges, or lung), cellulitis, bacteremia, skin necrosis, eyelid infection, ocular infection, neonatal conjunctivitis, osteomyelitis, impetigo, ecthyma, scalded skin syndrome, food poisoning, pneumonia, surgical infection, urinary tract infection, burn wound infection, meningitis, endocarditis, septicemia, toxic shock syndrome, and septic arthritis;
preferably, the cancer is selected from the group consisting of: breast cancer (e.g., HER2 positive breast cancer); squamous cell carcinoma (e.g., epithelial squamous cell carcinoma); lung cancer, including small cell lung cancer, non-small cell lung cancer, lung adenocarcinoma and lung squamous cell carcinoma; peritoneal cancer; liver cancer; gastric cancer; gastrointestinal cancer; pancreatic adenocarcinoma; glioblastoma; cervical cancer; ovarian cancer; bladder cancer; urethral carcinoma; hepatocellular carcinoma; intestinal cancer; colon cancer; rectal cancer; colorectal cancer; endometrial cancer; uterine cancer; salivary gland cancer; renal cancer; prostate cancer; vulvar cancer; thyroid cancer; anal cancer; penile cancer; melanoma; multiple myeloma and B-cell lymphoma; brain cancer; gallbladder cancer; esophageal cancer; cholangiocarcinoma; head and neck cancer and associated metastatic tumors;
preferably, the autoimmune disease includes, but is not limited to, STING-associated vasculopathy with onset in infancy (SAVI), Aicardi Goutières Syndrome (AGS), chilblain lupus, ataxia telangiectasia (also known as Louis-Bar syndrome), retinal vasculopathy with cerebral leukoencephalopathy (RCVL), systemic lupus erythematosus (SLE), cutaneous lupus, lupus nephritis, psoriasis, diabetes mellitus (including insulin dependent diabetes mellitus (IDDM), dermatomyositis, human immunodeficiency virus (HIV), AIDS, polymyositis, systemic sclerosis (scleroderma), and Sjogren's syndrome (SS), rheumatoid arthritis, psoriatic arthritis, polyarthritis, osteoarthritis, myasthenia gravis, polyarteritis nodosa, vasculitis, cutaneous vasculitis, anti-neutrophil cytoplasmic antibody (ANCA)-related vasculitis, Henoch-Schönlein purpura, autoimmune hepatitis, primary sclerosing cholangitis, granulomatosis with polyangiitis, microscopic polyangiitis, Behcet's disease, spondylitis, giant cell arteritis, polymyalgia rheumatica, Raynaud's phenomenon, primary biliary cirrhosis, primary vasculitis of the central nervous system, neuromyelitis optica, and mixed connective tissue disease.

18. Use of the compound, a tautomer, a geometric isomer, an optical isomer, a polymorph or a pharmaceutically acceptable hydrate or solvate thereof according to any one of claims 1 to 7 in the manufacture of a medicament for treating and/or preventing a STING-mediated disease or condition.
